(19) Europäisches Patentamt / European Patent Office / Office européen des brevets

(11) **EP 2 874 584 B1**

(12) **EUROPEAN PATENT SPECIFICATION**

(45) Date of publication and mention of the grant of the patent:
**25.05.2016 Bulletin 2016/21**

(21) Application number: **13745253.8**

(22) Date of filing: **19.07.2013**

(51) Int Cl.:
*A61F 9/008* (2006.01)

(86) International application number:
**PCT/US2013/051246**

(87) International publication number:
**WO 2014/015234 (23.01.2014 Gazette 2014/04)**

(54) **SYSTEMS AND METHODS FOR CORRECTING HIGH ORDER ABERRATIONS IN LASER REFRACTIVE SURGERY**

SYSTEME UND VERFAHREN ZUR KORREKTUR VON ABERRATIONEN HÖHERER ORDNUNG IN DER REFRAKTIVEN LASERCHIRURGIE

SYSTÈMES ET PROCÉDÉS DE CORRECTION D'ABERRATIONS D'ORDRE SUPÉRIEUR DANS UNE CHIRURGIE RÉFRACTIVE AU LASER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.07.2012 US 201213554276**

(43) Date of publication of application:
**27.05.2015 Bulletin 2015/22**

(73) Proprietor: **AMO Manufacturing USA, LLC Santa Ana, CA 92705 (US)**

(72) Inventors:
• **FABRIKANT, Anatoly**
  **Fremont, CA 94539 (US)**
• **DAI, Guang-Ming**
  **Fremont, CA 94555 (US)**
• **CHERNYAK, Dimitri**
  **Sunnyvale, CA 94087 (US)**
• **LOGAN, Ben**
  **Los Gatos, CA 95033 (US)**
• **HINDI, David**
  **Morgan Hill, CA 95037 (US)**
• **WANG, Qi**
  **San Jose, CA 95111 (US)**

(74) Representative: **Hoffmann Eitle Patent- und Rechtsanwälte PartmbB Arabellastraße 30 81925 München (DE)**

(56) References cited:
**WO-A2-02/43581     US-A1- 2005 254 006
US-B2- 6 673 062**

**Description**

BACKGROUND OF THE INVENTION

To some degree, all eyes deviate from a perfect optical system. These deviations, or aberrations, include imperfections, irregularities, or distortions of the optical quality of the eye, and can lead to refractive or visual errors. Aberrations can be classified into low order and high order aberrations, and can be described mathematically, for example, by Zernike polynomials.

Low order aberrations include prismatic, spherical, and cylindrical errors. First order, or prismatic, errors include vertical prism and horizontal prism errors. Second order, or defocus and astigmatism, errors include myopia, hyperopia, 45/135 astigmatism, and 90/180 astigmatism, for example. Traditional forms of optical correction involve measuring low order aberrations and prescribing sphero-cylindrical lenses in the form of glasses, contact lenses, and refractive surgery.

High order aberrations, on the other hand, are aberrations of the optical system that go beyond nearsightedness, farsightedness, and astigmatism. For example, third order aberrations include trefoil and coma. Fourth order aberrations include Z(4,-4), Z(4,-2), spherical aberration, Z(4,2), and Z(4,4) errors. Generally, high order aberrations include third order errors and above. Such aberrations are typically not corrected with glasses or contact lenses. High order optical errors of the human eye can be responsible for reduced visual acuity in spite of an optimal spherical or cylindrical refraction.

Wavefront-guided refractive surgery provides one method for measuring and treating low order and high order optical distortions in the eye. Wavefront systems measure how light is distorted as it passes into the eye and then is reflected back. An optical map of the eye can be created, detailing specific imperfections. There are several ways of analyzing the optical system of the eye using wavefront technology. One of the more common approaches involves the Hartmann-Shack wavefront sensing method.

Refractive surgery, including wavefront-guided custom ablation treatment, is effective in laser vision correction. However, current systems and methods may be less than ideal, and may even introduce or amplify high order aberrations. In light of the above, it would be desirable to have improved methods, devices, and systems that reduce optical aberrations or inhibit refractive surgery induced aberrations. Relatedly, it would be desirable to have improved methods, devices, and systems that determine, predict, or otherwise characterize optical aberrations or refractive surgery induced aberrations.

BRIEF SUMMARY OF THE INVENTION

The present invention provides a novel approach to evaluating and improving refractive surgery systems. Further, the present invention provides novel approaches to error source control and adjustment or optimization of ablation profiles or other optical data, for addressing high order aberrations. Relatedly, the present invention provides a novel simulation approach to identifying and characterizing system factors that can contribute to, or that can be adjusted to inhibit, optical aberrations. What is more, the present invention provides an approach to modeling of limitations or tolerances on system components so that adjustment of the system parameters such as, for example, the accuracy of registration, the accuracy of fitting in the ablation algorithm, the tracker speed, the accuracy and system latency time of tracking, and/or the laser beam uniformity and variability can be obtained for a certain level of high order aberration correction.

In a first aspect, the invention provides a method of inhibiting an induced aberration resulting from refractive surgery. Typically, the method can include inputting a target optical surface shape; determining a model optical surface shape based on the target optical surface shape and a set of refractive surgery system parameters; comparing the target optical surface shape and the model optical surface shape to determine an aberration induced by the set of refractive surgery system parameters; and adjusting the set of refractive surgery system parameters so as to inhibit the induced aberration. The set of refractive surgery system parameters can include at least one member selected from the group consisting of a wavefront device variable, a laser ablation profile variable, a laser registration and tracking system variable, a microkeratome variable, and a healing effect variable. The adjustment of the set of refractive surgery system parameters can be based on a metric selected from the group consisting of an accuracy variable, a heating variable, and a treatment time variable. The accuracy variable can be based on a root mean squares error factor, the heating variable can be based on a temperature factor, and the treatment time variable can be based on an ablation time factor.

In some aspects, the aberration may include a high order aberration. In other aspects, the target optical surface shape can be configured to address a low order aberration. The wavefront device variable can include a member selected from the group consisting of a spot identification factor, an accommodation factor, and a reconstruction factor. The reconstruction factor can include a member selected from a group consisting of uncompensated residual error portion, a measurement error portion, and a remaining error portion. The laser ablation profile variable can include a member selected from the group consisting of a pulse size factor, a spot size variability factor, a beam uniformity factor, and a laser pulse repetition rate factor. The microkeratome variable can include a member selected from the group consisting of a central flattening and peripheral thickening effect factor and a hinge effect factor. The laser registration and tracking system variable can include a member selected from the group consisting of a registration factor, a linear tracking factor, and a torsional tracking factor. In some aspects, the wavefront device variable can be configured to address a high order aberration. The wavefront device variable can include a gridsize factor adjusted to about 100 $\mu$m, and the laser ablation profile variable can include a flying spot scanning factor adjusted to range from about 1 mm to about 1.6 mm. The flying spot scanning factor can be adjusted to about 1.5 mm. The wavefront device variable can include a spot identification error adjusted to about 0.05 $\mu$m. The wavefront device variable can include a wavefront reconstruction error adjusted to about 0.05 $\mu$m. Similarly, the wavefront device variable can include an accommodation error adjusted to about 0.25D, equivalent to about 0.325 $\mu$m-wavefront RMS error for an approximately 6mm pupil.

In some related aspects, the microkeratome variable can include an induced positive spherical aberration adjusted to between about 0.1 $\mu$m and about 0.3 $\mu$m. The microkeratome variable can include a coma in the direction of the microkeratome hinge adjusted to between 0.1 $\mu$m and 0.3 $\mu$m. The healing effect variable can include a Gaussian kernel (e.g. a Gaussian low-pass filter kernel) adjusted to about 2 micron in height and about 0.5mm in full width at half maximum (FWHM).

In other related aspects, the set of refractive surgery system parameters can be adjusted such that a post-operative total high order RMS of about 0.3 $\mu$m is achieved. In some aspects, the pre-operative total high order RMS may be about 0.3 $\mu$m. In some aspects, each component of the total high order RMS does not exceed about 0.113 $\mu$m. In some aspects, each component of a sub-set of components of the total high order RMS does not exceed about 0.113 $\mu$m. In some cases, each of seven important components of the total high order RMS does not exceed about 0.113 $\mu$m. The set of refractive surgery system parameters can be adjusted such that a post-operative total high order RMS of about 0.1 $\mu$m is achieved. In some aspects, each component of the total high order RMS does not exceed about 0.038 $\mu$m. In some aspects, each component of a sub-set of components of the total high order RMS does not exceed about 0.038 $\mu$m. In some cases, each of seven important components of the total high order RMS does not exceed about 0.038 $\mu$m.

In still other aspects, the laser ablation profile variable can include a variable spot scanning factor, and the laser registration and tracking system variable can include a registration accuracy adjusted to less than about 10 $\mu$m in both the vertical and horizontal directions and a rotational error adjusted to less than about 0.5°. The laser ablation profile variable can include a flying spot scanning factor, and the laser registration and tracking system variable can include a registration accuracy adjusted to less than about 10 $\mu$m in both the vertical and horizontal directions and a rotational error adjusted to less than about 0.5°. The laser ablation profile variable can include a variable spot scanning factor, and the laser registration and tracking system variable can include a tracking accuracy adjusted to less than about 20 $\mu$m in both the vertical and horizontal directions, a latency time adjusted to less than about 10 ms, and a tracking speed adjusted to about 60 Hz or greater. The laser ablation profile variable can include a flying spot scanning factor, and the laser registration and tracking system variable can include a tracking accuracy adjusted to less than about 5 $\mu$m in both the vertical and horizontal directions, a latency time adjusted to less than 5 ms, and a tracking speed adjusted to about 200 Hz or greater. The laser ablation profile variable can include a variable spot scanning factor, and the laser registration and tracking system variable can include a cyclo-torsional tracking angular accuracy adjusted to 0.5° or better. The laser ablation profile variable can include a flying spot scanning factor, and the laser registration and tracking system variable can include a cyclo-torsional tracking angular accuracy adjusted to 0.25° or better. The laser ablation profile variable can include a variable spot scanning factor, and the laser registration and tracking system variable can include a laser energy fluctuation adjusted to less than 4%. The laser ablation profile variable can include a flying spot scanning factor, and the laser registration and tracking system variable can include a laser energy fluctuation adjusted to less than 2%.

In some embodiments, the target optical surface shape can include a set of 6-order Zernike polynomials, and the set of refractive surgery system parameters is adjusted such that each component of a post-operative total high order RMS does not exceed about 0.022 $\mu$m. The target optical surface shape can include a set of 6-order Zernike polynomials, and the set of refractive surgery system parameters is adjusted such that each component of a post-operative total high order RMS does not exceed about 0.0073 $\mu$m.

In some embodiments, the set of refractive surgery system parameters can be adjusted such that a post-operative total high order RMS is substantially equivalent to a pre-operative total high order RMS. The set of refractive surgery system parameters can be adjusted such that a post-operative total high order RMS is less than a pre-operative total high order RMS. The set of refractive surgery system parameters can be adjusted such that a post-operative total high order RMS is about one third the amount of a pre-operative total high order RMS.

In a second aspect, the present invention can provide a method of altering aberration distribution resulting from optical surface refractive surgery. The method can include inputting a target optical surface shape; determining a model optical surface shape based on the target optical surface shape and a set of refractive surgery system parameters; comparing the target optical surface shape and the model optical surface shape to determine an aberration distribution; and adjusting the set of refractive surgery system parameters so as to alter the aberration distribution.

In a third aspect, the present invention can provide a method of inhibiting a refractive surgery induced aberration.

The method can include inputting a target optical surface shape; determining a model optical surface shape based on the target optical surface shape and a set of refractive surgery system parameters, the model optical surface shape having an aberration; and adjusting the set of refractive surgery system parameters so as to inhibit the aberration.

In a fourth aspect, the present invention can provide a system for inhibiting an induced aberration resulting from refractive surgery. The system can include an input that accepts a target optical surface shape; a module that determines a model optical surface shape based on the target optical surface shape and a set of refractive surgery system parameters; and a module that adjusts the set of refractive surgery system parameters so as to inhibit an aberration in the model optical surface shape. The set of refractive surgery system parameters can include at least one member selected from the group consisting of a wavefront device variable, a laser ablation profile variable, a laser registration and tracking system variable, a microkeratome variable, and a healing effect variable. The module that adjusts the refractive surgery system parameters can include a metric selected from the group consisting of an accuracy variable, a heating variable, and a treatment time variable. The accuracy variable can be based on a root mean squares error factor, the heating variable can be based on a temperature factor, and the treatment time variable can be based on an ablation time factor.

In some aspects, the aberration may include a high order aberration. In other aspects, the target optical surface shape can be configured to address a low order aberration. The wavefront device variable can include a member selected from the group consisting of a spot identification factor, an accommodation factor, and a reconstruction factor. The reconstruction factor can include a member selected from a group consisting of uncompensated residual error portion, a measurement error portion, and a remaining error portion. The laser ablation profile variable can include a member selected from the group consisting of a pulse size factor, a spot size variability factor, a beam uniformity factor, and a laser pulse repetition rate factor. The microkeratome variable can include a member selected from the group consisting of a central flattening and peripheral thickening effect factor and a hinge effect factor. The laser registration and tracking system variable can include a member selected from the group consisting of a registration factor, a linear tracking factor, and a torsional tracking factor. In some aspects, the wavefront device variable can be configured to address a high order aberration. The wavefront device variable can include a gridsize factor adjusted to about 100 $\mu$m, and the laser ablation profile variable can include a flying spot scanning factor adjusted to range from about 1 mm to about 1.6 mm. The flying spot scanning factor can be adjusted to about 1.5 mm. The wavefront device variable can include a spot identification error adjusted to about 0.05 $\mu$m. The wavefront device variable can include a wavefront reconstruction error adjusted to about 0.05 $\mu$m. Similarly, the wavefront device variable can include an accommodation error adjusted to about 0.25D, equivalent to about 0.325 $\mu$m RMS error for an approximately 6mm pupil.

In some related aspects, the microkeratome variable can include an induced positive spherical aberration adjusted to between about 0.1 $\mu$m and about 0.3 $\mu$m. The microkeratome variable can include a coma in the direction of the microkeratome hinge adjusted to between 0.1 $\mu$m and 0.3 $\mu$m. The healing effect variable can include a Gaussian kernel (e.g. a Gaussian low-pass filter kernel) adjusted to about 2 micron in height and about 0.5mm in full width at half maximum (FWHM).

In other related aspects, the set of refractive surgery system parameters can be adjusted such that a post-operative total high order RMS of about 0.3 $\mu$m is achieved. In some aspects, the pre-operative total high order RMS may be about 0.3 $\mu$m. In some aspects, each component of the total high order RMS does not exceed about 0.113 $\mu$m. The set of refractive surgery system parameters can be adjusted such that a post-operative total high order RMS of about 0.1 $\mu$m is achieved. In some aspects, each component of the total high order RMS does not exceed about 0.038 $\mu$m.

In still other aspects, the laser ablation profile variable can include a variable spot scanning factor, and the laser registration and tracking system variable can include a registration accuracy adjusted to less than about 10 $\mu$m in both the vertical and horizontal directions and a rotational error adjusted to less than about 0.5°. The laser ablation profile variable can include a flying spot scanning factor, and the laser registration and tracking system variable can include a registration accuracy adjusted to less than about 10 $\mu$m in both the vertical and horizontal directions and a rotational error adjusted to less than about 0.5°. The laser ablation profile variable can include a variable spot scanning factor, and the laser registration and tracking system variable can include a tracking accuracy adjusted to less than about 20 $\mu$m in both the vertical and horizontal directions, a latency time adjusted to less than about 10 ms, and a tracking speed adjusted to about 60 Hz or greater. The laser ablation profile variable can include a flying spot scanning factor, and the laser registration and tracking system variable can include a tracking accuracy adjusted to less than about 5 $\mu$m in both the vertical and horizontal directions, a latency time adjusted to less than 5 ms, and a tracking speed adjusted to about 200 Hz or greater. The laser ablation profile variable can include a variable spot scanning factor, and the laser registration and tracking system variable can include a cyclo-torsional tracking angular accuracy adjusted to 0.5° or better. The laser ablation profile variable can include a flying spot scanning factor, and the laser registration and tracking system variable can include a cyclo-torsional tracking angular accuracy adjusted to 0.25° or better. The laser ablation profile variable can include a variable spot scanning factor, and the laser registration and tracking system variable can include a laser energy fluctuation adjusted to less than 4%. The laser ablation profile variable can include a flying spot scanning factor, and the laser registration and tracking system variable can include a laser energy fluctuation adjusted to less than 2%.

In some embodiments, the target optical surface shape can include a set of 6-order Zernike polynomials, and the set of refractive surgery system parameters is adjusted such that each component of a post-operative total high order RMS does not exceed about 0.022 μm. The target optical surface shape can include a set of 6-order Zernike polynomials, and the set of refractive surgery system parameters is adjusted such that each component of a post-operative total high order RMS does not exceed about 0.0073 μm.

In some embodiments, the set of refractive surgery system parameters can be adjusted such that a post-operative total high order RMS is substantially equivalent to a pre-operative total high order RMS. The set of refractive surgery system parameters can be adjusted such that a post-operative total high order RMS is less than a pre-operative total high order RMS. The set of refractive surgery system parameters can be adjusted such that a post-operative total high order RMS is about one third the amount of a pre-operative total high order RMS.

In one aspect, embodiments of the present invention encompass methods of evaluating error in a vision correction procedure. Exemplary methods may include receiving a treatment table containing laser device instructions, receiving a set of surgery parameters associated with the vision correction procedure, determining an original treatment based on original values for the set of surgery parameters, generating a plurality of random modified values for at least one parameter of the set of surgery parameters, simulating a treatment based on the plurality of random modified values and the treatment table, and evaluating error in the vision correction procedure based on a comparison between the original treatment and the simulated treatment. According to some embodiments, the set of surgery parameters may include a treatment plan parameter, a flap incision parameter, an ablation parameter, a human error parameter, a psychology parameter, a physiology parameter, a patient perception parameter, a surgical condition parameter, a surgical environment parameter, or any combination thereof. In some instances, a set of surgery parameters includes a treatment plan parameter such as an aberration measurement parameter or an ablation surface fit parameter, or both. In some instances, an aberration measurement parameter may include a wavefront measurement parameter. In some instances, a set of surgery parameters may include a flap incision parameter such as a shape parameter or a uniformity parameter, or both. In some instances, a set of surgery parameters may include an ablation parameter such as a position parameter, a spot shape parameter, or a pulse ablation depth parameter, or any combination thereof. In some instances, a set of surgery parameters may include a human factor parameter such as a manual input parameter or a physician adjustment parameter, or both. In some instances, a set of surgery parameters may include a psychology parameter such as a physician instruction parameter or a patient behavior parameter, or both. In some instances, a set of surgery parameters may include a physiology parameter such as a bio-mechanics parameter, an epithelial healing parameter, or a stroma regeneration parameter, or any combination thereof. In some instances, a treatment table may include laser device instructions for a refractive case such as myopia, hyperopia, myopic astigmatism, hyperopic astigmatism, mixed astigmatism, or a high-order (ocular wavefront) based therapeutic case. In some instances, a plurality of random modified values can be generated for a parameter associated with a permanent error. In some instances, a plurality of random modified values can be generated for a parameter associated with an alignment error. In some instances, a plurality of random modified values can be generated for a parameter associated with a calibration error. In some instances, a plurality of random modified values can be generated for a parameter associated with a treatment error. In some instances, a plurality of random modified values can be generated for a parameter associated with a noise fluctuation. In some instances, a set of surgery parameters can include a surface fitting parameter, a tracking parameter, a registration parameter, or a laser energy fluctuation parameter, or any combination thereof. In some instances, error in a vision correction procedure can be evaluated according to a root-mean-square analysis. In some instances, error in a vision correction procedure can be evaluated according to a low order aberration analysis. In some instances, error in a vision correction procedure can be evaluated according to a high order aberration analysis. In some instances, a set of surgery parameters can include a surgical condition parameter such as a keratometry parameter, a pachymetry parameter, or an intraocular eye pressure (IOP) parameter, or any combination thereof. In some instances, a set of surgery parameters may include a surgical environment parameter such as a temperature parameter, a humidity parameter, or a latitude parameter, or any combination thereof.

In another aspect, embodiments of the present invention encompass systems for evaluating error in a vision correction procedure. Exemplary systems may include an input that receives a treatment table containing laser device instructions, an input that receives a set of surgery parameters associated with the vision correction procedure, a module that determines an original treatment based on original values for the set of surgery parameters, a module that generates a plurality of random modified values for at least one parameter of the set of surgery parameters, a module that simulates a treatment based on the plurality of random modified values and the treatment table, and a module that evaluates error in the vision correction procedure based on a comparison between the original treatment and the simulated treatment. In some instances, a set of surgery parameters may include a treatment plan parameter, a flap incision parameter, an ablation parameter, a human error parameter, a psychology parameter, a physiology parameter, a patient perception parameter, a surgical condition parameter, or a surgical environment parameter, or any combination thereof. In some instances, a set of surgery parameters may include a treatment plan parameter such as an aberration measurement parameter or an ablation surface fit parameter, or both.. In some instances, an aberration measurement parameter may include a wavefront measurement parameter. In some instances, a set of surgery parameters may include a flap incision parameter such as a shape parameter or a uniformity parameter, or both. In some instances, a set of surgery parameters may include an ablation parameter such as a position parameter, a spot shape parameter, or a pulse ablation depth parameter, or any combination thereof. In some instances, a set of surgery parameters may include a human factor parameter such as a manual input parameter or a physician adjustment parameter, or both. In some instances, a set of surgery parameters may include a psychology parameter such as a physician instruction parameter or a patient behavior parameter, or both. In some instances, a set of surgery parameters may include a physiology parameter such as a bio-mechanics parameter, an epithelial healing parameter, or a stroma regeneration parameter, or any combination thereof. In some instances, a set of surgery parameters may include a surface fitting parameter, a tracking parameter, a registration parameter, or a laser energy fluctuation parameter, or any combination thereof. In some instances, a set of surgery parameters may include a surgical condition parameter such as a keratometry parameter, a pachymetry parameter, or an intraocular eye pressure (IOP) parameter, or any combination thereof. In some instances, a set of surgery parameters may include a surgical environment parameter such as a temperature parameter, a humidity parameter, or a latitude parameter, or any combination thereof.

In another aspect, embodiments of the present invention encompass methods for inhibiting an induced aberration resulting from refractive surgery. Exemplary methods may include inputting a refractive case to an input device of a computer system, and determining a model optical surface shape based on the refractive case and a set of refractive surgery system parameters with a determination module of the computer system, where the set of refractive surgery system parameters is embodied within a data file of a refractive surgery system. Methods may also include comparing the refractive case and the model optical surface shape to determine an aberration induced by the set of refractive surgery system parameters embodied within the data file of the refractive surgery system with a comparison module of the computer system, and adjusting the set of refractive surgery system parameters embodied within the data file of the refractive surgery system so as to inhibit the induced aberration with an adjustment module of the computer system.

In still another aspect, embodiments of the present invention encompass methods of altering aberration distribution resulting from optical surface refractive surgery. Exemplary methods may include inputting a refractive case to an input device of a computer system, and determining a model optical surface shape based on the refractive case and a set of refractive surgery system parameters with a determination module of the computer system, where the set of refractive surgery system parameters is embodied within machine readable data of a tangible storage media of a refractive surgery system. Methods may also include comparing the refractive case and the model optical surface shape to determine an aberration distribution with a comparison module of the computer system, and adjusting the set of refractive surgery system parameters embodied within machine readable data of the tangible storage media of the refractive surgery system so as to alter the aberration distribution with an adjustment module of the computer system.

In yet another aspect, embodiments of the present invention encompass methods of inhibiting a refractive surgery induced aberration. Exemplary methods may include inputting a refractive case to an input device of a computer system, and determining a model optical surface shape based on the refractive case and a set of refractive surgery system parameters, the model optical surface shape having an aberration with a determination module of the computer system, where the set of refractive surgery system parameters is embodied within a storage module of a refractive surgery system. Methods may also include adjusting the set of refractive surgery system parameters embodied within the storage module of the refractive surgery system so as to inhibit the aberration with an adjustment module of the computer system.

In still a further aspect, embodiments of the present invention encompass systems for inhibiting an induced aberration resulting from refractive surgery. Exemplary systems may include an input that accepts a refractive case, a module that determines a model optical surface shape based on the refractive case and a set of refractive surgery system parameters, and a module that adjusts the set of refractive surgery system parameters so as to inhibit an aberration in the model optical surface shape.

In still yet another aspect, embodiments of the present invention encompass methods of adjusting a set of refractive surgery system parameters for use in a refractive treatment. Exemplary methods may include inputting a refractive case, determining a model optical surface shape based on the refractive case and a set of refractive surgery system parameters, comparing the refractive case and the model optical surface shape to determine an aberration induced by the set of refractive surgery system parameters, adjusting the set of refractive surgery system parameters so as to inhibit the induced aberration, and administering the refractive treatment to a patient, where the refractive treatment is based on the adjusted set of refractive surgery system parameters.

In yet another aspect, embodiments of the present invention encompass systems for inhibiting an induced aberration resulting from refractive surgery. Exemplary systems may include an input device that accepts a refractive case, and a determination module that determines a model optical surface shape based on the refractive case shape and a set of refractive surgery system parameters, where the set of refractive surgery system parameters is embodied within a data file. Systems may also include a comparison module that compares the refractive case and the model optical surface shape to determine an aberration induced by the set of refractive surgery system parameters embodied within the data file, and an adjustment module that adjusts the set of refractive surgery system parameters embodied within the data file so as to inhibit the induced aberration.

In another aspect, embodiments of the present invention encompass systems for altering aberration distribution resulting from optical surface refractive surgery. Exemplary systems may include an input device that accepts a refractive case, and a determination module that determines a model optical surface shape based on the refractive case and a set of refractive surgery system parameters, where the set of refractive surgery system parameters is embodied within machine readable data of a tangible storage media. Systems may also include a comparison module that compares the refractive case and the model optical surface shape to determine an aberration distribution, and an adjustment module that adjusts the set of refractive surgery system parameters embodied within machine readable data of the tangible storage media so as to alter the aberration distribution.

In another aspect, embodiments of the present invention encompass systems for inhibiting a refractive surgery induced aberration. Exemplary systems may include an input device that accepts a refractive case, and a determination module that determines a model optical surface shape based on the refractive case and a set of refractive surgery system parameters, where the model optical surface shape has an aberration, and where the set of refractive surgery system parameters is embodied within a storage module. Systems may also include an adjustment module that adjusts the set of refractive surgery system parameters embodied within the storage module so as to inhibit the aberration.

In still a further aspect, embodiments of the present invention encompass systems for adjusting a set of refractive surgery system parameters for use in a refractive treatment. Exemplary systems may include an input device that accepts a refractive case, a determination module that determines a model optical surface shape based on the refractive case and a set of refractive surgery system parameters, a comparison module that compares the refractive case and the model optical surface shape to determine an aberration induced by the set of refractive surgery system parameters, an adjustment module that adjusts the set of refractive surgery system parameters so as to inhibit the induced aberration, and a treatment module that administers the refractive treatment to a patient, wherein the refractive treatment is based on the adjusted set of refractive surgery system parameters.

These and other aspects will be apparent in the remainder of the figures, description, and claims.

BRIEF DESCRIPTION OF THE DRAWINGS

Figure 1 illustrates a laser ablation system according to an embodiment of the present invention.

Figure 2 illustrates a simplified computer system according to an embodiment of the present invention.

Figure 3 illustrates a wavefront measurement system according to an embodiment of the present invention.

Figure 3A illustrates another wavefront measurement system according to an embodiment of the present invention.

Figure 4 illustrates a system flow diagram according to an embodiment of the present invention.

Figure 5 illustrates a system flow diagram according to an embodiment of the present invention.

Figure 6 illustrates a simulator flow diagram according to an embodiment of the present invention.

Figure 7 illustrates RMS error due to accommodation according to an embodiment of the present invention.

Figure 8 illustrates RMS error due to reconstruction according to an embodiment of the present invention.

Figure 8A illustrates high order aberrations of a therapeutic eye according to an embodiment of the present invention.

Figure 9A illustrates a flying spot scanning profile according to an embodiment of the present invention.

Figure 9B illustrates a variable spot scanning profile according to an embodiment of the present invention.

Figure 9C illustrates a fitting error comparison according to an embodiment of the present invention.

Figures 10A illustrates fitting performance according to an embodiment of the present invention.

Figures 11A illustrates fitting performance according to an embodiment of the present invention.

Figure 12A illustrates an RMS distribution graph for FSS according to an embodiment of the present invention.

Figure 12B illustrates a PV distribution graph for FSS according to an embodiment of the present invention.

Figures 13A-E illustrate registration error analysis according to an embodiment of the present invention.

Figure 14A illustrates real X motion according to an embodiment of the present invention.

Figures 14B-D illustrate additional real and simulated eye motions according to an embodiment of the present invention.

Figures 15A-F illustrate a comparison of VSS and FSS observed tracking according to an embodiment of the present invention.

Figure 16A illustrates VSS torsional tracking efficiency according to an embodiment of the present invention.

Figure 16B illustrates torsional tracking efficiency with RMS error for VSS, with respect to tracking error, according to an embodiment of the present invention.

Figure 16C illustrates torsional tracking efficiency with RMS error for VSS, with respect to system latency, according to an embodiment of the present invention.

Figure 16D illustrates torsional tracking efficiency for FSS, with respect to tracking speed, according to an embodiment of the present invention.

Figure 16E illustrates torsional tracking efficiency for FSS, with respect to tracking error, according to an embodiment of the present invention.

Figure 16F illustrates torsional tracking efficiency for FSS, with respect to system latency, according to an embodiment of the present invention.

Figure 17A illustrates a tracking error comparison between VSS and FSS according to an embodiment of the present invention.

Figure 17B illustrates a torsional error comparison between VSS and FSS according to an embodiment of the present invention.

Figure 17C illustrates a torsional error (no tracking) comparison between VSS and FSS according to an embodiment of the present invention.

Figure 18A illustrates a beam uniformity induced RMS error in VSS according to an embodiment of the present invention.

Figure 18B illustrates a beam variability induced RMS error in VSS, with respect to laser energy decay, according to an embodiment of the present invention.

Figure 18C illustrates a beam variability induced RMS error in VSS, with respect to laser pulse repetition rate, according to an embodiment of the present invention.

Figure 18D illustrates a beam uniformity induced RMS error in FSS according to an embodiment of the present invention.

Figure 18E illustrates a beam variability induced RMS error in FSS, with respect to laser energy decay, according to an embodiment of the present invention.

Figure 18F illustrates a beam variability induced RMS error in FSS, with respect to laser pulse repetition rate, according to an embodiment of the present invention.

Figure 19A illustrates a beam variability error comparison between VSS and FSS according to an embodiment of the present invention.

Figure 19B illustrates a beam uniformity error comparison between VSS and FSS according to an embodiment of the present invention.

Figure 20 illustrates an input myopic ablation profile pre- and post-healing according to an embodiment of the present invention.

Figure 21 illustrates an input hyperopic profile pre- and post-healing according to an embodiment of the present invention.

Figure 22 illustrates an error comparison between VSS and FSS, without a healing effect.

Figures 23A and 23B illustrate comparisons of VSS and FSS for various error sources according to an embodiment of the present invention.

Figures 24 to 24E depict exemplary aspects of systems and methods according to embodiments of the present invention.

Figure 25 illustrates exemplary aspects of systems and methods according to embodiments of the present invention.

Figure 26 illustrates exemplary aspects of systems and methods according to embodiments of the present invention.

Figure 27 graphically represents aspects of various parameters, in terms of error for selected components in a laser system, according to embodiments of the present invention.

Figure 28 depicts aspects of error analysis systems and methods according to embodiments of the present invention.

Figure 29 depicts aspects of error analysis systems and methods according to embodiments of the present invention.

Figure 30 depicts aspects of error analysis systems and methods according to embodiments of the present invention.

Figure 31 depicts fluence or integrator transmission fluctuations according to embodiments of the present invention.

Figure 32 shows ozone calibration repeatability curves according to embodiments of the present invention.

Figure 33 illustrates measured ablation optical path differences compared to laser pulse fluences according to embodiments of the present invention.

Figure 34A depicts a rotating lens mount driven by a rotating pinion according to embodiments of the present invention.

Figure 34B depicts spot shift due to shift in the steering lens according to embodiments of the present invention.

Figure 35 illustrates spot positioning errors along the X and Y arcs according to embodiments of the present invention.

Figure 36 shows effects of head shift on positioning error according to embodiments of the present invention.

Figure 37 depicts a window of a graphical user interface software or product for error or numerical analysis according to embodiments of the present invention.

Figures 38A and 38B depict factor analysis for various simulated errors in baseline and modified systems, respectively, according to embodiments of the present invention.

Figures 38C and 38D depict factor analysis for various simulated errors in baseline and modified systems, respectively, according to embodiments of the present invention.

Figure 39 illustrates factor analysis for both low order aberrations and high order aberrations, for baseline and modified systems, according to embodiments of the present invention.

Figures 39A and 39B depict factor analysis for aberrations with significant error sources for baseline and modified systems, respectively, according to embodiments of the present invention.

Figure 40 illustrates aspects of ablation error estimates, according to embodiments of the present invention.

Figure 41 illustrates typical vertical (Z) oscillations of the patient cornea measured with a baseline system eye tracker, according to embodiments of the present invention.

Figure 42 shows aspects of beamlet divergence and spot widening, according to embodiments of the present invention.

Figure 43 depicts aspects of X and Y components of a wall reflection spot charted with respect to time, according to embodiments of the present invention.

DETAILED DESCRIPTION OF THE INVENTION

The present invention can be readily adapted for use with existing laser systems, wavefront measurement systems, and other optical measurement devices. While the systems, software, and methods of the present invention are described primarily in the context of a laser eye surgery system, it should be understood the present invention may be adapted for use in alternative eye treatment procedures and systems such as spectacle lenses, intraocular lenses, contact lenses, corneal ring implants, collagenous corneal tissue thermal remodeling, and the like.

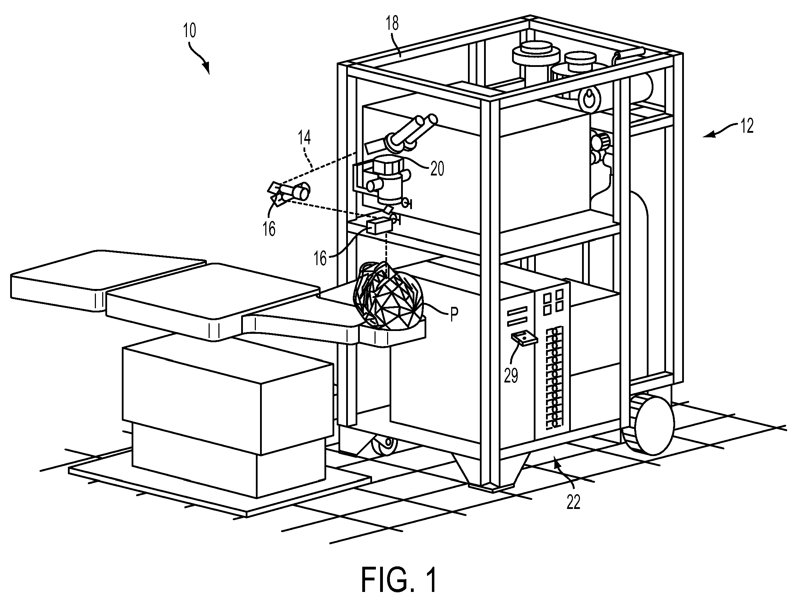

Turning now to the drawings, **Fig. 1** illustrates a laser eye surgery system 10 of the present invention, including a laser 12 that produces a laser beam 14. Laser 12 is optically coupled to laser delivery optics 16, which directs laser beam 14 to an eye E of patient P. A delivery optics support structure (not shown here for clarity) extends from a frame 18 supporting laser 12. A microscope 20 is mounted on the delivery optics support structure, the microscope often being used to image a cornea of eye E.

Laser 12 generally comprises an excimer laser, ideally comprising an argon-fluorine laser producing pulses of laser light having a wavelength of approximately 193 nm. Laser 12 will preferably be designed to provide a feedback stabilized fluence at the patient's eye, delivered via delivery optics 16. The present invention may also be useful with alternative sources of ultraviolet or infrared radiation, particularly those adapted to controllably ablate the corneal tissue without causing significant damage to adjacent and/or underlying tissues of the eye. Such sources include, but are not limited to, solid state lasers and other devices which can generate energy in the ultraviolet wavelength between about 185 and 205 nm and/or those which utilize frequency-multiplying techniques. Hence, although an excimer laser is the illustrative source of an ablating beam, other lasers may be used in the present invention.

Laser system 10 will generally include a computer or programmable processor 22. Processor 22 may comprise (or interface with) a conventional PC system including the standard user interface devices such as a keyboard, a display monitor, and the like. Processor 22 will typically include an input device such as a magnetic or optical disk drive, an internet connection, or the like. Such input devices will often be used to download a computer executable code from a tangible storage media 29 embodying any of the methods of the present invention. Tangible storage media 29 may take the form of a floppy disk, an optical disk, a data tape, a volatile or non-volatile memory, RAM, or the like, and the processor 22 will include the memory boards and other standard components of modern computer systems for storing and executing this code. Tangible storage media 29 may optionally embody wavefront sensor data, wavefront gradients, a wavefront elevation map, a treatment map, a corneal elevation map, and/or an ablation table. While tangible storage media 29 will often be used directly in cooperation with an input device of processor 22, the storage media may also be remotely operatively coupled with processor by means of network connections such as the internet, and by wireless methods such as infrared, Bluetooth, or the like.

Laser 12 and delivery optics 16 will generally direct laser beam 14 to the eye of patient P under the direction of a computer 22. Computer 22 will often selectively adjust laser beam 14 to expose portions of the cornea to the pulses of laser energy so as to effect a predetermined sculpting of the cornea and alter the refractive characteristics of the eye. In many embodiments, both laser beam 14 and the laser delivery optical system 16 will be under computer control of processor 22 to effect the desired laser sculpting process, with the processor effecting (and optionally modifying) the pattern of laser pulses. The pattern of pulses may by summarized in machine readable data of tangible storage media 29 in the form of a treatment table, and the treatment table may be adjusted according to feedback input into processor 22 from an automated image analysis system in response to feedback data provided from an ablation monitoring system feedback system. Optionally, the feedback may be manually entered into the processor by a system operator. Such feedback might be provided by integrating the wavefront measurement system described below with the laser treatment system 10, and processor 22 may continue and/or terminate a sculpting treatment in response to the feedback, and may optionally also modify the planned sculpting based at least in part on the feedback. Measurement systems are further described in U.S. Patent No. 6,315,413, the full disclosure of which is incorporated herein by reference.

Laser beam 14 may be adjusted to produce the desired sculpting using a variety of alternative mechanisms. The laser beam 14 may be selectively limited using one or more variable apertures. An exemplary variable aperture system having a variable iris and a variable width slit is described in U.S. Patent No. 5,713, 892. The laser beam may also be tailored by varying the size and offset of the laser spot from an axis of the eye, as described in U.S. Patent Nos. 5,683,379, 6,203,539, and 6,331,177.

Still further alternatives are possible, including scanning of the laser beam over the surface of the eye and controlling the number of pulses and/or dwell time at each location, as described, for example, by U.S. Patent No. 4,665,913 ; using masks in the optical path of laser beam 14 which ablate to vary the profile of the beam incident on the cornea, as described in U.S. Patent No. 5,807,379; hybrid profile-scanning systems in which a variable size beam (typically controlled by a variable width slit and/or variable diameter iris diaphragm) is scanned across the cornea; or the like. The computer programs and control methodology for these laser pattern tailoring techniques are well described in the patent literature.

Additional components and subsystems may be included with laser system 10, as should be understood by those of skill in the art. For example, spatial and/or temporal integrators may be included to control the distribution of energy within the laser beam, as described in U.S. Patent No. 5,646,791. Ablation effluent evacuators/filters, aspirators, and other ancillary components of the laser surgery system are known in the art. Further details of suitable systems for performing a laser ablation procedure can be found in commonly assigned U.S. Pat. Nos. 4,665,913, 4,669,466, 4,732,148, 4,770,172, 4,773,414, 5,207,668, 5,108,388, 5,219,343, 5,646,791 and 5,163,934. Suitable systems also include commercially available refractive laser systems such as those manufactured and/or sold by Alcon, Bausch & Lomb, Nidek, WaveLight, LaserSight, Schwind, Zeiss-Meditec, and the like. Basis data can be further characterized for particular lasers or operating conditions, by taking into account localized environmental variables such as temperature, humidity, airflow, and aspiration.

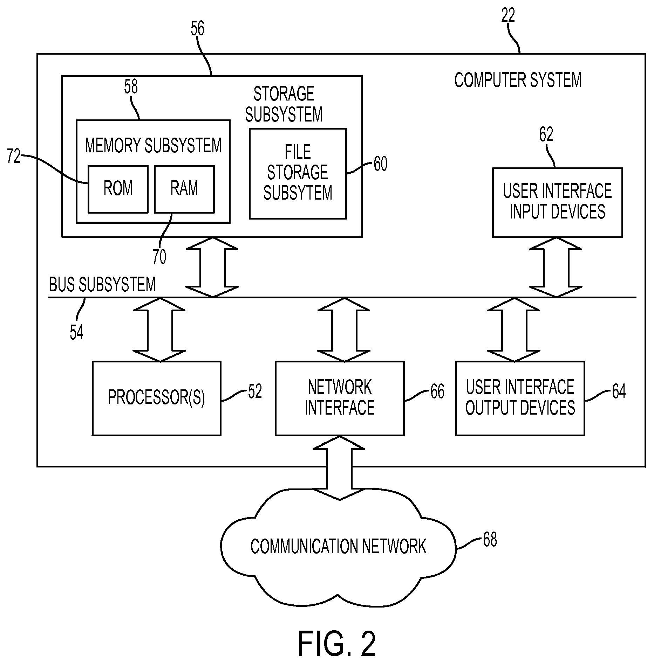

Fig. 2 is a simplified block diagram of an exemplary computer system 22 that may be used by the laser surgical system 10 of the present invention. Computer system 22 typically includes at least one processor 52 which may communicate with a number of peripheral devices via a bus subsystem 54. These peripheral devices may include a storage subsystem 56, comprising a memory subsystem 58 and a file storage subsystem 60, user interface input devices 62, user interface output devices 64, and a network interface subsystem 66. Network interface subsystem 66 provides an interface to outside networks 68 and/or other devices, such as the wavefront measurement system 30.

User interface input devices 62 may include a keyboard, pointing devices such as a mouse, trackball, touch pad, or graphics tablet, a scanner, foot pedals, a joystick, a touchscreen incorporated into the display, audio input devices such as voice recognition systems, microphones, and other types of input devices. User input devices 62 will often be used to download a computer executable code from a tangible storage media 29 embodying any of the methods of the present invention. In general, use of the term "input device" is intended to include a variety of conventional and proprietary devices and ways to input information into computer system 22.

User interface output devices 64 may include a display subsystem, a printer, a fax machine, or non-visual displays such as audio output devices. The display subsystem may be a cathode ray tube (CRT), a flat-panel device such as a liquid crystal display (LCD), a projection device, or the like. The display subsystem may also provide a non-visual display such as via audio output devices. In general, use of the term "output device" is intended to include a variety of conventional and proprietary devices and ways to output information from computer system 22 to a user.

Storage subsystem 56 can store the basic programming and data constructs that provide the functionality of the various embodiments of the present invention. For example, a database and modules implementing the functionality of the methods of the present invention, as described herein, may be stored in storage subsystem 56. These software modules are generally executed by processor 52. In a distributed environment, the software modules may be stored on a plurality of computer systems and executed by processors of the plurality of computer systems. Storage subsystem 56 typically comprises memory subsystem 58 and file storage subsystem 60.

Memory subsystem 58 typically includes a number of memories including a main random access memory (RAM) 70 for storage of instructions and data during program execution and a read only memory (ROM) 72 in which fixed instructions are stored. File storage subsystem 60 provides persistent (non-volatile) storage for program and data files, and may include tangible storage media 29 (**Fig. 1**) which may optionally embody wavefront sensor data, wavefront gradients, a wavefront elevation map, a treatment map, and/or an ablation table. File storage subsystem 60 may include a hard disk drive, a floppy disk drive along with associated removable media, a Compact Digital Read Only Memory (CD-ROM) drive, an optical drive, DVD, CD-R, CD-RW, solid-state removable memory, and/or other removable media cartridges or disks. One or more of the drives may be located at remote locations on other connected computers at other sites coupled to computer system 22. The modules implementing the functionality of the present invention may be stored by file storage subsystem 60.

Bus subsystem 54 provides a mechanism for letting the various components and subsystems of computer system 22 communicate with each other as intended. The various subsystems and components of computer system 22 need not be at the same physical location but may be distributed at various locations within a distributed network. Although bus subsystem 54 is shown schematically as a single bus, alternate embodiments of the bus subsystem may utilize multiple busses.

Computer system 22 itself can be of varying types including a personal computer, a portable computer, a workstation, a computer terminal, a network computer, a control system in a wavefront measurement system or laser surgical system, a mainframe, or any other data processing system. Due to the ever-changing nature of computers and networks, the description of computer system 22 depicted in **Fig. 2** is intended only as a specific example for purposes of illustrating one embodiment of the present invention. Many other configurations of computer system 22 are possible having more or less components than the computer system depicted in **Fig. 2**.

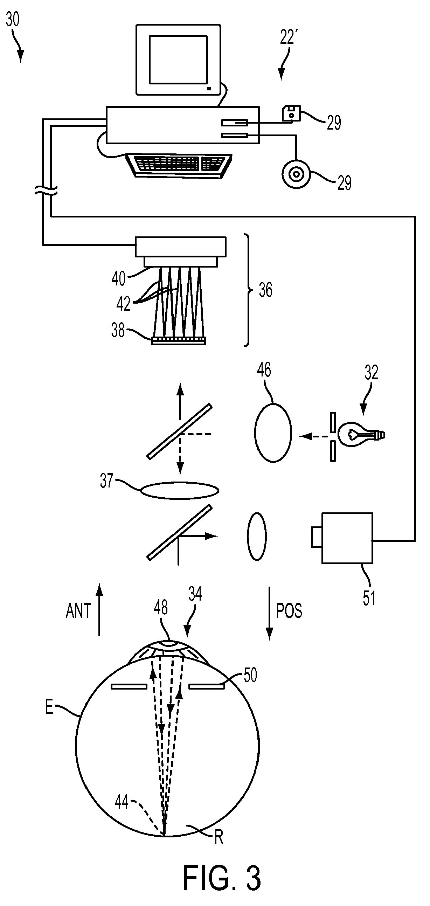

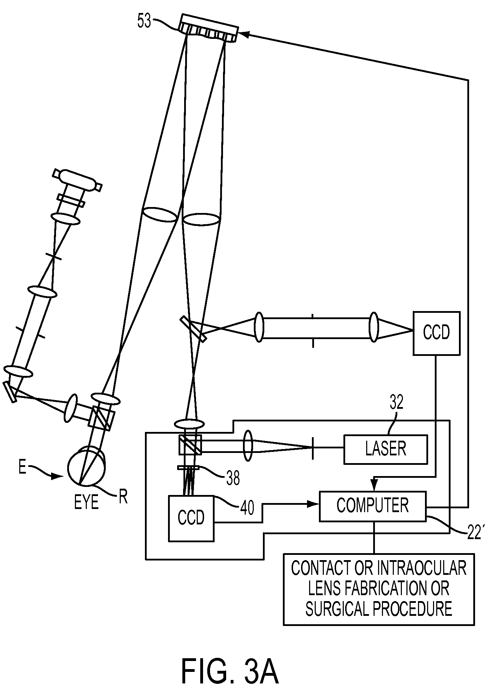

Referring now to **Fig. 3**, one embodiment of a wavefront measurement system 30 is schematically illustrated in simplified form. In very general terms, wavefront measurement system 30 is configured to sense local slopes of a gradient map exiting the patient's eye. Devices based on the Hartmann-Shack principle generally include a lenslet array to sample the gradient map uniformly over an aperture, which is typically the exit pupil of the eye. Thereafter, the local slopes of the gradient map are analyzed so as to reconstruct the wavefront surface or map.

More specifically, one wavefront measurement system 30 includes an image source 32, such as a laser, which projects a source image through optical tissues 34 of eye E so as to form an image 44 upon a surface of retina R. The image from retina R is transmitted by the optical system of the eye (e.g., optical tissues 34) and imaged onto a wavefront sensor 36 by system optics 37. The wavefront sensor 36 communicates signals to a computer system 22' for measurement of the optical errors in the optical tissues 34 and/or determination of an optical tissue ablation treatment program. Computer 22' may include the same or similar hardware as the computer system 22 illustrated in **Figs. 1 and 2.** Computer system 22' may be in communication with computer system 22 that directs the laser surgery system 10, or some or all of the components of computer system 22, 22' of the wavefront measurement system 30 and laser surgery system 10 may be combined or separate. If desired, data from wavefront sensor 36 may be transmitted to a laser computer system 22 via tangible media 29, via an I/O port, via an networking connection 66 such as an intranet or the Internet, or the like.

Wavefront sensor 36 generally comprises a lenslet array 38 and an image sensor 40. As the image from retina R is transmitted through optical tissues 34 and imaged onto a surface of image sensor 40 and an image of the eye pupil P is similarly imaged onto a surface of lenslet array 38, the lenslet array separates the transmitted image into an array of beamlets 42, and (in combination with other optical components of the system) images the separated beamlets on the surface of sensor 40. Sensor 40 typically comprises a charged couple device or "CCD," and senses the characteristics of these individual beamlets, which can be used to determine the characteristics of an associated region of optical tissues 34. In particular, where image 44 comprises a point or small spot of light, a location of the transmitted spot as imaged by a beamlet can directly indicate a local gradient of the associated region of optical tissue.

Eye E generally defines an anterior orientation ANT and a posterior orientation POS. Image source 32 generally projects an image in a posterior orientation through optical tissues 34 onto retina R as indicated in **Fig. 3**. Optical tissues 34 again transmit image 44 from the retina anteriorly toward wavefront sensor 36. Image 44 actually formed on retina R may be distorted by any imperfections in the eye's optical system when the image source is originally transmitted by optical tissues 34. Optionally, image source projection optics 46 may be configured or adapted to decrease any distortion of image 44.

In some embodiments, image source optics 46 may decrease lower order optical errors by compensating for spherical and/or cylindrical errors of optical tissues 34. Higher order optical errors of the optical tissues may also be compensated through the use of an adaptive optic element, such as a deformable mirror (described below). Use of an image source 32 selected to define a point or small spot at image 44 upon retina R may facilitate the analysis of the data provided by wavefront sensor 36. Distortion of image 44 may be limited by transmitting a source image through a central region 48 of optical tissues 34 which is smaller than a pupil 50, as the central portion of the pupil may be less prone to optical errors than the peripheral portion. Regardless of the particular image source structure, it will be generally be beneficial to have a well-defined and accurately formed image 44 on retina R.

In one embodiment, the wavefront data may be stored in a computer readable medium 29 or a memory of the wavefront sensor system 30 in two separate arrays containing the x and y wavefront gradient values obtained from image spot analysis of the Hartmann-Shack sensor images, plus the x and y pupil center offsets from the nominal center of the Hartmann-Shack lenslet array, as measured by the pupil camera 51 (**Fig. 3**) image. Such information contains all the available information on the wavefront error of the eye and is sufficient to reconstruct the wavefront or any portion of it. In such embodiments, there is no need to reprocess the Hartmann-Shack image more than once, and the data space required to store the gradient array is not large. For example, to accommodate an image of a pupil with an 8 mm diameter, an array of a 20 x 20 size (i.e., 400 elements) is often sufficient. As can be appreciated, in other embodiments, the wavefront data may be stored in a memory of the wavefront sensor system in a single array or multiple arrays.

While the methods of the present invention will generally be described with reference to sensing of an image 44, it should be understood that a series of wavefront sensor data readings may be taken. For example, a time series of wavefront data readings may help to provide a more accurate overall determination of the ocular tissue aberrations. As the ocular tissues can vary in shape over a brief period of time, a plurality of temporally separated wavefront sensor measurements can avoid relying on a single snapshot of the optical characteristics as the basis for a refractive correcting procedure. Still further alternatives are also available, including taking wavefront sensor data of the eye with the eye in differing configurations, positions, and/or orientations. For example, a patient will often help maintain alignment of the eye with wavefront measurement system 30 by focusing on a fixation target, as described in U.S. Patent No. 6,004,313. By varying a position of the fixation target as described in that reference, optical characteristics of the eye may be determined while the eye accommodates or adapts to image a field of view at a varying distance and/or angles.

The location of the optical axis of the eye may be verified by reference to the data provided from a pupil camera 52. In the exemplary embodiment, a pupil camera 52 images pupil 50 so as to determine a position of the pupil for registration of the wavefront sensor data relative to the optical tissues.

An alternative embodiment of a wavefront measurement system is illustrated in **Fig. 3A.** The major components of the system of **Fig. 3A** are similar to those of **Fig. 3**. Additionally, **Fig. 3A** includes an adaptive optical element 53 in the form of a deformable mirror. The source image is reflected from deformable mirror 98 during transmission to retina R, and the deformable mirror is also along the optical path used to form the transmitted image between retina R and imaging sensor 40. Deformable mirror 98 can be controllably deformed by computer system 22 to limit distortion of the image formed on the retina or of subsequent images formed of the images formed on the retina, and may enhance the accuracy of the resultant wavefront data. The structure and use of the system of **Fig. 3A** are more fully described in U.S. Patent No. 6,095,651.

The components of an embodiment of a wavefront measurement system for measuring the eye and ablations may comprise elements of a VISX WaveScan®, available from VISX, INCORPORATED of Santa Clara, California. One embodiment includes a WaveScan® with a deformable mirror as described above. An alternate embodiment of a wavefront measuring system is described in U.S. Patent No. 6,271,915. It is appreciated that any wavefront aberrometer could be employed for use with the present invention.

I. TARGET OPTICAL SURFACE SHAPE

Refractive surgery is typically based on a target optical surface shape that is selected or determined to treat a vision condition in a patient. A target optical surface shape can be based on or represented by any of a variety of target optical surface shape data or data formats. In this context, a vision condition can be analogous to a refractive case. Examples of refractive cases include the following.

| Refractive Case | Optical Zone x Ablation Zone |
| --- | --- |
| 1. Myopic (-4D) | 6mm X 8mm |
| 2. Hyperopic (+2D) | 5mm X 9mm |
| 3. Myopic Astigmatism (-2DS/-1DC X 34°) | 6mm X 8mm |
| 4. Hyperopic Astigmatism (+2DS/-1DC X 65°) | 5mm X 9mm |
| 5. Mixed Astigmatism (+2DS/-3DC X 45°) | 5mm X 9mm |
| 6. Therapeutic (+2.35DS/-3.51DC X 17°) | 6mm X 8mm |

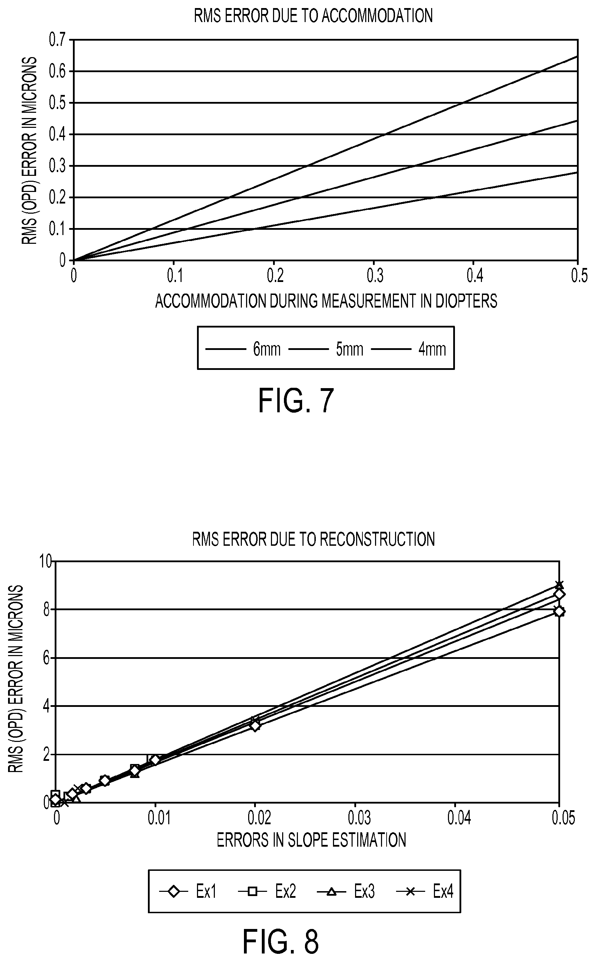

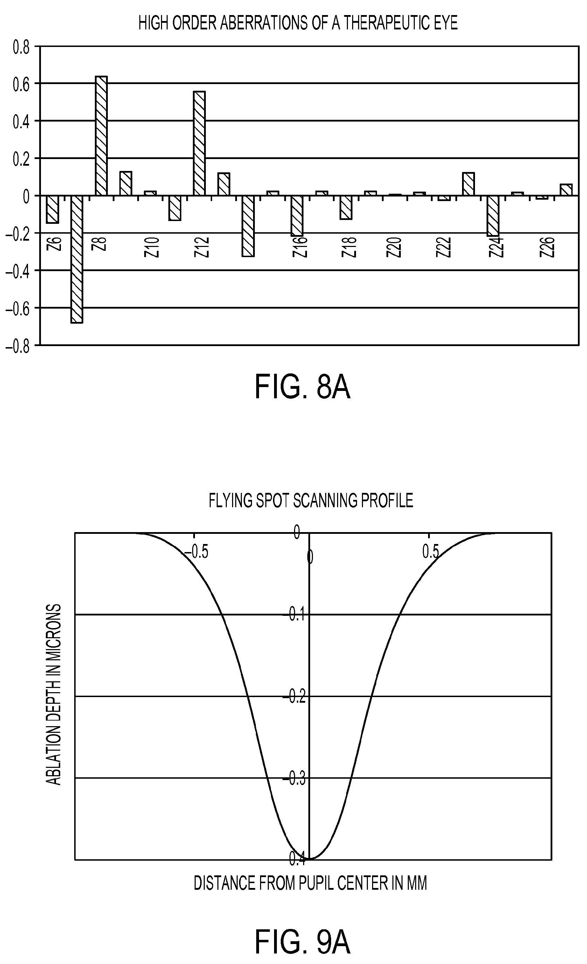

Refractive cases 1 through 5 represent hypothetical refractive cases, and the therapeutic eye of case 6 represents a real eye case having more than a 1 $\mu$m high order aberration RMS with large coma and spherical components (for example a single high order Zernike mode $Z_8^8$, or Z45, with 1 $\mu$m RMS error). The optical zone can be based on a hypothetical pupil diameter. In the real eye case, the optical zone can correspond to a pupil diameter under standard lighting conditions used during wavefront evaluation. The high order part of the exemplary therapeutic eye refractive case is shown in **Fig. 8A.** In one embodiment of the present invention, the target optical surface shape includes a set of 6-order Zernike polynomials.

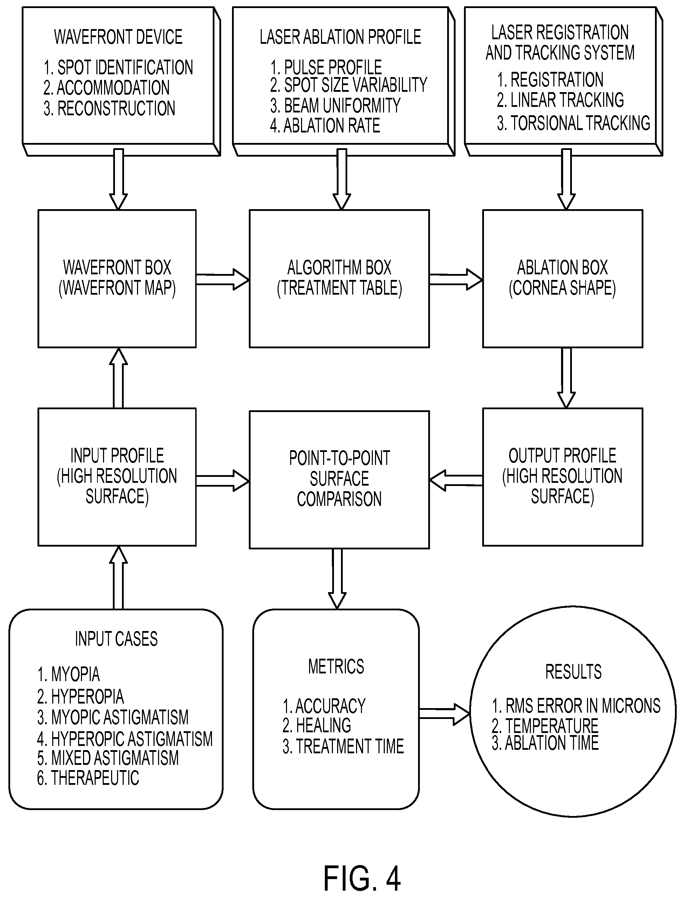

Refractive cases such as these can be determined with a wavefront sensing device, which can determine both low and high order aberrations. In some cases, the target optical surface shape can be configured to address a low order aberration. Some refractive cases may present both low and high order aberrations, and may benefit from a combined target optical surface shape treatment. As shown in **Fig. 4**, given a particular vision condition or refractive case, it is possible to generate a corresponding high resolution target optical surface shape, or input profile, for treating the condition.

II. REFRACTIVE SURGERY SYSTEM PARAMETERS

Given a target optical surface shape, it is possible to determine a model optical surface shape based on the target shape and a set of refractive surgery system parameters. The refractive surgery system parameters correspond to the individual system components of the system. For example, as shown in **Fig. 4**, one embodiment of the refractive surgery system can include components such as a wavefront device, a laser ablation profile, and a laser-servo system such as a laser registration and tracking system. These components can introduce errors into the model optical surface shape. The surgery system can have error sources including, for example, a wavefront device measurement error, a wavefront surface fitting error or algorithm imperfection, a laser beam uniformity and variability error, a registration error, and a tracking error. Thus, the model surface shape can include aberrations that are introduced or amplified by the surgery system parameters, and these aberrations can be described and evaluated by certain mathematical equations.

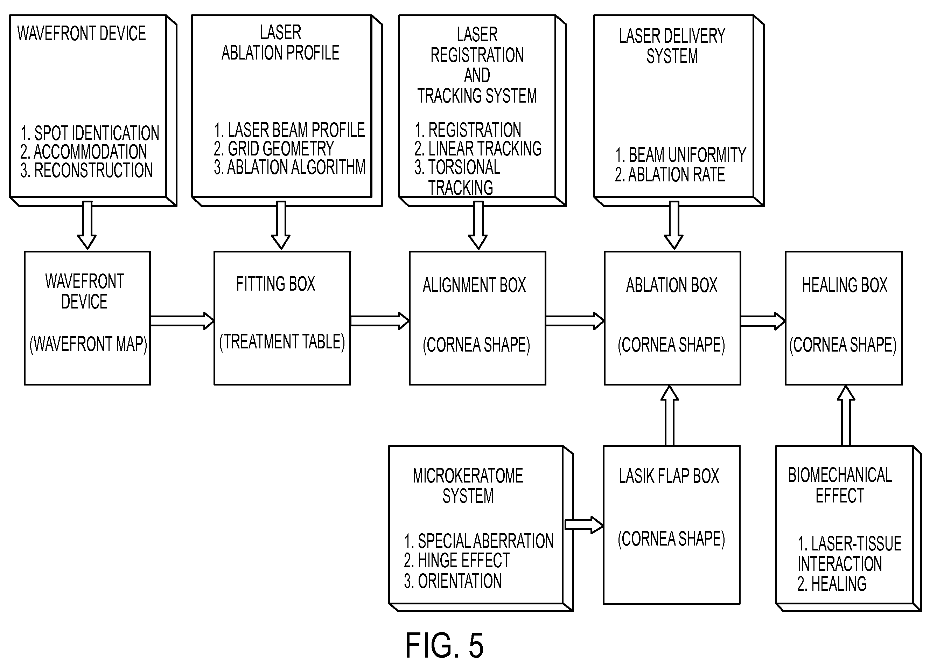

**Fig. 5** illustrates another embodiment of an overall refractive system according to the present invention, which can include components such as a wavefront device, a laser ablation profile, a laser registration and tracking system, and a laser delivery system. Such a refractive surgery system can have error sources including, for example, a wavefront device measurement error, a laser beam profile error, a laser registration and tracking system error, and a laser delivery system error. Accordingly, a set of refractive surgery system parameters can be selected from the group consisting of a wavefront device variable, a laser ablation profile variable, a laser registration and tracking system variable, a biomechanical variable, and a healing effect variable.

As noted above, different components of the refractive surgery system, as represented by the surgery system parameters, can by their own accord introduce different errors or aberrations into the model optical surface shape, and they can exacerbate different errors or aberrations present in the target optical surface shape. Consequently, there may be different RMS values or other error values associated with the different system components. The present invention provides a numerical approach to characterizing or identifying error sources in such a system.

To evaluate the error sources, it is helpful to consider the overall system. Assuming that all of the error sources are statistically independent, the overall error associated with the system embodiment shown in **Fig. 4** can be represented as

$$\Delta = \sigma_{WF}^2 + \sigma_{AB}^2 + \sigma_{RT}^2 \qquad (1)$$

where $\sigma_{WF}^2$ represents a WF (wavefront) measurement induced error or variance, $\sigma_{AB}^2$ represents an ablation profile related variance or fitting error, and $\sigma_{RT}^2$ represents a laser system registration and tracking error or variance. This total error is a representation of the system source errors that can contribute aberrations to a model optical surface shape.

In another example, the total error associated with the surgical system parameters can be written as

$$\sigma_{total}^2 = \sigma_w^2 + \sigma_f^2 + \sigma_r^2 + \sigma_t^2 + \sigma_b^2 \qquad (2)$$

where $\sigma_w^2$ represents a measurement error in the wavefront device, $\sigma_f^2$ represents an error induced in surface fitting with respect to a certain algorithm such as a simulated annealing algorithm, $\sigma_r^2$ represents an error induced by registration, $\sigma_t^2$ represents a tracking error, and $\sigma_b^2$ represents an error due to laser beam uniformity and variability.

As shown in **Fig. 5,** another exemplary surgical system can include a wavefront device, a laser ablation profile, a laser registration and tracking component, and a laser delivery system. As indicated in the figure, errors introduced by a microkeratome can also be factored into the total system error analysis. When assuming that all the error sources are statistically independent, the overall error can be represented as

$$\sigma_{total}^2 = H(\sigma_w^2 + \sigma_f^2 + \sigma_r^2 + \sigma_t^2 + \sigma_b^2 + \sigma_m^2), \qquad (3)$$

where $H(.)$ represents a non-linear healing operator, $\sigma_w^2$ represents a total error in the wavefront device, $\sigma_f^2$ represents an error induced in surface fitting with respect to a certain algorithm such as a simulated annealing algorithm, $\sigma_r^2$ represents an error induced by the registration, $\sigma_t^2$ represents a tracking error, $\sigma_b^2$ represents an error due to laser beam uniformity and variability, and $\sigma_m^2$ represents an error induced from the LASIK flap, or biomechanical effect. The individual error sources are discussed in further detail below.

In some embodiments, the set of refractive surgery system parameters can be adjusted such that a post-operative total high order RMS is substantially equivalent to a pre-operative total high order RMS. In other embodiments, the set of refractive surgery system parameters can be adjusted such that a post-operative total high order RMS is less than a pre-operative total high order RMS. In still other embodiments, the set of refractive surgery system parameters can be adjusted such that a post-operative total high order RMS is about one third the amount of a pre-operative total high order RMS.

The set of refractive surgery system parameters can be adjusted such that a post-operative total high order RMS of about 0.1 $\mu$m to about 0.3 $\mu$m is achieved. In related embodiments, the set of refractive surgery system parameters can be adjusted such that each system component of the total high order RMS does not exceed from about 0.038 $\mu$m to about 0.113 $\mu$m. In other embodiments, where the total RMS error is about 0.1 $\mu$m to about 0.3 $\mu$m and the system includes 3 components, the set of refractive surgery system parameters can be adjusted such that each system component of the total high order RMS does not exceed from about 0.0577 $\mu$m to about 0.173 $\mu$m. In yet other embodiments, where the total RMS error is about 0.1 $\mu$m to about 0.3 $\mu$m and the system includes 10 components, the set of refractive surgery system parameters can be adjusted such that each system component of the total high order RMS does not exceed from about 0.0316 $\mu$m to about 0.0949 $\mu$m.

In one embodiment of the present invention, the target optical surface shape includes a set of 6-order Zernike polynomials, and the set of refractive surgery system parameters is adjusted such that each component of a post-operative total high order RMS does not exceed about 0.022 $\mu$m. In another embodiment, the target optical surface shape includes a set of 6-order Zernike polynomials, and the set of refractive surgery system parameters is adjusted such that each component of a post-operative total high order RMS does not exceed about 0.0073 $\mu$m. In other embodiments, where the total RMS error is about 0.1 $\mu$m to about 0.3 $\mu$m and the system includes 3 components, the set of refractive surgery system parameters can be adjusted such that each system component of the total high order RMS does not exceed from about 0.0111 $\mu$m to about 0.0333 $\mu$m. In yet other embodiments, where the total RMS error is about 0.1 $\mu$m to about 0.3 $\mu$m and the system includes 10 components, the set of refractive surgery system parameters can be adjusted such that each system component of the total high order RMS does not exceed from about 0.0061 $\mu$m to about 0.0111 $\mu$m.

A. Wavefront Device Parameters

A wavefront device measurement error, which can be represented as $\sigma_w^2$, can originate from errors associated with any of a variety of parameters. For example, as shown in **Figs. 4 and 5,** a wavefront device can include parameters such as spot identification (e.g. Hartmann-Shack spot pattern identification), accommodation, and reconstruction. Accordingly, a wavefront device variable can be selected from the group consisting of a spot identification factor, an accommodation factor, and a reconstruction factor. In some cases, the wavefront device variable is configured to address a high order aberration.

1. Accommodation Error

Accommodation error can be due to partial accommodation or micro-accommodation of the patient, which can be translated to a root mean squares (RMS) error. Micro-fluctuation, or accommodation drift, can be present in a patient as they gaze at, but are unable to fixate upon, a distant target. Most patients will accommodate at least slightly, and micro-accommodation corresponds to slight changes in the relaxation of accommodation. To the extent a patient cannot fully relax and therefore accommodates during this procedure, this accommodation can become part of the error. Assuming the random error of accommodation is $a$ for an eye with pupil radius of $R$, an RMS accommodation error can be expressed as

$$\sigma_{ac}^2 = \frac{a^2 R^4}{48}, \qquad\qquad (4)$$

where $a$ is given in diopters and R is given in mm. $a$ can represent a variable averaged microaccommodation for several patients being measured.

In a practical clinical setting, it may be difficult to keep the accommodation drift, or accommodation error, under 0.1D. In some cases, accommodation error of more than one diopter has been observed. Taking 0.1D as a limit, the minimum RMS accommodation error for a 6mm pupil is then 0.15 $\mu$m. In some embodiments, the wavefront device variable includes an accommodation error of 0.25D, equivalent to 0.325 $\mu$m RMS error for a 6mm pupil.

For the accommodation error, **Fig. 7** shows the contribution of the accommodation error to the total RMS accommodation error for different pupil sizes. It is clear to see that larger pupil sizes correspond with larger total RMS errors when the amount of accommodation remains constant.

2. Reconstruction Error

For the wavefront device error, it is also possible to consider error induced from a wavefront reconstruction error. The sources of a reconstruction error can include an uncompensated error due to truncation of the number of basis functions, such as Zernike polynomials; a measurement error; and a remaining error due to aliasing of the derivatives of basis functions. A complete theoretical analysis is given by Dai, in "Modal wave-front reconstruction with Zernike polynomials and Karhunen-Loève functions," J. Opt. Soc. Am. A 13, 1218-1225 (1996).

In one embodiment, the reconstruction error can be written as

$$\sigma_{rc}^2 = \sigma_{uc}^2 + \sigma_{sp}^2 + \sigma_{rs}^2 . \tag{5}$$

where $\sigma_{uc}^2$ is this the uncompensated error, $\sigma_{sp}^2$ is this the spot identification error, and $\sigma_{rs}^2$ is this the remaining error.

The uncompensated error may be difficult to estimate due to the lack of statistics of the Zernike expansion for human eye's aberrations. However, a treatment with consideration of the Hartmann-Shack sensor configuration is possible. The measurement error, which is often directly related to spot identification, can be treated as spot identification error. Finally, the remaining error can be small, especially when the number of sub-apertures is relatively small. For example, in one embodiment the VISX WaveScan® device uses 37 sub-apertures.

For a wavefront reconstruction error, it is possible to assume that different error sources result in the final uncertainty in a slope estimate. These error sources include CCD detector noise, noise in pixel round-off position error, as well as error contained in the reconstruction algorithm, and these could affect spot identification error and reconstruction error. **Fig. 8** shows the contribution of slope estimation error to the total RMS error for four example cases, illustrating that an error in slope estimation can affect the total RMS error. In some embodiments, the wavefront reconstruction error can be about 0.05 μm.

A spot identification error can be an error due to round off of pixel position (integer pixel position), low contrast spots due to corneal reflection, or low signal to noise (S/N) ratio. A complete theoretical derivation of the total spot identification error is not given here. It is possible to use a simple Gaussian random noise model for the simulation of spot identification error. However, a general formulation can be given as

$$\sigma_{sp}^2 = \sigma_{ro}^2 + \sigma_{sn}^2 . \tag{6}$$

where $\sigma_{ro}^2$ is this the round off error and $\sigma_{sn}^2$ is this the signal to noise ratio error. In some embodiments, the spot identification error can be about 0.05 μm.

3. Total Wavefront Device Error

The total wavefront device error can be written as

$$\sigma_{w}^2 = \sigma_{rc}^2 + \sigma_{ac}^2 . \tag{7}$$

which in some embodiments is the final formula for RMS calculation for a wavefront device, and reflects the sum of the accommodation error and the reconstruction error.

In one embodiment, a reconstruction error can reflect a typical slope estimation error of 0.001 corresponding to an RMS reconstruction error of about 0.2 μm. In this embodiment, the total RMS error from a wavefront device can be at the order of 0.25 μm, assuming an RMS accommodation error of 0.1 μm.

A large portion of the wavefront device error may often be manifested as errors in low orders, or mostly in the sphere error. Therefore, the end result, or true ablation (e.g. the model optical surface shape) may be a random over correction or under correction. If the total RMS wavefront device error is entirely a low order aberration, it may correspond to a 0.2D refractive error, which can be considered as small. The statistical trend, though, would result in relatively small total RMS wavefront device error. The truly induced high order total RMS error, which typically originates from the system parameters, will often be below 0.1 $\mu$m.

One approach to correcting or inhibiting high order aberrations involves controlling the overall wavefront error to a certain limit. For instance, using a 100 $\mu$m scanning resolution of a Gaussian spot (FWHM = 0.75 mm) to correct a -4DS eye without high order aberrations can induce 0.21 $\mu$m high order aberration (HOA). In some embodiments, the wavefront device variable includes a 100 $\mu$m gridsize factor.

B. Laser Ablation Profile Parameters

Laser ablation profile errors are sometimes referred to as wavefront surface fitting errors, or algorithm errors. Wavefront surface fitting errors can be the result of a numerical solution of a multi-dimensional problem in fitting individual laser pulses to the expected wavefront surface, or model optical surface shape. Laser ablation profile errors, which can be represented as $\sigma_f^2$, can originate from errors associated with any of a variety of parameters. Accordingly, a laser ablation profile variable can include a pulse size factor, a spot size variability factor, a beam uniformity factor, and a laser pulse repetition rate factor.

In one embodiment of the present invention, as shown in **Fig. 4,** a laser ablation profile can include parameters such as pulse profile, spot size variability, beam uniformity, and laser pulse repetition rate. In another embodiment of the present invention, as shown in **Fig. 5,** a laser ablation profile can include parameters such as laser beam profile, grid geometry, and ablation algorithm. In **Fig. 5,** the beam uniformity and laser pulse repetition rate are characterized as laser delivery system parameters, and are further discussed under that section heading below.

1. Laser Pulse Profile Fitting Errors

Laser ablation pulse profiles can be generated in a variety of ways. In the following examples, the Y(r) function describes how to generate the ablation pulse profile, where $\sigma$ represents the standard deviation of a Gaussian profile, and FWHM represents the full width at half maximum of the Gaussian profile. Different types of pulse profiles can contribute different amounts of error to the laser ablation profile error. For example, a laser ablation profile variable can include a variable spot scanning factor or a flying spot scanning error.

a. Flying Spot Scanning (FSS) Pulse Profile

A Flying Spot Scanning (FSS) pulse profile can be represented by the following formula:

$$Y(r) = -0.4 \exp \left[ (-8\ln2/\sigma^2)(4-r)^2 \right] \qquad (8)$$

where $\sigma$ = D/2 and D is the spot size, and where FWHM is D/$\sqrt{8}$ = 0.3536D. Y(r) represents the ablation depth, and r represents the distance from the pupil center, in mm. Thus, for a 0.75 mm FWHM spot, D = 2 mm. This profile is depicted in **Fig. 9A.**

In one embodiment, a -4 Diopter input is used to generate the basis function, or basis data, for a Flying Spot Scanning profile. The following results were obtained: 5481 pulses, PV = 1.03 $\mu$m, RMS = 0.14 $\mu$m (OPD), which is the profile fitting error. All measurements are in optical path difference (OPD). PV is a peak to valley measurement, and represents the difference between maximum and minimum in sequence of values. It reflects the magnitude of fluctuation. The RMS is similar to standard deviation. In some embodiments, the flying spot scanning factor can be about 1.5 mm.

b. Variable Spot Scanning (VSS) Pulse Profile

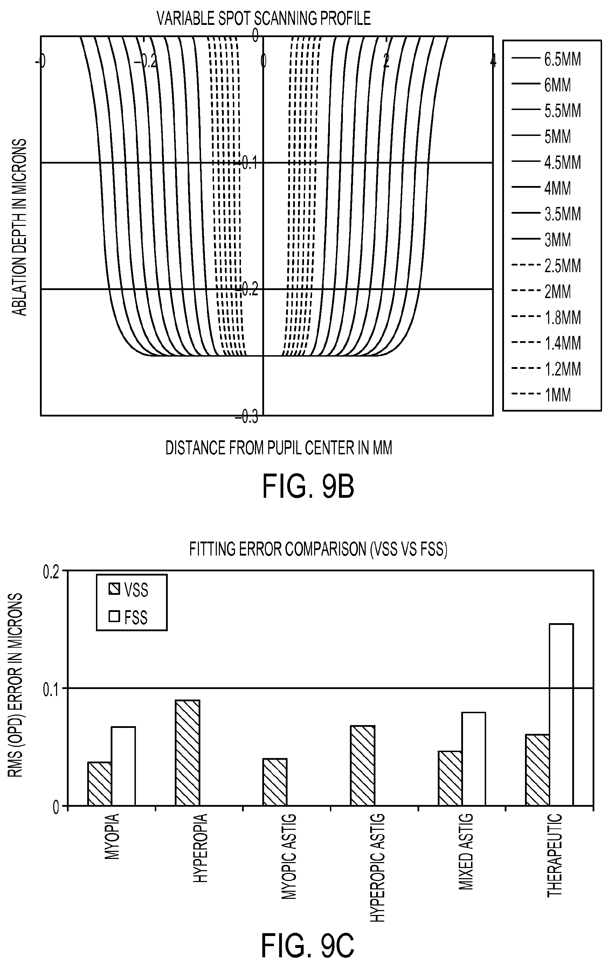

In a Variable Spot Scanning (VSS) profile laser, a top hat shape can be used. This profile is depicted in **Fig. 9B,** at 15 different diameters. In the -4 Diopter input embodiment describe above, for the VISX Variable Spot Scanning profile, the following results were obtained: 339 pulses, PV = 0.78 $\mu$m, RMS = 0.11 $\mu$m, which is the profile fitting error. All measurements are in optical path difference (OPD).

c. Comparison Between Various Pulse Profiles

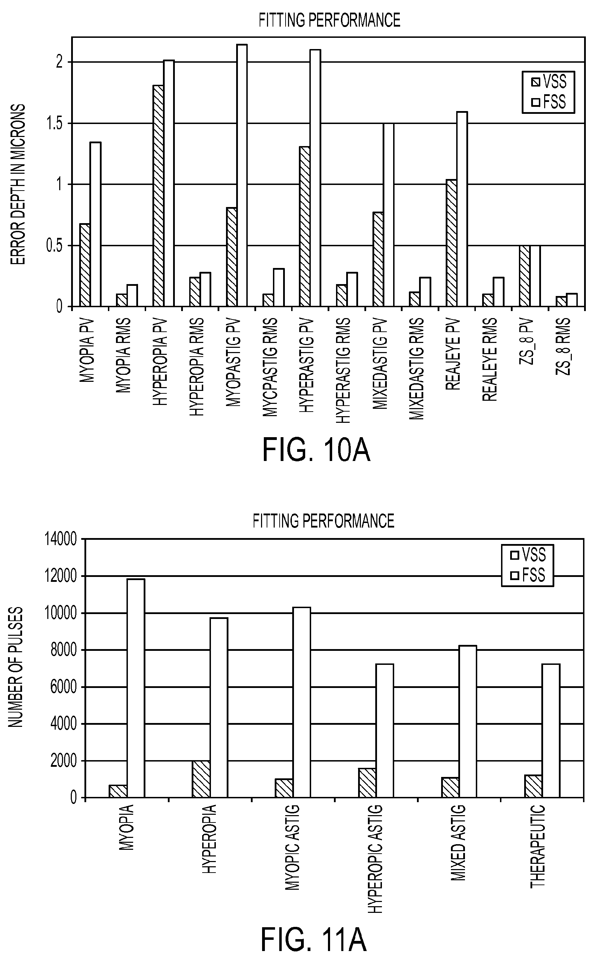

The fitting errors, or $\sigma_f^2$, for the following laser pulse profiles were evaluated with respect to a variety of refractive cases, and the results are shown in **Fig. 10A.** This assumes that all other laser ablation profile parameters, such as spot size variability and grid geometry, were equal, and there were no other error sources.

## 2. Spot Size Variability Errors

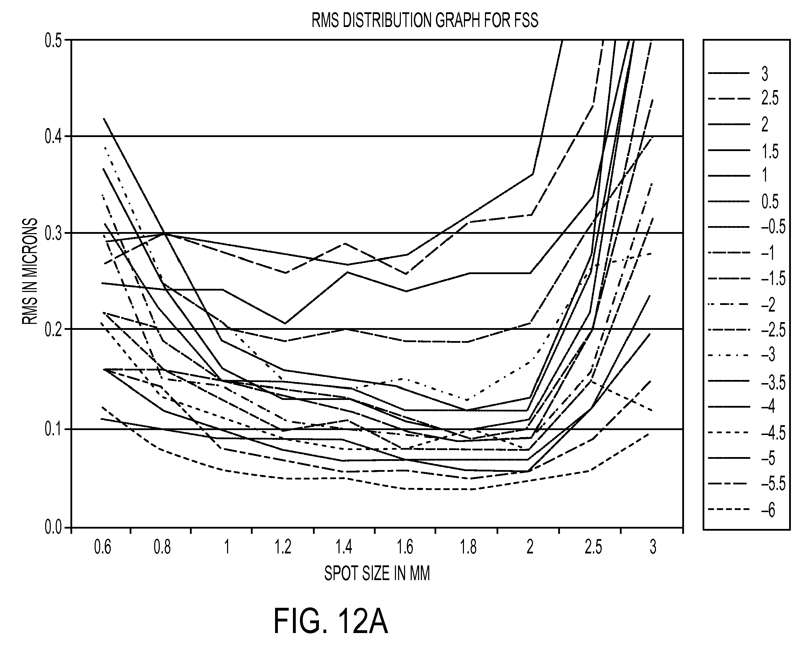

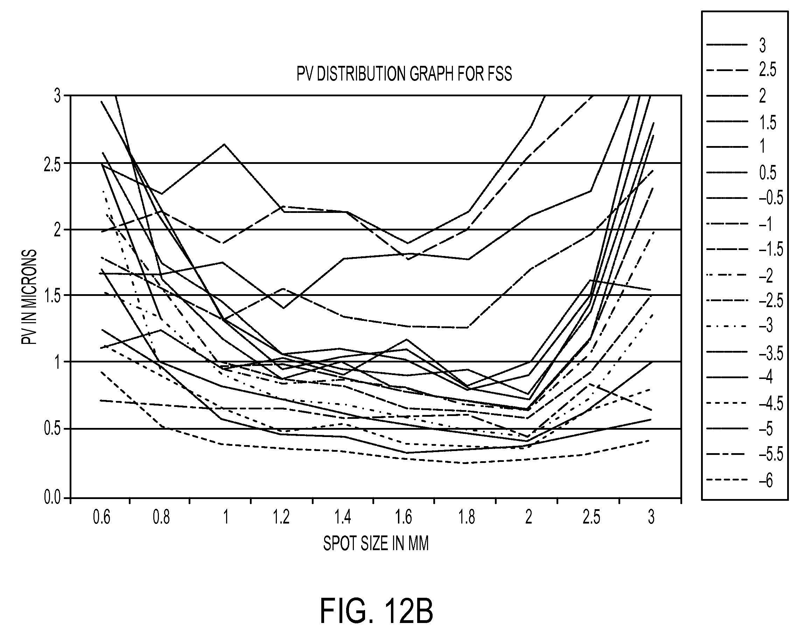

A spot size variability error can also contribute to a laser ablation profile error. **Fig. 12A** illustrates an RMS Distribution Graph, showing the fitting RMS error for several different refractive cases (refractive powers) at various spot sizes. **Fig. 12B** illustrates a PV Distribution Graph. The units on the x-axis represent the spot size diameter in millimeters. Based on the example shown in these figures, an optimal spot size (i.e. lowest error) for Flying Spot Scanning (FSS) can range from about 1.0 mm to about 1.6mm, and more specifically can be about 1.5 mm or about 0.5 mm FWHM. The FWHM is often about one third of the spot size. In this way, an optimal spot size can be determined for each refractive case, which can confer the maximum inhibition of aberration in model optical surface shape. In this way, it is possible to control the amount of error by controlling the spot size.

A simple spherical refraction is shown in **Figs. 12A and 12B.** The RMS and PV change may not be very significant over a big range (e.g. +3D to -6D), except possibly for the low refraction cases as shown in these examples. The optimal spot size may not appear to change with refractions, again except for very low refraction cases such as, for example, $\pm 0.5$D or cases close to emmetropia.

The VSS spot can range between 0.65 mm and 6.5 mm. Though only discrete number of spots are shown, it should be recognized that the spot sizes can be continuous. VSS can have an ablation depth of about 0.25 $\mu$m (tissue) while the FSS can have an optimal spot size of 1.5 mm with 0.5 mm FWHM and 0.4 $\mu$m depth Gaussian profile. Often, there is no variability of the FSS spot size, meaning that the spot size can be fixed. In some embodiments, the laser ablation profile variable includes a flying spot scanning factor ranging from about 1 mm to about 1.6 mm.

## 3. Grid Geometry

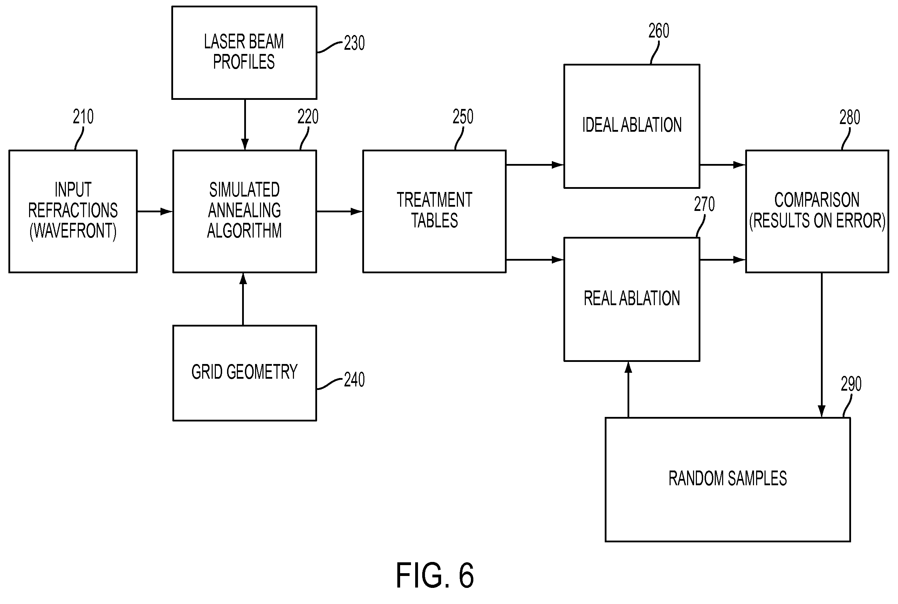

See **Fig. 6,** reference number 240. Grid geometry decides the solution space for the simulated annealing algorithm. In some embodiments, the wavefront device variable may include a 100 $\mu$m gridsize factor.

## 4. Ablation Algorithm

It is also possible to use fitting performance, which is related to the fitting error, to determine the number of ablation pulses employed to perform the ablation, as shown in **Fig. 11A.** In this example, the Flying Spot Scanning profile can have about 10,000 pulses, for example, for each of the refractive cases, whereas the Variable Spot Scanning profile can have less than about 1,000. Consequently, in this example if VSS operates at a laser pulse repetition rate of 20 Hz it takes about 50 seconds for the ablation. To perform an FSS ablation in the same amount of time, the system should operate at a laser pulse repetition rate of 200 Hz.

An analysis of fitting error comparison of all 6 exemplary profile cases in shown in **Fig. 9C,** including fitting (algorithm), registration, tracking and beam uniformity. These 6 cases can be evaluated for RMS error with VSS and FSS being the two sets of beams. Laser pulse repetition rate for VSS is 10 Hz and FSS is 100 Hz. Based on **Fig. 9C,** it is clear that VSS is superior to FSS in all of the six refractive cases, based on the RMS error, with therapeutic and myopic astigmatism having the biggest gain, or difference between VSS and FSS.

Suitable ablation algorithm approaches are also discussed in US patent number 6,673,062, issued January 6, 2004.

## C. Laser Registration and Tracking System Parameters

As shown in **Figs. 4 and 5, a** laser registration and tracking system can include parameters corresponding to registration source errors ($\sigma_r^2$), and linear and torsional tracking source errors ($\sigma_t^2$). Accordingly, a laser registration and tracking system variable may be selected from the group consisting of a registration factor, a linear tracking factor, and a torsional tracking factor. In one embodiment, the laser registration and tracking system variable can include a registration accuracy less than about 10 $\mu$m in both the vertical and horizontal directions and a rotational error less than about 0.5°.

## 1. Registration Error

Registration error, position error, and rotational alignment error can be modeled or determined. The registration error is typically the sum of the position error and the rotational alignment error. In general, the pupil center at the time the wavefront is taken with an aberrometer or phoropter can differ from the pupil center at the time the treatment procedure is performed. This shift in pupil center position error can induce a high order aberration. Similarly, rotational alignment error can also contribute to an increase in high order aberrations. Further, pupil size changes and rotational or alignment errors can also contribute to aberrations. These aberrations can be modeled as randomly distributed errors.

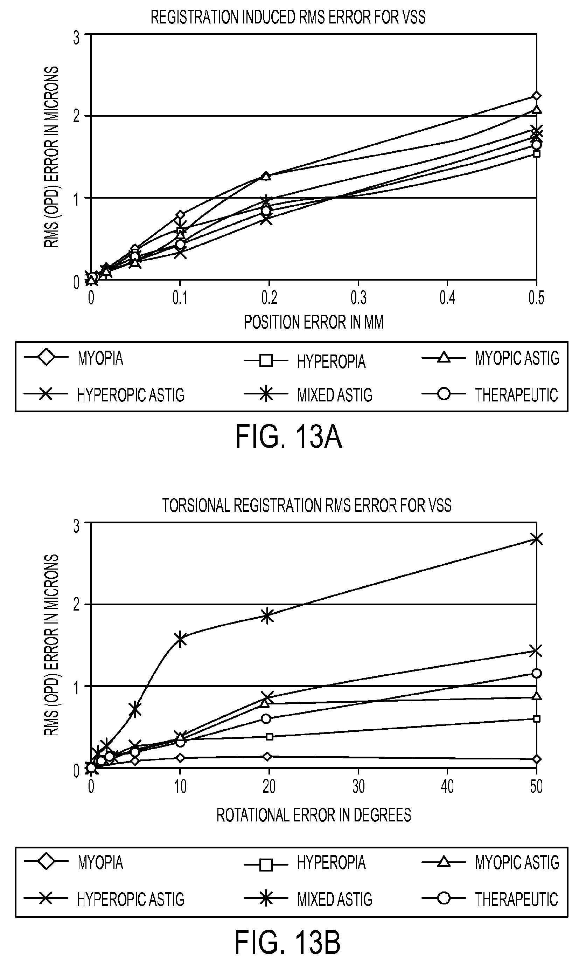

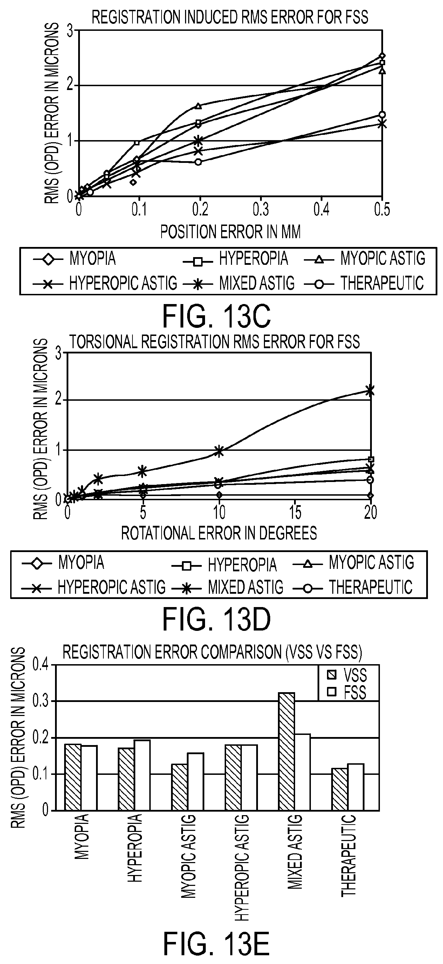

**Figs. 13A-E** illustrate registration error analysis. For linear registration induced RMS error, VSS and FSS appear to perform similarly, as shown in **Figs. 13A** and **13C,** which depict linear, or X/Y, registration. VSS can be volumnable (larger) on mixed astigmatism. For the comparison, values of 20 $\mu$m accuracy in position and 5° in the rotational alignment can be used. In the case of a cylinder with a large angle, VSS registration can be larger than FSS registration, but theoretically, if there is no fitting error, they may be similar. **Figs. 13B and 13D** depict VSS and FSS torsional registration induced RMS error, and based on these figures, torsional registration appears to be correlated to the refractive case. VSS and FSS torsional registration induced RMS error appear to behave similarly, and for mixed astigmatism, VSS appears to exhibit slightly more error which may be due to mixed astigmatism having the least symmetric profile. **Fig. 13E** shows a comparison between VSS and FSS for the combined linear and torsional registration error. Again, VSS and FSS combined linear and torsional registration induced RMS error appear to behave similarly, and for mixed astigmatism, VSS appears to exhibit slightly more error which may be due to mixed astigmatism having the least symmetric profile.

2. Linear and Torsional Tracking Errors

It may be desirable to keep the center of the pupil tracked during the whole course of ablation as the eye moves vertically, horizontally, and cyclo-rotationally. Thus, the tracking component can account for vertical, horizontal, and cyclo-torsional tracking. Parameters such as eye motion speed, duration of each motion, tracking speed, tracking accuracy, and the system latency time can be considered.

It can be helpful to evaluate eye motion during visual fixation. Without fixation, eye movement can be large. During fixation, the eye can undergo micro-motion, which can be modeled as a set of random walks with fixed speed and duration. This is often true for linear motion, although for torsional movement a similar approach can also be applied in terms of an error source parameter. In some cases, the linear motion can have a speed of about 5°-10°/second, or about 20 $\mu$m with duration of about 22 ms. Hence, a grid size of 1001 X 1001 can be used because the grid spacing will be 10 $\mu$m, which represents the distance between each location. The amount of torsional movement can be, for example, between 0.4°$\pm$0.3°.

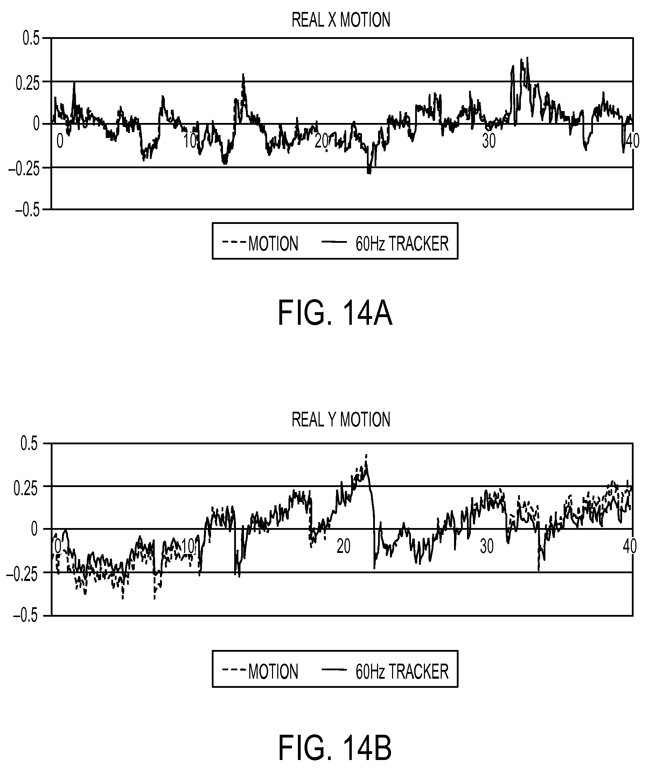

45 real eye motions were captured with a 200 Hz eye motion camera and a 60 Hz VISX Eye Tracker. **Figs. 14A and 14B** show the X and Y real motion of the 19th OS. For eye Y motion, the tracker can have tracking error ranging from about 0.5 mm to about 1.0 mm. Spectral analysis can provide another approach to eye motion analysis. In some embodiments, tracking can compensate for a certain amount of eye motion, and the uncompensated motion can contribute to a high order aberration.

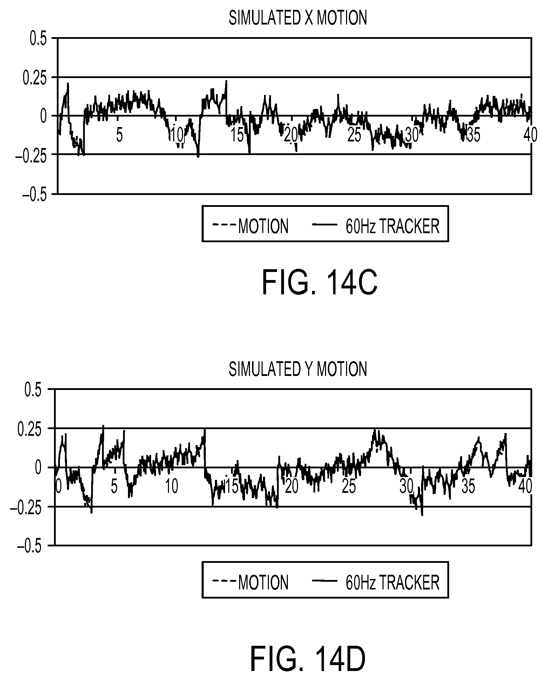

Types of eye movement include (a) saccadic motion, (b) smooth pursuit, (c) tremor, and (d) nystagmus. From the example shown in **Figs. 14A and 14B** mostly smooth pursuit and saccadic motion is observed. The standard deviation can be about 0.1 mm. There are zones of deviation that are present toward the left and right side of the graphs, where the error is more easily observed. **Figs. 14C and 14D** show simulated eye movement examples that are comparable to real eye movement shown in **Figs. 14A and 14B.**

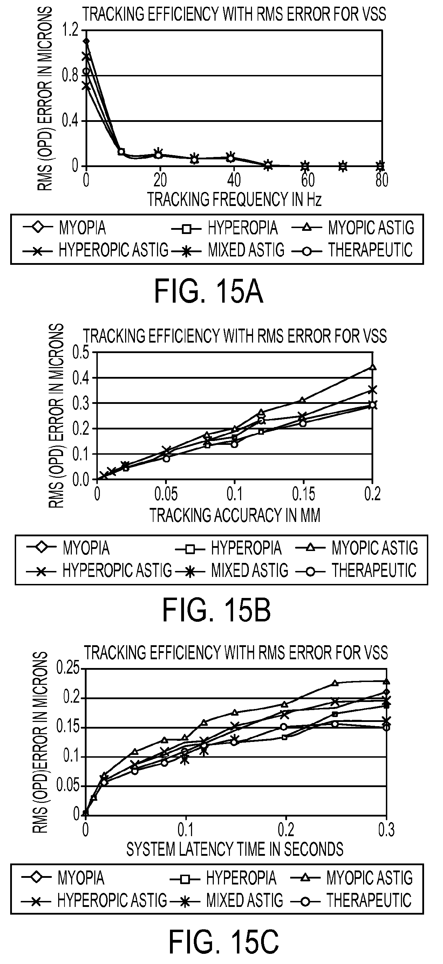

**Figs. 15A - 15F** illustrate a comparison of VSS and FSS observed tracking efficiency. Tracking efficiency can be based on tracking speed, tracking accuracy, and system latency time. A model for eye tracking in the X-Y (or vertical/horizontal) direction can be constructed, based on the following input parameters:

| parameter | description |
|---|---|
| num | number of treatment |
| gridsize | grid size for wavefront |
| speed | eye movement (mm/s) |
| duration | eye movement time (s) |
| laser pulse repetition rate | 10 Hz VSS |
|  | 100 Hz FSS |
| tracking rate | tracking speed (Hz) |
| tracking error | accuracy (mm) |
| system latency time | how fast system responds (s) |

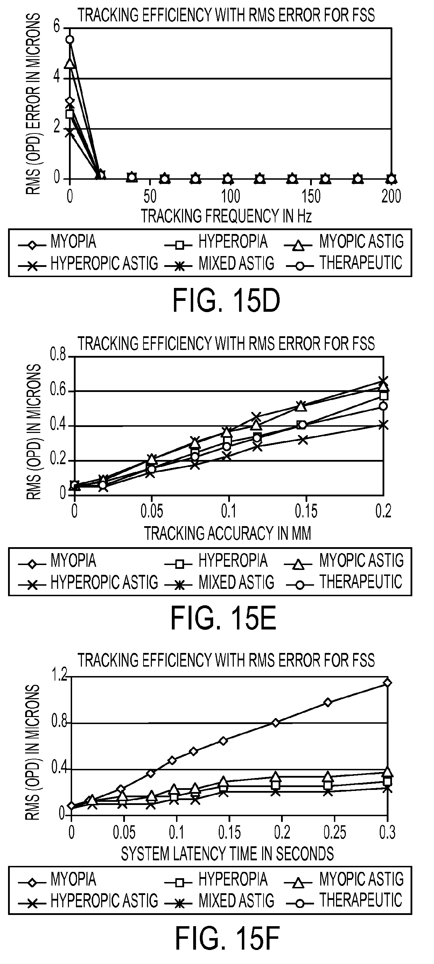

In one example, a 2 mm/s eye speed and 0.022s duration were used. According to the results, the tracking speed can be exponential (**Figs. 15A and 15D**), the tracking error can be linear (**Figs. 15B and 15E**), and the latency time can be quadratic (**Figs. 15C and 15F**). In **Figs. 15A and 15D,** no tracking accuracy and system latency errors were assumed. In **Figs. 15B and 15E,** no tracking speed and system latency errors were assumed. In **Figs. 15C and 15F,** no tracking speed and tracking accuracy errors were assumed.

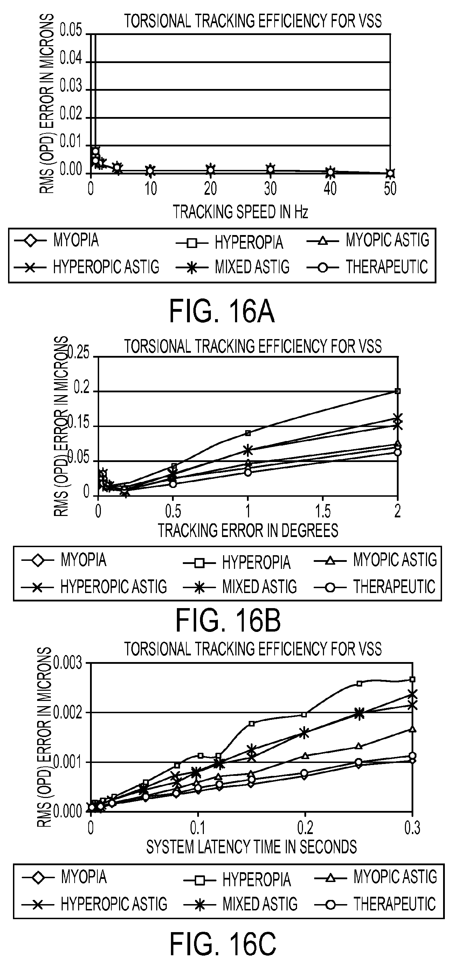

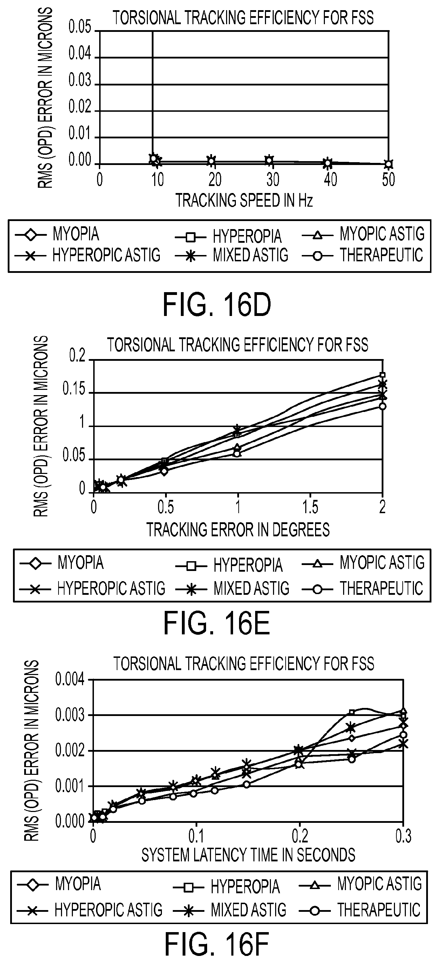

**Figs. 16A - 16F** show a comparison of torsional tracking efficiency between VSS and FSS. Based on this data, torsional tracking error values appear to be lower than horizontal and vertical tracking error values. Overall, the linear tracking appears to be one magnitude larger than torsional tracking error. However, when the torsional tracker is not on, the error is larger, especially for FSS. In general, the tracker error is about twice as large for FSS, as compared to VSS.

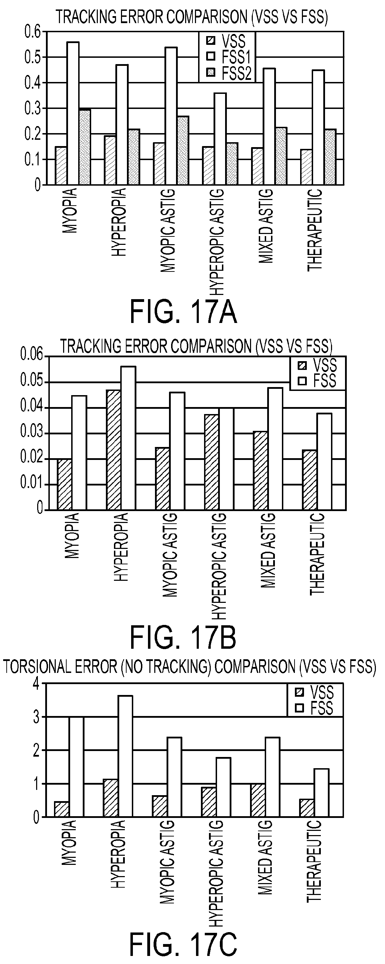

**Figs. 17A-C** show linear and torsional tracking error comparisons between VSS and FSS. For linear tracking, 60 Hz tracking for FSS1, VSS as well as for torsional was used, and 120 Hz was used for FSS2. This example also used 2 mm/s with 0.022 ms duration for linear eye movement, 0.1 °/s with 5 s duration for torsional movement. Linear 0.05 mm accuracy and 0.1s latency time, torsion 0.5° accuracy and 0.1s latency time values were observed. Based on these figures, it appears that VSS confers lower errors than FSS.

In one embodiment, the laser ablation profile variable includes a variable spot scanning factor, and the laser registration and tracking system variable includes a tracking accuracy less than about 20 $\mu$m in both the vertical and horizontal directions, a latency time less than about 10 ms, and a tracking speed of about 60 Hz or greater. In another embodiment, the laser ablation profile variable includes a flying spot scanning factor, and the laser registration and tracking system variable includes a tracking accuracy less than about 5 $\mu$m in both the vertical and horizontal directions, a latency time less than 5 ms, and a tracking speed of about 200 Hz or greater. Relatedly, the laser ablation profile variable can include a variable spot scanning factor, and the laser registration and tracking system variable can include a cyclo-torsional tracking angular accuracy of about 0.5° or better. Likewise, the laser ablation profile variable can include a flying spot scanning factor, and the laser registration and tracking system variable can include a cyclo-torsional tracking angular accuracy of about 0.25° or better.

In other embodiments, the laser ablation profile variable includes a variable spot scanning factor, and the laser registration and tracking system variable includes a laser energy fluctuation less than about 4%. Similarly, the laser ablation profile variable can include a flying spot scanning factor, and the laser registration and tracking system variable can include a laser energy fluctuation less than about 2%.

D. Laser Delivery System Parameters

As noted above, a laser ablation profile variable can be selected from the group consisting of a pulse size factor, a spot size variability factor, a beam uniformity factor, and a laser pulse repetition rate factor.

1. Beam Uniformity and Variability

Laser beam uniformity source errors can be represented as $\sigma_b^2$, and can result from the laser beam profile deviating from the theoretically claimed shapes due to micro-fluctuations in the energy profile. It is also possible that the laser energy fluctuates over time due to physical or mechanical reasons producing laser beam variability.

For laser beam uniformity, the laser energy can be fluctuating during ablation. This energy fluctuation can cause deviation of ablation depth in each laser pulse. This deviation can eventually translate to high order RMS errors. Typically, laser beam uniformity is not dependent on the laser pulse repetition rate.

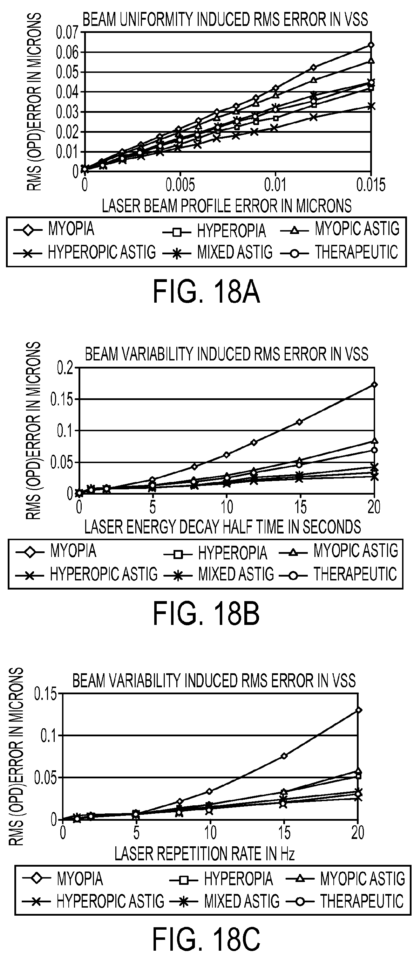

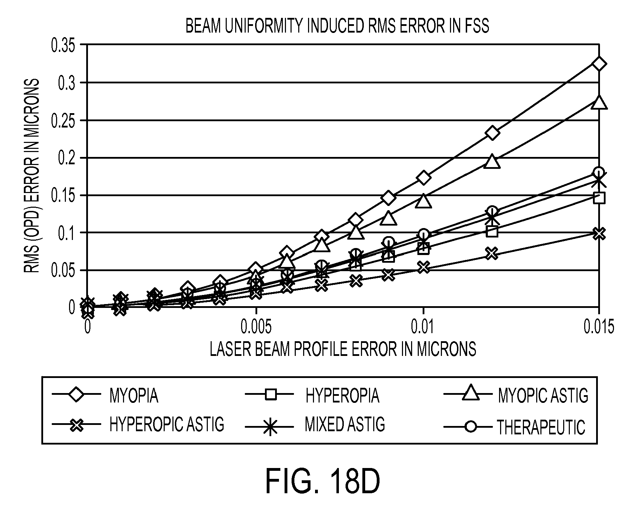

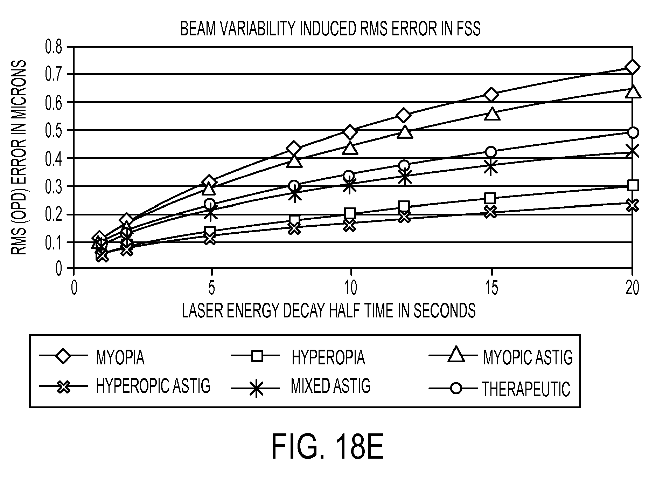

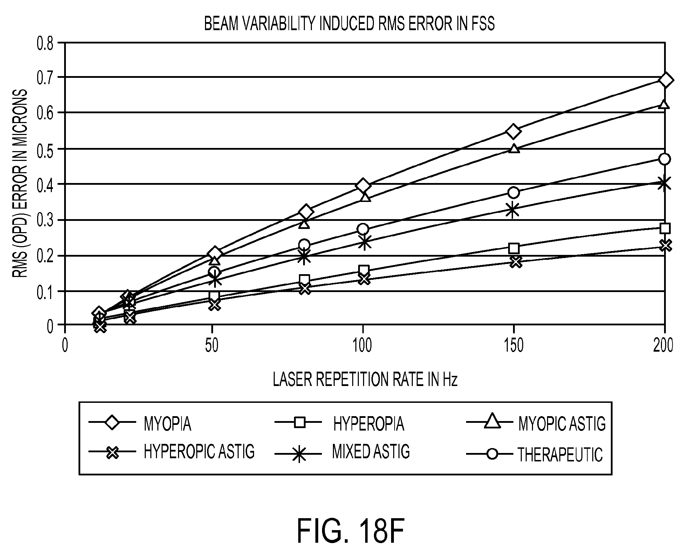

**Figs. 18A and 18D** show laser beam uniformity analysis, and **Figs. 18B, 18C, 18E, and 18F** show laser beam variability analysis. Micro-fluctuations and variability may be due to laser energy decay because of ozone formation. **Fig. 18A** considers uniformity only, without decay. Often, the first 20 seconds the laser is pulsed are considered when determining whether high order aberrations are induced. Exponential time decay (half time) can be about 7 seconds. Two factors to consider are decay half time and laser pulse repetition rate. Laser beam variability may be dependent on laser pulse repetition rate.

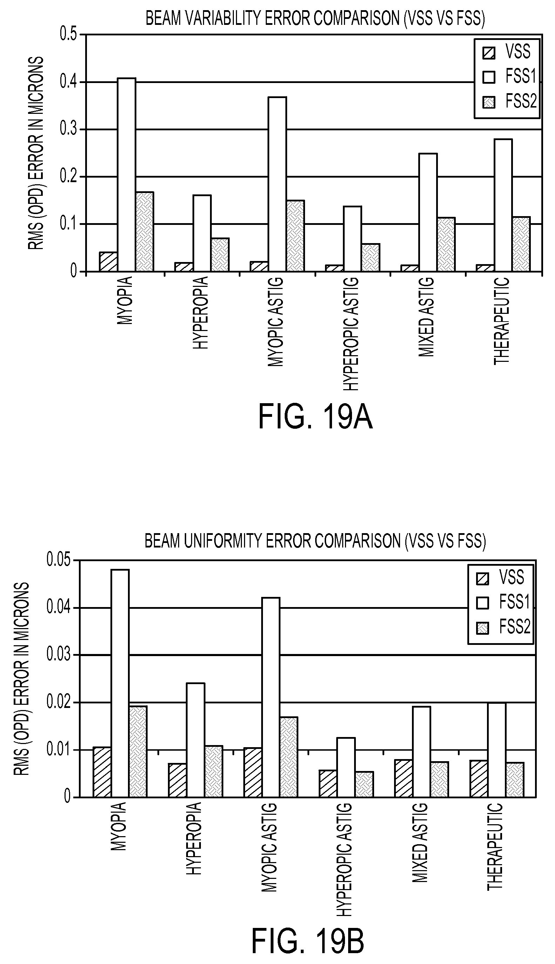

**Figs. 19A and 19B** are derived from known uniformity and variability calculations. Such analysis can include factors such as ozone buildup, laser decay, and uniformity change, which can cause or amplify high order aberrations and/or under-correction. For the VSS case, a 7 second decay half time is assumed, as is the case for FSS1. For FSS2 the decay half time is 2 seconds. For uniformity, $\pm 1\%$ error in laser energy is assumed for VSS. Apparently, the error induced by laser energy micro-fluctuation is much smaller. For uniformity for FSS, FSS1 used $\pm 1\%$ error in laser energy while FSS 2 used $\pm 0.5\%$ error. Laser beam uniformity and variability are further discussed in US patent application number 60/553,580, filed March 15, 2004.

2. Laser Pulse Repetition Rate

Another laser ablation profile variable is the laser pulse repetition rate factor. In some VSS embodiments, the laser pulse repetition rate can range from 10 Hz to about 20Hz. In some FSS embodiments, the laser pulse repetition rate can range from about 100 Hz to about 200 Hz.

### E. Microkeratome Parameters

Microkeratome source errors can be represented as $\sigma_m^2$, and can result from aberrations associated with, for example, a LASIK flap. The LASIK flap is typically generated from a LASIK procedures, but not PRK or LASEK. The LASIK flap may tend to induce spherical aberrations and coma, and the orientation of the coma may be consistent with the orientation of the LASIK flap hinge. Microkeratome-induced errors, represented by Zernike polynomials, may spread to all modes. The general consideration lies on the biomechanical changes of the stroma both long term and short term. The water content redistribution and stress changes in different layers of lamella cause deformation of the cornea.
Biomechanical effects of microkeratome incision were described in more detail by Cynthia Roberts ("The cornea is not a piece of plastic", J. Refract. Surg. 16(4): 407-413, 2000), while aberration effects were studied by Jason Porter et al. ("Separate effects of the microkeratome incision and laser ablation on the eye's wave aberration", Am. J. Ophthalmol. 136(2): 327-337, 2003).
It is possible to take an approach that only considers a population average effect on the induced spherical aberrations as well as coma. It is possible to claim, on average, 0.1 $\mu$m in spherical aberration and a 0.1 $\mu$m in coma (at the same orientation as flap hinge). Therefore, the combined effect, represented as a lasik flap error, can often be at the order of 0.15 $\mu$m. Microkeratome parameters may include spherical aberrations (e.g. central flattening and peripheral thickening effects), hinge effects, and orientation effects. A LASIK flap box may also reflect a biomechanical effect that it based on a laser/tissue interaction.

### 1. Spherical Aberration

After a flap cut, there may be a central flattening and a peripheral thickening of the cornea, hence inducing positive spherical aberrations. In some embodiments, the microkeratome variable can include an induced positive spherical aberration between about 0.1 $\mu$m and about 0.3 $\mu$m. In some embodiments, the microkeratome variable can include a coma in the direction of the microkeratome hinge in an amount between 0.1 $\mu$m and 0.3 $\mu$m.

### 2. Hinge Effect

However, due to the hinge of the flap, the spherical aberration induced might not be circularly symmetric. Therefore, a small amount of coma can also be induced.

### 3. Orientation Effect

It is possible to model the flap effect as a random process to induce positive spherical aberration as well as direction oriented (toward hinge direction) coma as

$$\sigma_m^2 = \sigma_{sph}^2 + \sigma_{coma}^2 , \qquad (10)$$

where $\sigma_m^2$ represents the total error of the flap effect, $\sigma_{sph}^2$ represents the error induced by the positive spherical aberration, and $\sigma_{coma}^2$ represents error induced by the coma.

### F. Healing Effect

Finally, the healing effect is a smoothing process, which can be modeled as a Gaussian kernel (e.g. Gaussian low-pass filter) applied to the ablation target or final wavefront. In general, this is an error reduction process for random noise but an error generation process for uniform error-free shapes. It is possible that the smoothing effect of healing can reduce a local RMS error while not reducing the overall RMS error. The healing effect can be expressed as H(.) The healing effect can be considered as a low pass filter. The healing effect can be thought of as a first order Butterworth low-pass filter. It may be desirable to simplify the model to be a standard Gaussian filter. There are two reasons for doing that. First, the first order Butterworth filter can be approximated with a standard Gaussian filter. Second, the Gaussian filter is often more commonly used, and may be easier to implement than Butterworth. See David Huang et al. ("Mathematical model of corneal surface smoothing after laser refractive surgery", Am. J. Ophthalmol. 135(3): 267-278 (2003).

In some embodiments, the healing effect variable includes a Gaussian kernel with 2 micron in height and 0.5mm in full width at half maximum (FWHM).

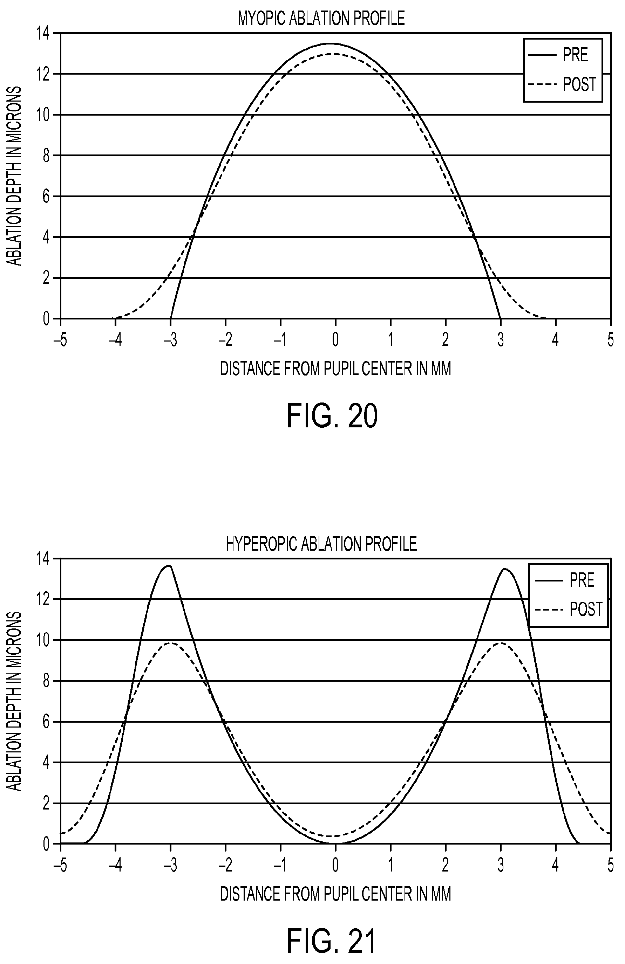

**Fig. 20** shows the healing effect with a Gaussian filter for a -1D myopic treatment profile (Munnerlyn shape) with a 6mm pupil. **Fig. 21** shows the healing effect with a Gaussian filter for a +1D hyperopic treatment profile (Munnerlyn shape) with a 6mm pupil. In both cases the resulting healing effect compared very well with those shown in Huang et al. (see above).

With the application of a Gaussian filter, the end effect is that it may alter the general, or ideal, shape to induce some low order aberrations as well as some lower high order aberrations such as coma and spherical aberrations. However, at the same time, it can also smooth out some higher order aberrations due to its nature of smoothing out rapid fluctuations.

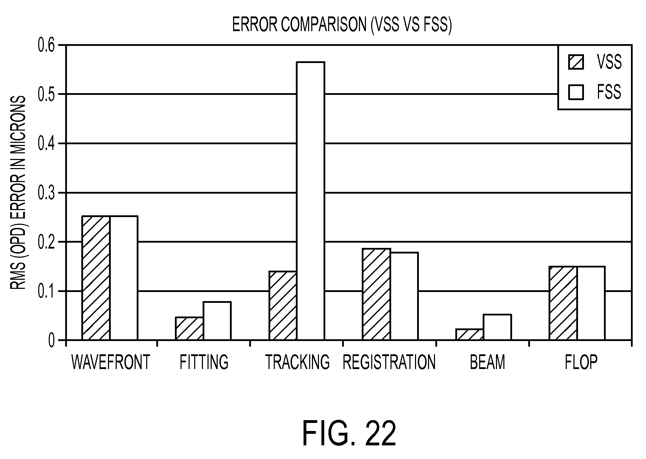

**Fig. 22** illustrates the error from different error sources without consideration of healing effect. Apparently, registration, tracking, wavefront device error and flap effect may be somewhat more important than the laser beam variability as well as fitting error. The error due to healing may reduce the high order RMS errors because it has a "smoothing" effect. It may also induce additional somewhat lower order and some lower high order aberrations such as sphere, cylinder, comas and spherical aberrations. It is possible to consider healing as one of the factors that affects the overall refractive surgery outcome.

## III. DETERMINING A MODEL OPTICAL SURFACE SHAPE BASED ON THE TARGET OPTICAL SURFACE SHAPE AND A SET OF REFRACTIVE SURGERY SYSTEM PARAMETERS

Given a target optical surface shape (e.g. refractive case) and a set of refractive surgery system parameters, it is possible to determine or predict a model optical surface shape. Essentially, this is the target optical surface shape "*as applied*" by the surgery system, and can also represent an optical surface shape after healing. In one embodiment, the present invention provides a system for inhibiting an induced aberration resulting from refractive surgery, where the system includes an input that accepts a target optical surface shape; a module that determines a model optical surface shape based on the target optical surface shape and a set of refractive surgery system parameters; and a module that adjusts the set of refractive surgery system parameters so as to inhibit an aberration in the model optical surface shape.

## IV. COMPARING THE TARGET OPTICAL SURFACE SHAPE AND THE MODEL OPTICAL SURFACE SHAPE TO DETERMINE AN ABERRATION DISTRIBUTION

The comparison of the target optical surface shape and the model optical surface shape can be based on a metric selected from the group consisting of an accuracy variable, a heating variable, and a treatment time variable. The accuracy variable can be based on a root mean squares error factor. The heating variable can be based on a temperature factor. The treatment time variable can be based on an ablation time factor.

## V. ADJUSTING THE SET OF REFRACTIVE SURGERY SYSTEM PARAMETERS SO As TO INHIBIT THE ABERRATION

The adjustment of the set of refractive surgery system parameters can be based on a metric selected from the group consisting of an accuracy variable, a heating variable, and a treatment time variable. The accuracy variable can be based on a root mean squares error factor. The heating variable can be based on a temperature factor. The treatment time variable can be based on an ablation time factor.

## VI. SIMULATION

The present invention provides an approach to modeling components of a refractive correction system. Any of the system parameters discussed above that can introduce errors or contribute to or exacerbate aberrations can also be simulated. Because system parameter error sources can contribute to or amplify aberrations in a model optical surface shape, adjustment of system parameters such as the accuracy of registration, the accuracy of fitting in the ablation algorithm, the tracker speed, the accuracy and system latency time of tracking, the laser beam uniformity and variability, or any other system component, can have effects on those aberrations.

Simulated laser systems can be useful in modeling the effects of various system components, and the present invention provides laser simulators for simulating laser ablation. An exemplary flow diagram of a laser refractive surgery system simulator is shown in Fig. 6. The simulator includes an input refraction module 210, a laser beam profile module 230, a grid geometry module 240, a simulated annealing algorithm module 220, a treatment table module 250, an ideal ablation module 260, a real ablation module 270, a comparison module, 280, and a random sample module 290.

### A. Input Refractions

A simulator may include an input refraction module 210 that can present various target optical surface shapes, or refractive cases, to the simulator. Such target optical surface shapes may include low order refractive cases such as myopic, hyperopic, myopic astigmatism, hyperopic astigmatism, and mixed astigmatism, and may also include high order refractive cases such as a therapeutic case from a real eye that has more than 1.0 microns high order total RMS error. Often refractive cases are determined from a wavefront measurement device.

### B. Laser Beam Profiles

A simulator may include a laser beam profile module 230. In simulating fitting errors for, example, a laser beam profile 230, a 100 micron grid size can be used. A validator, which can simulate the laser ablation, and a pulse instruction, which can simulate the characteristic of each laser pulse, can be used to formulate the basis data for different cases, such as VSS and FSS.

For a 100 $\mu$m sampling resolution in the algorithm fitting of the surface to be solved, flying Gaussian small spot scanning with spot size of or around 1.5mm (FWHM 0.5mm (FSS)) was observed to give a smaller amount of RMS errors compared to other spot sizes.

A laser simulator can be constructed such that given a set of commands (e.g. beam size and location) the accumulated tissue surface during the ablation can be modeled. The basis laser beam shapes, or the craters created by each individual pulse can be, for example, the flat top shapes for the variable spot scanning (VSS) or a Gaussian shape with 0.5mm FWHM for the flying spot scanning (FSS). A laser registration and tracking component, as well as a laser delivery component, can be incorporated into the real ablation module 270.

For the fitting error, the input shape can be based on a wavefront surface. This shape is given to the fitting algorithm for the ideal ablation solution. A calculated list of commands (e.g. spot size and position), when passed through the laser simulator, can form another surface, the real ablation solution. The difference in the two surfaces can be represented as an RMS error. In general, it is possible to obtain a more accurate result if the spot size is smaller. However, due to the limitation in grid size, smaller spot size may not result in smaller fitting error. In addition, if the spot size can be variable, the fitting error can also be smaller.

### C. Grid Geometry

A simulator may include a grid geometry module 240. As discussed above, it is helpful to use at least 1001 X 1001 grid size, that is, 10$\mu$m resolution for the simulation. But the program can be designed with any configuration for grid size or grid geometry.

### D. Simulated Annealing Algorithm

A simulator may include a simulated annealing algorithm module 220. The example wavefronts are then used for the algorithm module 220 to determine an ablation solution. Algorithm module 220 here is analogous to the algorithm box of Fig. 4. Laser beam profiles 230 can provide basis data for constructing laser delivery beam profiles to drive a simulated annealing algorithm, and grid geometry 240 can determine the solution space for the simulated annealing algorithm.

### E. Treatment Tables

A simulator may include a treatment table module 250. It is possible to calculate the treatment table 250 with a simulated annealing algorithm for both VSS and FSS. The same treatment table 250 can be used to adjust variation on the specific basis data for any pulse profile (e.g. VSS or FSS) to determine both the ideal ablation 260 and the real ablation 270. Thus, VSS can be associated with a real ablation and an ideal ablation, and similarly FSS can be associated with a real ablation and an ideal ablation. For each case (e.g. VSS, ideal ablation), there can be 6 input shapes (e.g. myopia, hyperopia, myopic astigmatism, hyperopic astigmatism, mixed astigmatism, and a high-order based therapeutic case). A fitting error can be included prior to table creation. Typically, the treatment table is not considered to contain significant errors. However, a simulated annealing algorithm may introduce certain errors.

In the instance where the treatment table 250 is used to determine the ideal ablation 260, no device errors are assumed, and essentially the target optical surface shape becomes the ideal ablation. In the instance where the treatment table 250 is used to determine the real ablation 270, certain device errors are assumed, and essentially the target optical surface shape is used to determine the model optical surface shape.

For tracking in vertical and horizontal directions, the limitation can be set to 20μm tracking accuracy and system can respond quicker than 10ms, and tracking speed of 60 Hz for VSS. In the case of FSS, the vertical and horizontal tracking accuracy should be less than 5μm and the system latency time shorter than 5ms with a 200 Hz tracking speed.

For cyclo-torsional tracking, the angular accuracy can be within half a degree for VSS and within a quarter degree for FSS. As for laser beam uniformity and variability, VSS benefits from laser energy fluctuation less than 4% and FSS benefits from fluctuation less than 2%.

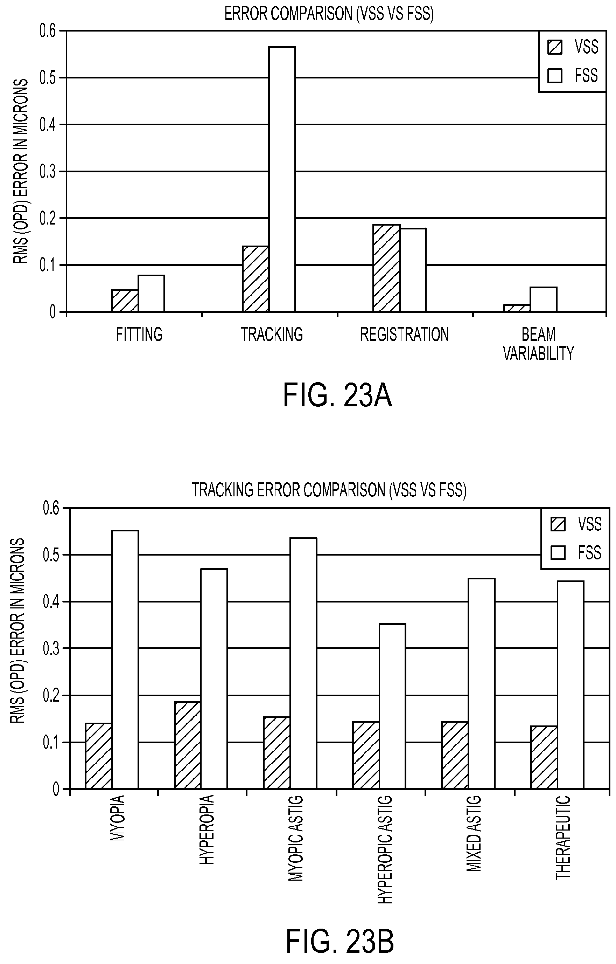

Of the various error sources, tracking error and registration error appear to be relatively large, as can be seen from **Figs. 23A and 23B,** which show a comparison of VSS and FSS for various error sources for a myopia case (**Fig. 23A**) and for tracking error only for all refractive cases (**Fig. 23B**). In general, VSS performs better than FSS in terms of fitting, tracking, and beam variability. In order to correct high order aberrations, limitations on each component in a refractive surgery system can be considered in the system design phase. To achieve same-level error reduction, FSS systems may benefit from tighter restrictions.

For tracking errors, 60Hz tracking speed, 0.05 mm tracking accuracy, and 0.1s system latency time can be assumed. As for registration, 0.025 mm and 2°alignment error can be used. Finally, in beam variability simulation, a 1% fluctuation in delivered laser energy can be assumed. Different parameters with all six refractive cases can be applied to all simulations.

Cyclo-torsional registration was observed to induce only about one tenth RMS error as compared to registration in vertical and horizontal position errors. Cyclo-torsional tracking was observed to induce only about one tenth RMS error as compared to tracking in vertical and horizontal eye movements.

Simulations of tracking can be based on studies of eye motion. Parameters such as eye motion speed, duration of each motion, tracking speed, tracking accuracy, and the system latency time can be used in the simulation.

F. Ideal Ablation

A simulator may include an ideal ablation module 260. Typically, the ideal ablation will contain no errors. When applied with no device errors, the overall induced root-mean-squares (RMS) error should be zero. Ideal shapes can be useful, for example, in evaluating or calculating the healing effect, in which a shape-based propagation may be used. For refractive cases, a Munnerlyn shape or equation can be used to construct an ideal shape. For therapeutic applications, a wavefront shape or Zernike equation can be used.

G. Real Ablation

A simulator may include a real ablation module 270. Due to the imperfection of the components in the refractive surgery system, error can be introduced in the real ablation. Most, if not all, of the components can induce high order aberrations, and some errors appear to be random. As data from the treatment table is processed by the simulator, the source errors are compiled in the real ablation module 270. This may reflect laser tissue interaction, which deals with biomechanics on the cornea. It may also include the healing effect, which can be modeled as a low-pass filter. Relatedly, as a LASIK flap is cut, a microkeratome effect can be included. Registration error as well as tracking error can be considered. The laser delivery system itself can induce further errors. Healing effects can also be considered.

H. Comparison

A simulator may include a comparison module 280. Two surfaces can be compared, on a point by point basis. The results on error can be the overall RMS error. This process can be repeated with random samples 290 to simulate the randomness of different errors.

After healing, it may be desirable to compare the final shape to the original, or ideal, shape, repeatedly several times against each modified real ablation to calculate the statistics. Simulator errors, except for fitting error and wavefront device error, can be represented in real ablation 270.

About 100 to about 1000 cases can be generated, each of which would have random walk based on time, so that the pulses in the treatment table may not exactly land on the expected location. The simulator can add up the pulses and can calculate the difference between the target shape and the model shape. Finally averages for those about 100 to about 1000 wavefronts can be calculated to obtain the RMS error.

I. Random Samples

A simulator may include a random sample module 290. Each of the random samples generated by module 290 can contain different errors. In some embodiments, module 290 can generate 100 random samples, and each is iteratively cycled through the real ablation module 270 for comparison with the ideal ablation module 260.

Modeled ablations of multiple (e.g. 100) surfaces can be used to simulate the random errors introduced by system parameters such as registration, tracking, and laser beam variability. Root-mean-squares (RMS) errors can be used as a performance metric. Multiple simulations can be run to eliminate bias.

To simulate the eye motion, a model of combined smooth pursuit and saccadic motion can be used. The model is a random walk of position (constructed with known distance and random angle) in a certain speed with certain duration. These are the parameters considered for smooth pursuit. The saccadic motion part can be constrained such that when the accumulated eye motion deviates a certain amount from a fixed target, then it quickly drifts back to origin. The model speed can be 2mm/s and the duration can be 22ms. The saccadic limit can be ±0.25mm with a weight of (0.5 + rand()).

The rand() represents a random number generator, and generates a number having a value ranging from 0 to 1. If the eye motion goes beyond the weighted limit, one saccadic motion would move it back to zero.

**Fig. 15A** depicts the simulated X motion, and **Fig. 15B** depicts the simulated Y motion. When comparing **Figs. 14A and 14B to Figs. 15A and 15B,** the simulated motion appears similar to the real eye motion.

Simulation
| | | |
|---|---|---|
| StDev | X = 0.087 mm | Y = 0.099 mm |
| Mean | X = -0.027 mm | Y = -0.029 mm |

Real eye
| | | |
|---|---|---|
| St Dev | X = 0.093 mm | Y = 0.134 mm |

J. Adjustments

The present invention also provides for simulation of system parameter adjustments. It may be desirable to adjust a set of refractive surgery system parameters based on a metric such as an accuracy variable, a heating variable, or a treatment time variable. In some embodiments, the model optical surface shape can correspond to a post-operative optical surface shape such as a healed cornea. By adjusting the set of parameters, it is possible to inhibit aberrations in the post-operative optical surface shape. Simulators can assist in evaluating parameter adjustments to inhibit aberrations.

For example, to inhibit an increase in high order aberrations post-operatively, the wavefront device can have a per-term accuracy of about 0.0183 to about 0.0333 $\mu$m when a set of 6-order Zernike polynomials are used. Relatedly, the target optical surface shape can include a set of 6-order Zernike polynomials, and the set of refractive surgery system parameters can be adjusted such that each component of a post-operative total high order RMS does not exceed about 0.0061 to about 0.0111 $\mu$m. Further, the set of refractive surgery system parameters can be adjusted such that a post-operative total high order RMS is substantially equivalent to a pre-operative total high order RMS. What is more, the set of refractive surgery system parameters can be adjusted such that a post-operative total high order RMS is less than a pre-operative total high order RMS. The set of refractive surgery system parameters can also be adjusted such that a post-operative total high order RMS is about one third the amount of a pre-operative total high order RMS. The procedure includes error evaluation of wavefront device, registration, fitting, tracking, and laser beam uniformity and variability.

Relatedly, to correct or inhibit high order aberrations, the total RMS error can be limited to less than the pre-operative high order RMS. In general, the pre-operative eyes have an average high order RMS on the order of 0.3 $\mu$m. Assuming a 7 component system, each component having an equal limitation, this leads to a maximum limit of 0.113 $\mu$m for each component. This can keep a high order RMS from increasing post-operatively. In other embodiments, where the total RMS error is about 0.1 $\mu$m to about 0.3 $\mu$m and the system includes 3 components, the set of refractive surgery system parameters can be adjusted such that each system component of the total high order RMS does not exceed from about 0.0111 $\mu$m to about 0.0333 $\mu$m. In yet other embodiments, where the total RMS error is about 0.1 $\mu$m to about 0.3 $\mu$m and the system includes 10 components, the set of refractive surgery system parameters can be adjusted such that each system component of the total high order RMS does not exceed from about 0.0061 $\mu$m to about 0.0111 $\mu$m.

To correct high order aberrations, however, it is possible the limitation can be a few times lower as evaluated on terms of total RMS error. For example, assuming a three-fold increase, that is, 0.1$\mu$m total high order RMS, 0.038 $\mu$m is the limitation for each component. This can result in a reduced post-treatment error as compared to the pre-treatment error.

Similarly, to correct high order aberrations, the registration accuracy can be within 10$\mu$m in both vertical and horizontal direction and rotational error within half a degree for VSS. For FSS, the registration accuracy should be less than 10$\mu$m and the rotation accuracy should be less than half a degree. The healing effect can be modeled after each source of error is evaluated. This healing effect, which can be modeled as low pass filter, can effectively decrease the total RMS errors.

The principles of the present invention can be used as guidelines for developing next generation refractive systems, and can also be used as guidelines for individual eye surgeries with existing systems. These principles can also be used to develop strategies for improving parameters of existing systems. For example, by idealizing certain components, i.e. attributing no error to all components but one, it is possible to determine how much error of the total error is associated with the specific component.

In some embodiments, several components of the wavefront/laser system can be controlled to correct high order aberrations in, for example, VISX variable spot scanning (VSS) or flying spot scanning systems (FSS). In a refractive surgery system, discrete pulses can be applied to fit the surface or model optical surface shape. The total error can be associated with model surface shape, or total RMS after surgery.

Once a laser simulator is constructed, and simulation procedures are followed, design guidance can be obtained by plugging in the maximum allowable high order RMS errors for each component. Essentially, design guidance is the design of a laser ablation system involving different components. Relatedly, a simulator can be used to evaluate error source effects.

System performance evaluation for laser vision correction can include all possible error sources, such as errors from wavefront device, fitting, registration, tracking, laser delivery system, LASIK flap effect as well as healing effect. Each component can be considered separately. To evaluate the overall effect, it may be desirable to consider all components at the same time. In some embodiments, tracking, registration, wavefront device as well as LASIK flap effect are the major error sources; whereas fitting and laser beam variability are the minor sources.

**Related Aspects of Error Budgeting Systems and Methods**

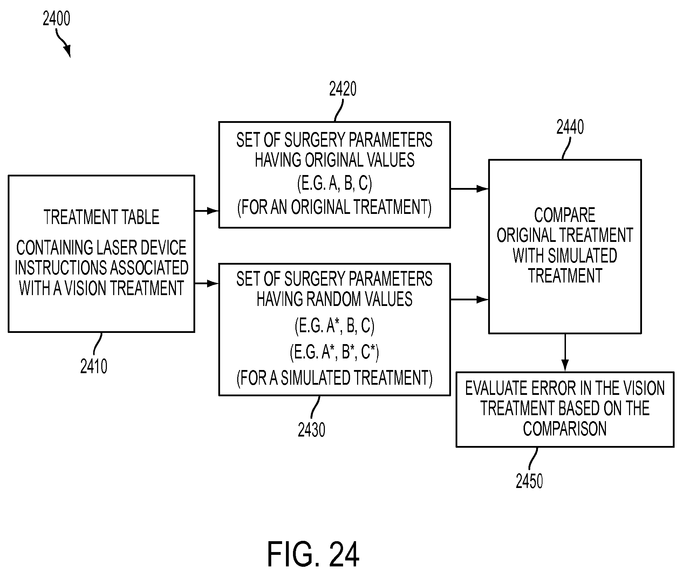

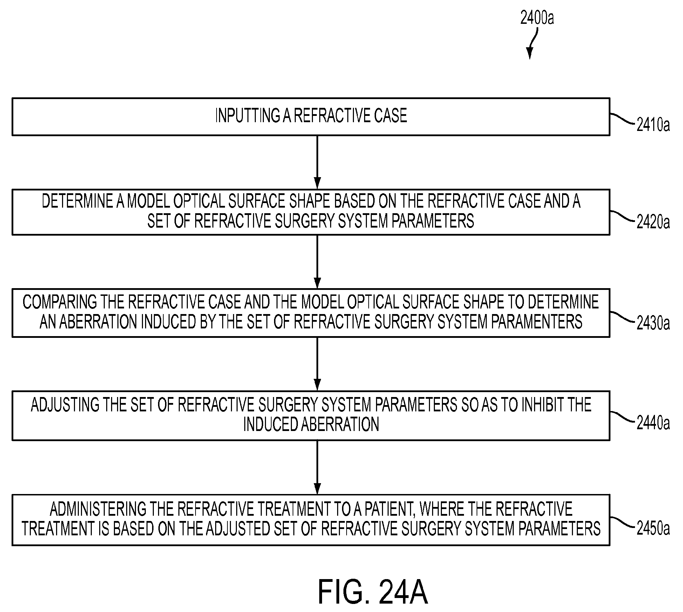

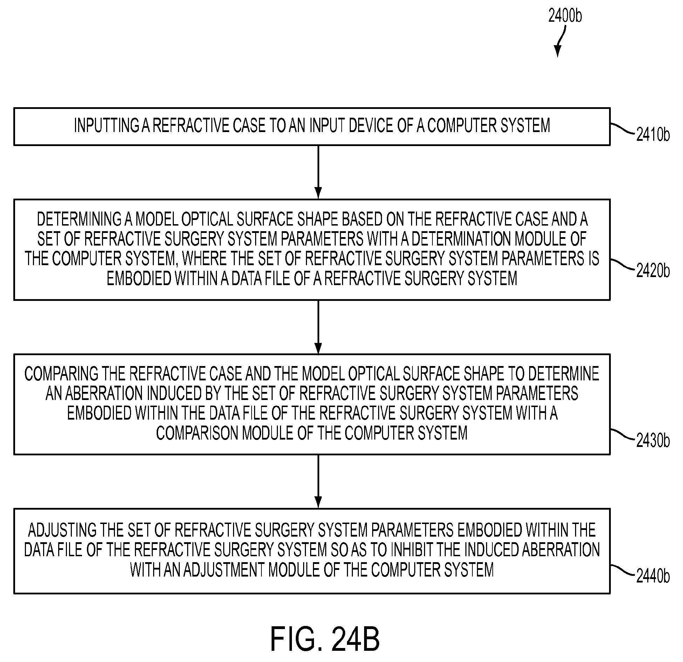

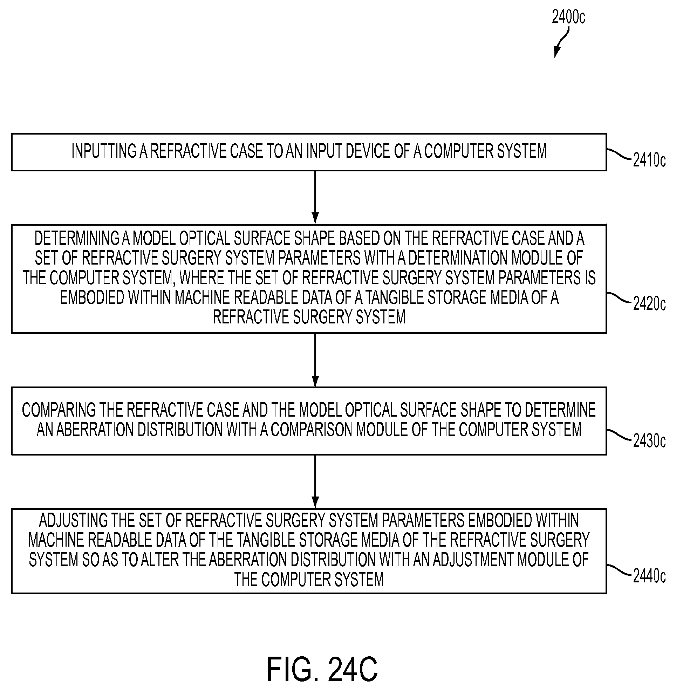

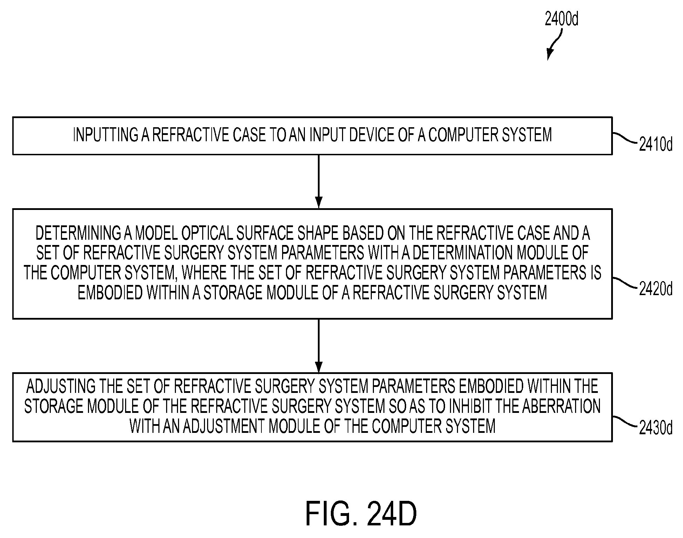

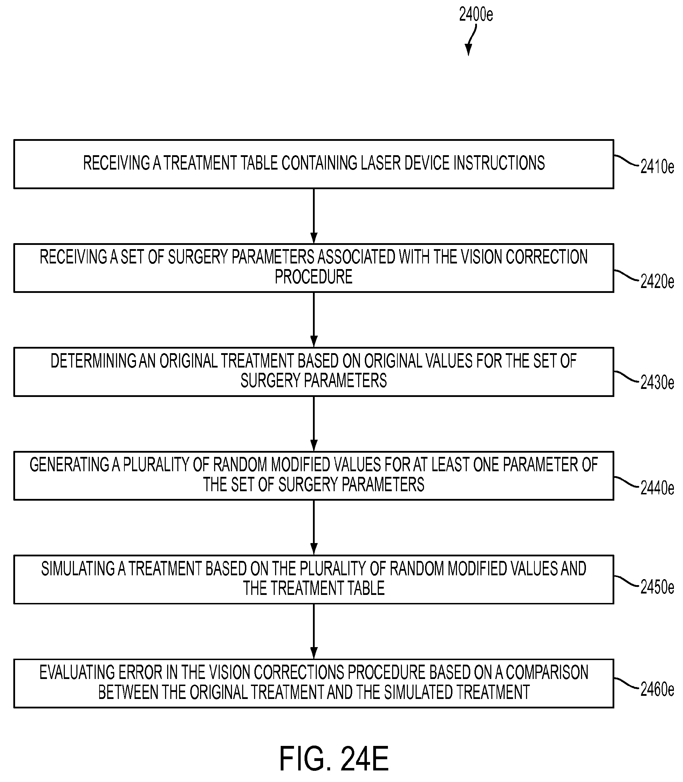

**FIG. 24** depicts various features of exemplary evaluation techniques, according to embodiments of the present invention. As shown here, a method 2400 of evaluating error in a vision correction procedure can utilize a treatment table 2410 containing laser device instructions associated with a vision treatment, for developing a set of surgery parameters having original values (e.g. for an original or desired treatment), as shown in step 2420, and for developing a set of surgery parameters having random values (e.g. for a simulated or randomly deviated treatment), as shown in step 2430. For instance, methods may include receiving a treatment table containing laser device instructions, and receiving a set of surgery parameters associated with the vision correction procedure. According to some embodiments, the original value parameters 2420 may include a set of system parameters having expected or default values. Relatedly, methods may include determining an original treatment based on original values for the set of surgery parameters. According to some embodiments, the random value parameters 2430 may include a set of system parameters having random values. Relatedly, methods may include generating a plurality of random modified values for at least one parameter of the set of surgery parameters, and simulating a treatment based on the plurality of random modified values and the treatment table. As shown by step 2440, the method may include comparing the original or desired treatment (or parameter values associated therewith) with the simulated or randomly deviated treatment (or parameter values associated therewith). Methods may further include evaluating error in the vision treatment based on the comparison, as indicated by step 2550. The techniques disclosed herein may be used for any of a variety of surgery modalities, including excimer laser surgery, femtosecond surgery, and the like.

Embodiments of the present invention are particularly well suited for eliminating or reducing high order aberrations, for example in a wavefront-driven laser refractive system such as a LASIK (laser-assisted *in situ* keratomileusis) apparatus. The quality of the laser refractive surgery, which may involve low order aberrations and high order aberrations, can be evaluated on the basis of the laser performance, as well as on the basis of aspects of the treatment planning, pre-operational diagnostics, and various biological factors. Any of a variety of sources may contribute to the induction of high order aberrations. Embodiments of the present invention encompass techniques for evaluating sources contributing to residual low order aberrations and inducing high order aberrations, which may in some cases operate synergistically so that a final surgical outcome may deviate from an ideal surgical outcome. Embodiments of the present invention encompass comprehensive analysis of an expansive error budget to help set realistic expectations for refractive surgery and improving surgical performance. According to some embodiments, classification and comparative analysis of error sources in laser refractive surgery can reduce potential post-operative high order aberrations as well as residual low order aberrations, thereby improving a surgical outcome. Simulations, based on a system model, allow a comprehensive analysis of the surgical quality issues including over- and under-treatment and induced aberrations.

In some cases, error sources can be classified by their origin, by statistical properties, or by mutual correlation. Relatedly, error sources or surgery parameters can be associated with various functional modules of a diagnostic system (e.g wavefront device), aspects of treatment planning software or modules (e.g. compensating for laser-tissue interactions, reflections from a curved surface, or biomechanical healing changes), as well as functional modules of a laser treatment or delivery system (e.g. delivering the treatment instructions to the eye, centration, energy level control, or positioning). Embodiments encompass error evaluation techniques based on system modeling optionally in combination with experimental data and/or statistical data analysis. In some instances, error sources can be parameterized, quantified, and prioritized, for example in terms of their effect on residual refractive error and high order aberrations. Relatedly, embodiments of the present invention further encompass the classification and comparative analysis of error sources in surgical procedures such as laser refractive surgery.

Embodiments of the present invention encompass software products (e.g. GUI-based packages) for error budget analysis. In operation, a user can upload, or otherwise transmit, a pre-defined list of system or surgery parameters, and optionally, assign or edit their tolerances. Similarly, one or more treatment tables can be uploaded, or otherwise transmitted, and analyzed, for example simultaneously. In some cases, analysis of various error sources of arbitrary magnitude can be carried out in terms of their effect on an arbitrary treatment. In some instances, both systematic and random system errors can be simulated in order to evaluate their effect on a refractive surgery outcome, including, for example, spherical and cylindrical refraction, spherical aberration, and total RMS error. Error sources may be compared and prioritized based on a factor analysis, which may consider each source individually with other sources absent. According to some embodiments, synergistic effects associated with correlated error sources can be analyzed with Monte Carlo simulation, which can yield a total system error distribution.

Systems and methods disclosed herein encompass techniques, such as Monte Carlo simulations, for modeling a virtual laser system. In some cases, models may account for the effects of imperfection in components such as surface fitting, tracking, registration, and laser energy fluctuation. Error as a function of system or surgery parameters can be evaluated for various refractive cases. Embodiments of the present invention provide a framework that allows for facile inclusion of various error sources into the evaluation, such that any of a variety of error sources can be identified and modeled. True Monte Carlo simulations can be performed to achieve desired error budgeting performance.

Embodiments of the present invention also encompass techniques for adjusting operational or system parameters based on aberration or error analysis results. For example, as depicted in **FIG. 24A,** a method 2400a of adjusting a set of refractive surgery system parameters for use in a refractive treatment may include inputting a refractive case as indicated by step 2410a, determining a model optical surface shape based on the refractive case and a set of refractive surgery system parameters as indicated by step 2420a, comparing the refractive case and the model optical surface shape to determine an aberration induced by the set of refractive surgery system parameters as indicated by step 2430a, and adjusting the set of refractive surgery system parameters so as to inhibit the induced aberration as indicated by step 2440a. Optionally, the method may further include administering the refractive treatment to a patient as indicate by step 2450a, where the refractive treatment is based on the adjusted set of refractive surgery system parameters.

Embodiments of the present invention further encompass techniques for inhibiting induced aberration based on surgery parameter or error analysis results. For example, as depicted in **FIG. 24B,** a method 2400b of inhibiting an induced aberration resulting from refractive surgery may include inputting a refractive case to an input device of a computer system as indicated by step 2410b, and determining a model optical surface shape based on the refractive case and a set of refractive surgery system parameters with a determination module of the computer system as indicated by step 2420b. The set of refractive surgery system parameters may be embodied within a data file, which may be configured for use with a refractive surgery system. Methods may also include comparing the refractive case and the model optical surface shape to determine an aberration induced by the set of refractive surgery system parameters embodied within the data file with a comparison module of the computer system as indicated by step 2430b, and adjusting the set of refractive surgery system parameters embodied within the data file so as to inhibit the induced aberration with an adjustment module of the computer system as indicated by step 2440b.

What is more, embodiments of the present invention encompass methods of altering distribution resulting from surgery based on surgery parameter or error analysis results. For example, as depicted in **FIG. 24C**, a method 2400c of altering aberration distribution resulting from optical surface refractive surgery may include inputting a refractive case to an input device of a computer system as indicated by step 2410c, and determining a model optical surface shape based on the refractive case and a set of refractive surgery system parameters with a determination module of the computer system as indicated by step 2420c. The set of refractive surgery system parameters may be embodied within machine readable data of a tangible storage media. Methods may also include comparing the refractive case and the model optical surface shape to determine an aberration distribution with a comparison module of the computer system as indicated by step 2430c and adjusting the set of refractive surgery system parameters embodied within machine readable data of the tangible storage media so as to alter the aberration distribution with an adjustment module of the computer system as indicated by step 2440c.

Embodiments of the present invention also encompass methods of inhibiting a surgery induced aberration based on surgery parameter or error analysis results. For example, as depicted in **FIG. 24D**, a method 2400d of inhibiting a refractive surgery induced aberration may include inputting a refractive case to an input device of a computer system as indicated by step 2410d, and determining a model optical surface shape based on the refractive case and a set of refractive surgery system parameters with a determination module of the computer system as indicated by step 2420d. The model optical surface shape typically has an aberration. The set of refractive surgery system parameters may be embodied within a storage module of a refractive surgery system. Methods may also include adjusting the set of refractive surgery system parameters embodied within the storage module of the refractive surgery system so as to inhibit the aberration with an adjustment module of the computer system as indicated by step 2430d.

**FIG. 24E** depicts aspects of a method 2400e of evaluating error in a vision correction procedure, according to embodiments of the present invention. As shown here, the method may include receiving a treatment table containing laser device instructions as indicated by step 2410e, receiving a set of surgery parameters associated with the vision correction procedure as indicated by step 2420e, determining an original treatment based on original values for the set of surgery parameters as indicated by step 2430e, generating a plurality of random modified values for at least one parameter of the set of surgery parameters as indicated by step 2440e, simulating a treatment based on the plurality of random modified values and the treatment table as indicated by step 2450e, and evaluating error in the vision correction procedure based on a comparison between the original treatment and the simulated treatment as indicated by step 2460e. Optionally, methods may include inhibiting induced aberration based on the evaluated error, altering distribution resulting from surgery based on the evaluated error, inhibiting surgery induced aberration based on the evaluated error, and the like.

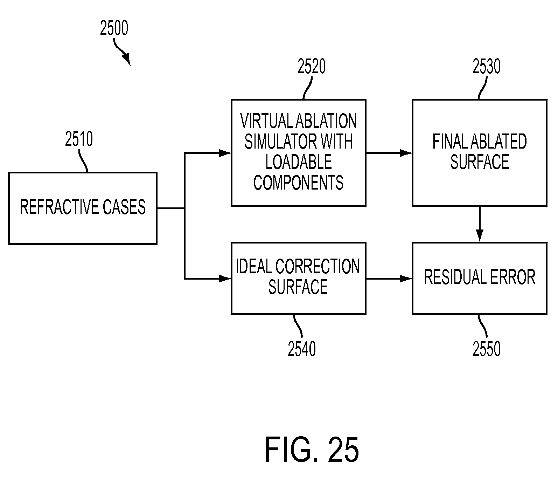

**FIG. 25** depicts an exemplary evaluation framework 2500 that can be used to develop systems, methods, code and/or computer program products for evaluating error sources. As shown here, refractive cases 2510 can be myopia, myopic astigmatism, hyperopia, hyperopic astigmatism, mixed astigmatism, or ocular wavefront based. In some cases, the framework 2500 can be used to construct a MATLAB code that is able to conduct Monte Carlo simulations for various possible errors happening in a refractive laser system for the purpose of evaluating the error sources and their relative contributions to the overall residual refractive error and high order aberrations. In some cases, the code can be used to evaluate various components of a laser system, which can be loaded into the system to perform simulated ablations, based on the refractive case, as indicated by step 2520. Final ablated surfaces can be determined based on the simulated ablation, as indicated by step 2530. Similarly, as shown here, an ideal correction surface can be determined based on the refractive case, as indicated by step 2540.

Further, it is possible to evaluate residual error (e.g. similar to error in FIG. 24) in a vision treatment, based on a comparison between the final ablated surface and the ideal correction surface, as indicated by step 2550.

According to some embodiments, the residual error determination illustrated by step 2550 can be calculated to present low order and high order components, where the low order components relate to the residual refractive error, and the high order components relate to high order aberrations.

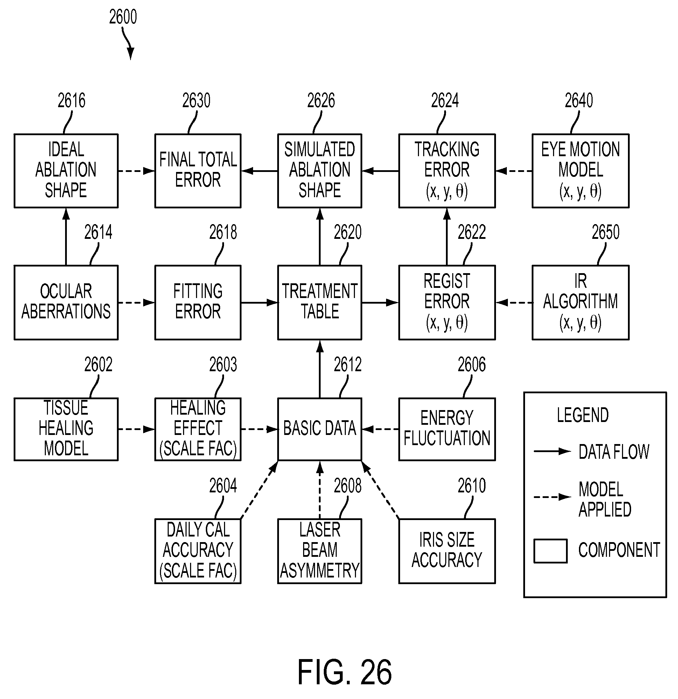

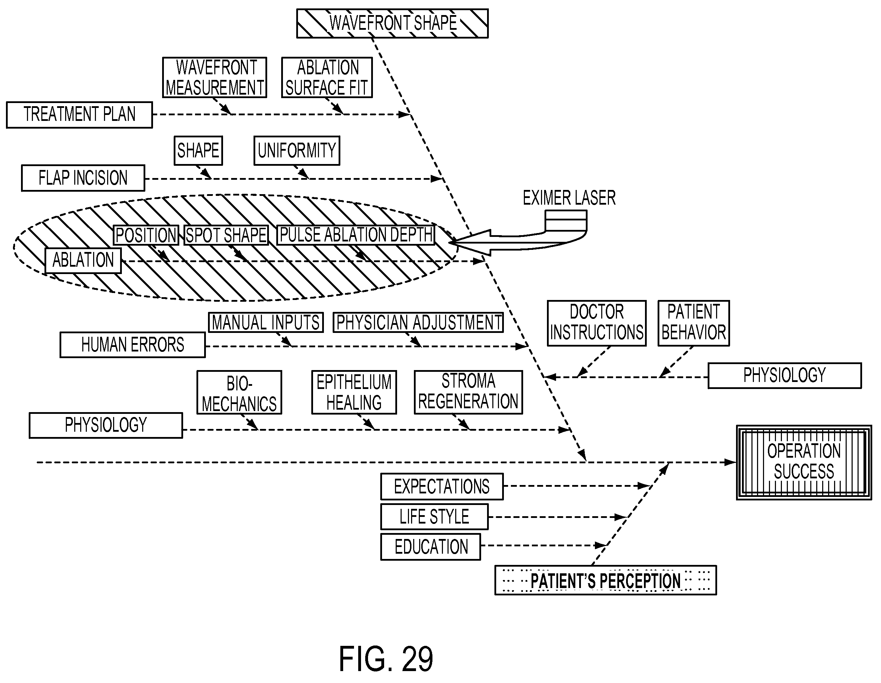

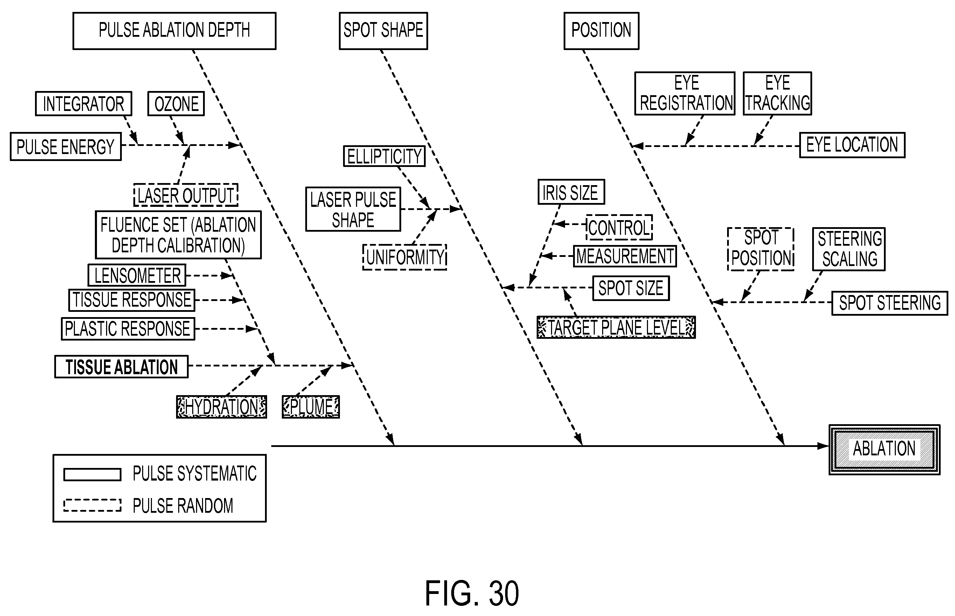

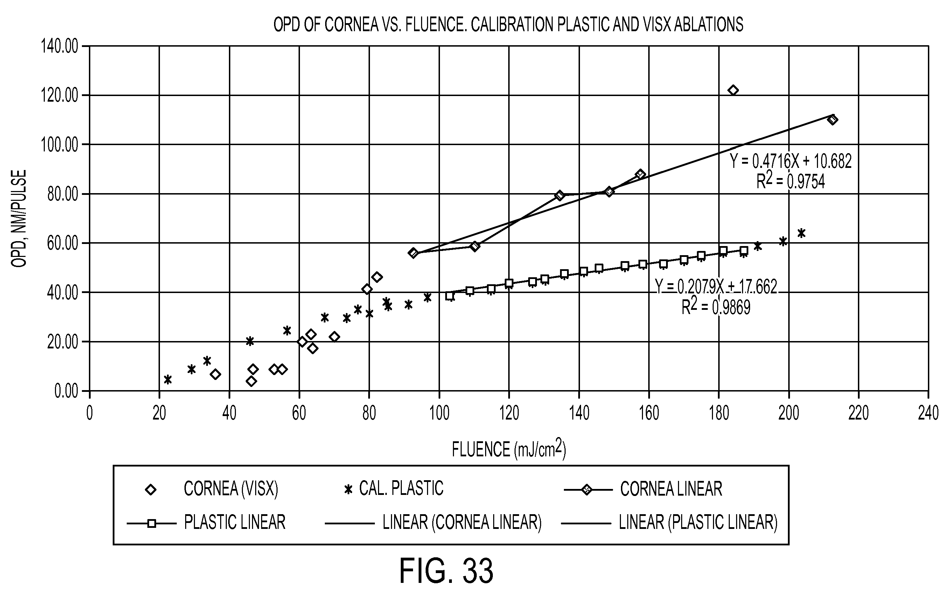

**FIG. 26** depicts aspects of an error analysis process 2600 according to embodiments of the present invention. As shown here, several factors are identified which can affect the basis data accuracy. In some cases, a biomechanical and healing effect can potentially affect the true shape of each laser pulse that is ablated on human tissue, after the eye heals, and hence a tissue healing model 2602, optionally in combination with a healing effect scale factor model 2603, can be considered to drive or account for such an effect. A daily calibration accuracy model 2604, in some cases combined with a fluence set, can be used to reflect or determine the accuracy of the basis data of laser pulses actually delivered. According to some embodiments, daily calibration can be a significant error source, particularly for a 20 Hz machine. Aspects of calibration are further discussed herein in connection with **FIG. 33**. Relatedly, **FIGS. 29 and 30** depict aspects pulse ablation depth and fluence set, respectively, which can also relate to calibration error or sources thereof.

In some cases, laser energy can exhibit a Gaussian distributed random fluctuation which may cause uncertainties on the basis data, and hence an energy fluctuation model 2606 can be considered to drive or account for such an effect. In some cases, when a laser beam is delivered off-axis, the resulting laser beam spot imposed is not radially symmetric, but rather asymmetric, and thus, a laser beam asymmetry model 2608 can be considered to drive or account for this effect, so as to provide an adjustment for the basis data. In some cases, the aperture size defining a laser beam may not be the exact size it is expected to be, and hence an iris size accuracy model 2610 can be considered to drive or account for such an effect.

As depicted by the dashed arrows leading to the basis data module 2612, various component models (e.g. 2602, 2603, 2604, 2606, 2608, 2610) or combinations thereof can be used for a single simulation, or for multiple simulations, as desired, for example as part of a Monte Carlo simulation. An initial ablation shape may correspond to a myopia, myopic astigmatism, hyperopia, hyperopic astigmatism, mixed astigmatism, or ocular wavefront based refractive case. As shown here, any of a variety of influences can be captured in the basis data, and a realistic set of basis data can be used for simulating a virtual laser ablation. Often, treatment table information, which may be determined based at least in part on basis data information, can be used to drive operation of the laser.

As depicted by the solid arrows ultimately culminating with the Final Total Error module 2630, data flow may be initiated with ocular aberrations as indicated by ocular aberration module 2614 to obtain an ideal ablation shape as indicated by ideal aberration shape module 2616. Similarly, data flow initiated with ocular aberration module 2614 may go through fitting error module 2618 to treatment table module 2620. A simulated ablation shape may be determined by the simulated ablation shape module 2626, based on the treatment table data or information received from the treatment table module 2620.

In some instances, the simulated ablation shape module 2626 may also utilize tracking and/or registration error data when determining the simulated ablation shape. For example, the x- and y-positions in the treatment table may be affected because the eye moves. A validated eye motion model 2640 can be used to simulate eye motion to include error produced in registration (torsional and x and y), for example as modeled by IR Algorithm model 2650 and calculated by registration module 2622, as well as tracking as calculated by tracking error module 2624. Additionally, for each treatment case, the simulated ablation shape and the corresponding ideal shape can be compared and the residual error determined.

Optionally, an iris registration (IR) Algorithm model 2650 can operate to determine a registration error, and an Eye Motion model 2640 can operate to determine a registration error. According to some embodiments, the tracking error can be realistically determined by comparing the ablation outcome when the tracking is turned off, is turned on but running realistically, and when it is running ideally. When it is running ideally, it means there is no latency time, no inherent tracking error, and the like. In this way, operation of the Eye Motion model 2640 can be related to operation of the tracking module 2624. With regard to the IR, according to some embodiments the IR Algorithm 2650 can be related to the iris registration error 2622 by means of an IR process. By registering the coordinates of the wavefront with respect to the iris features during the pre-operative exam, and by re-registering the coordinates of the wavefront under the laser after the LASIK flap is cut, each laser pulse can be offset by a certain distance in x and y to account for the relative movement between the two sittings. So with and without IR can cause a large error. Similarly, ideal IR and realistic IR also can present some difference, as in the tracking situation.

The data flow also goes through a simulated annealing process, for treatment table creation. According to some embodiments, the fitting error module 2618 can implement a simulated annealing process. Fitting error can be included as a component of the error sources.

In some instances, to derive an error budget, variation of one or more parameters can be performed. For example, it is possible to change the tracking speed, registration accuracy, daily calibration accuracy, and the like, to calculate the final error. With a vast majority of simulation cases, a relationship between each of the components can be determined and the priority set. For example, it is possible to determine which components most significantly affect final error, and prioritize the components accordingly based on their relative contributions to the total error.

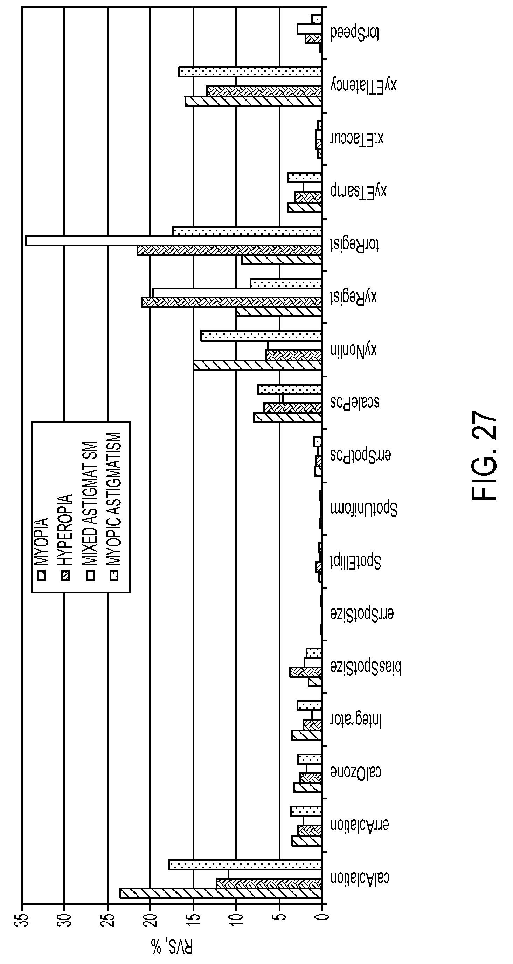

**FIG. 27** depicts a graph for various parameters, in terms of the error for selected components in a laser system. **Table 1** provides an explanation for the parameter values.

| Error type | Error source name | Max value | Pulse-to-pulse variations | Description |
|---|---|---|---|---|
| **Pulse ablation depth** | calAblation | 5% | Systematic | Calibration accuracy of ablation depth (lens cal) |
| | errAblation | 9% | Random | Precision of a single pulse ablation depth |
| | Integrator | 6% | Dynamic | Amplitude of integrator transmission oscillations |
| | calOzone | 1.8% | Dynamic | Max ozone calibration error |
| **Laser spot** | calSpotSize | 25 um | Systematic | Accuracy of spot size calibration |
| | errSpotSize | 10 um | Random | Precision of spot size calibration |
| | SpotEllipt | 50 um | Systematic | Spot ellipticity factor (max deviation from circle) |
| | SpotUnif | 2% | Random | Spot uniformity: max fluence deviation from |
| **Spot steering** | errSpotPos | 20 um | Random | Precision of spot positioning |
| | scalePos | 1.0% | Systematic | Accuracy of spot positioning scaling factor |
| | xyNonlin | 50 um | Dynamic | Amplitude of periodic positioning errors |
| | xyRegist | 50 um | Systematic | Accuracy of XY iris registration |
| | torRegist | 1 deg | Systematic | Accuracy of torsional iris registration |

(continued)

| Error type | Error source name | Max value | Pulse-to-pulse variations | Description |
|---|---|---|---|---|
| Eye location | xyETsamp | 17ms | Random | XY tracker sampling period |
| | xyETaccur | 20 um | Random | Positioning precision of XY tracker |
| | xyETlatenc | 0.05 sec | Random | Latency of XY tracker |
| | torETsamp | N/A | Random | Torsional tracker sampling period |
| | torETaccur | N/A | Random | Positioning precision of torsional tracker |
| | torLatencv | N/A | Random | Latency of torsional tracker |
| | torSpeed | 0.1 deg/s | Random | Eye torsional random-walk speed |

As shown in **FIG. 27**, or **Table 1**, or a combination thereof, in some cases ablation depth calibration, iris registration, and eye tracking errors can significantly contribute to the overall error, for example relative to beam shape random errors. Similarly, in some instances, random errors, which fluctuate from pulse-to-pulse, can be mostly averaged out and, therefore, their overall contribution can be be relatively small.

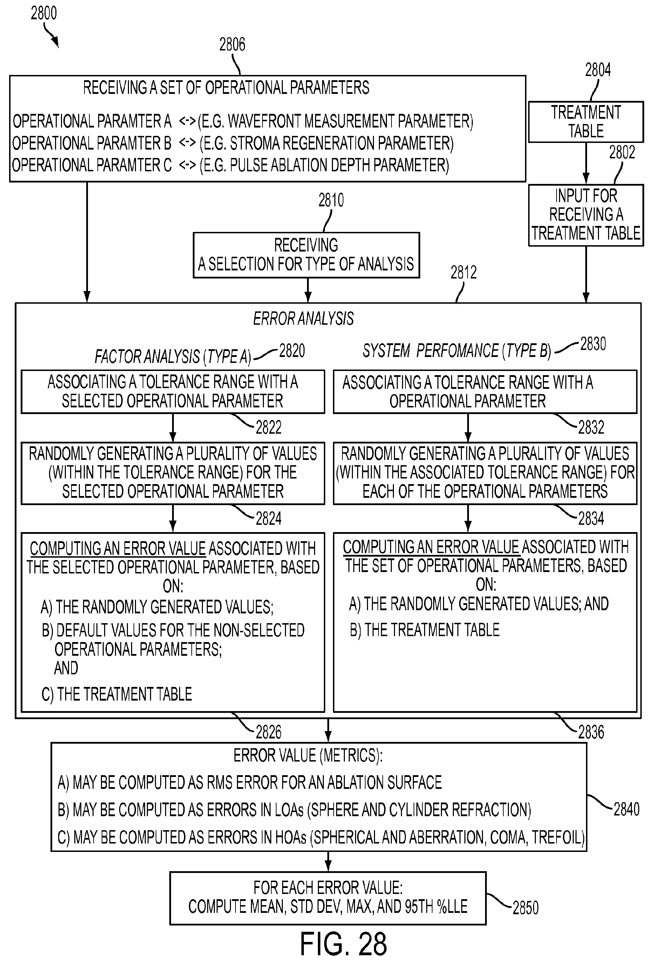

FIG. 28 depicts aspects of error analysis techniques according to embodiments of the present invention. As shown here, a method 2800 of evaluating error in a vision correction procedure may include receiving, at an input 2802, a treatment table 2804 containing laser device instructions. A treatment table may include laser device instructions for any of a variety of refractive cases. Exemplary refractive cases include myopia cases, hyperopia cases, myopic astigmatism cases, hyperopic astigmatism cases, mixed astigmatism cases, and high-order (ocular wavefront) based therapeutic cases. According to some embodiments, a processor or software program can be used to generate such instructions for a treatment laser, for example based on data obtained from an aberrometer. In some instances, a treatment table may include laser ablation instructions for treating a patient's eye. Optionally, treatment table instructions may account for low order and high order aberrations of an eye. Treatment tables may in some cases be based on wavefront data obtained from an eye. A laser treatment table can include a listing of coordinate references for delivery of a laser beam during an ablation of the cornea. In some cases, a treatment table can include the value of the discrete radial and angular positions of the optomechanical elements used to scan an image over a portion of the anterior corneal surface. In some cases, a treatment table may include laser pulse instructions such as size, location, sequence, and the number of laser pulses per position.

The method may also include receiving a set of surgery or operational parameters associated with the vision correction procedure as indicated by step 2806. A set of operational or surgery parameters can include any of a variety of parameters. Exemplary parameters include treatment plan parameters, flap incision parameters, ablation parameters, human factor or error parameters, psychology parameters, physiology parameters, patient perception or expectation parameters, patient life style parameters, patient education parameters, surgical condition parameters, and surgical environment parameters. In some cases, a treatment plan parameter may be an aberration measurement parameter such as a wavefront measurement parameter, or an ablation surface fit parameter. In some cases, a flap incision parameter may be a shape parameter or a uniformity parameter. In some cases, an ablation parameter may be a position parameter, a spot shape parameter, or a pulse ablation depth parameter. In some cases, a human error or factor parameter may be a manual input parameter, or a physician adjustment parameter. In some cases, a psychology parameter may be a physician instruction parameter or a patient behavior parameter. In some cases, a physiology parameter may be a bio-mechanics parameter, an epithelial healing parameter, or a stroma regeneration parameter. In some cases, a surgery parameter may be a surface fitting parameter, a tracking parameter, a registration parameter, or a laser energy fluctuation parameter. In some cases, a surgical condition parameter may include a keratometry parameter, a pachymetry parameter, or an intraocular eye pressure (IOP) parameter. In some cases, a surgical environment parameter may include a temperature parameter, a humidity parameter, or a latitude parameter. In some cases, a default value may be associated with an operational or surgery parameter. In some cases, such default values may be specific to a particular treatment and/or diagnostic system. Optionally, methods may include editing default values or tolerance ranges associated therewith.

Methods may also include determining an original treatment based on original values for the set of surgery parameters, and generating a plurality of randomly modified values for at least one parameter of the set of surgery parameters, for example as shown by step 2824 or step 2834. In some cases, the plurality of randomly modified values may be generated for a parameter associated with a permanent error. In some cases, the plurality of randomly modified values may be generated for a parameter associated with an alignment error. In some cases, the plurality of randomly modified values may be generated for a parameter associated with a calibration error. In some cases, the plurality of randomly modified values may be generated for a parameter associated with a treatment error. In some cases, the plurality of randomly modified values may be generated for a parameter associated with noise fluctuations during laser treatment.

According to some embodiments, methods may include simulating a treatment based on the plurality of randomly modified values and the treatment table, and evaluating error in the vision correction procedure based on a comparison between the original treatment and the simulated treatment. As shown in **FIG. 28**, methods may also include receiving a selection for a type of analysis as indicated by step 2810. For example, a user or operator may wish to select a factor analysis 2820, a system performance analysis 2830, or another type of analysis. A factor analysis type may include associating a tolerance range with a selected surgical or operational parameter as indicated by step 2822, randomly generating a plurality of values (within the tolerance range) for the selected operational or surgical parameter as indicated by step 2824, and computing an error value associated with the selected operational or surgery parameter as indicated by step 2826. The error value can be computed based on the randomly generated values, default values for the non-selected operational or surgery parameters, and the treatment table. A system performance analysis type may include associating a tolerance range with each operational or surgery parameter as indicated by step 2832, randomly generating a plurality of values (within the associated tolerance range) for each of the operational parameters as indicated by step 2834, and computing an error value associated with the set of operational parameters as indicated by step 2836. The error value can be computed based on the randomly generated values and the treatment table. In some instances, the magnitude of errors associated with each operational parameter can be evaluated. In some cases, the magnitude of errors associated with a set of selected operational parameters can be selected. Optionally, errors can be calculated via Monte Carlo simulation for each of the selected parameters separately in a factor analysis approach. In some cases, errors (or errors distribution functions) can be calculated via probability distribution for every selected operational parameter.

As shown in method step 2840, error in a vision correction procedure can be evaluated according to a root-mean-square analysis, a low order aberration analysis, or a high order aberration analysis. For example, an error value or metric may be computed as a root-mean-square (RMS) error for an ablation surface. In some cases, an error value or metric may be computed as an error in a low order aberration (LOA) such as sphere or cylinder refraction. In some cases, an error value or metric may be computed as an error in a high order aberration (HOA) such as spherical aberration, coma, or trefoil. Method step 2850 involves computing a mean, a standard deviation, a maximum, a 95th percentile, or the like, for one or more of the error values.

Although laser refractive correction can allow more than 90% of patients to achieve 20/20 vision without glasses, complicated cases, for example those which require deep spherical and cylindrical ablation and/or treatment for strong high-order aberrations, in particular will benefit from further improvements. It is possible to achieve such improvements by analyzing which factors, deviations from selected models, or inaccuracies in system parameters may contribute to variations from perfect outcome of laser vision correction procedures.

At one level, errors can be considered as factors which define operation success. These factors may be defined differently depending on the level of detail of the system description. In general, the success of a refractive surgery operation may depend on both objective (e.g. wavefront shape) and subjective (e.g. patient perception) factors, as indicated by **FIG. 29**, which provides an error sources diagram for different levels of system detail.

Factors such as treatment plan parameters, flap incision parameters, and ablation parameters may be relatively technical in nature. A fair amount of knowledge may be available regarding these parameters, and it may be possible to control such parameters to a significant degree. In contrast, factors such as human error parameters, psychology parameters and physiology parameters may be less technical in nature. Less knowledge may be available regarding these parameters, and it may be more difficult to control such parameters to a significant degree.

A laser refractive correction procedure has several stages, which include manual operations as well as patient's physiological and psychological responses. Therefore, the operation success may be affected by various distinctive types of errors, including technological errors (e.g. errors in machine performance, calibrations, and/or numerical algorithms), physiological errors (e.g. individual differences in tissue response and healing process), human errors (e.g. deviations from a standard procedure, including such possibilities as doctor's mishandling of the equipment or patient's violations of a prescribed behavior), an psychological errors (e.g. patient perception differences, which may affect visual acuity measurements). Any of these errors may be within the scope of error analysis according to embodiments of the present invention. Individual error sources can be addressed by various means. For example, error sources can be addressed by further improvement in machine performance. Error sources can also be addressed by higher levels of procedure automation to eliminate or reduce human errors, by algorithm corrections for physiological errors, and by educating doctors and patients to alleviate human errors and psychological errors.

Objective errors can be classified into the following types, which may have different statistical behavior: permanent, alignment, calibration, treatment, and fluctuations. According to some embodiments, permanent errors can be considered to be those which are always the same for all machines, all patients and treatments, and all ablation steps (pulses). Such errors may stem from lack of information of some physical effect, systematic errors in our models for machine calibration, ablation effects, and the like. According to some embodiments, alignment errors can be considered to be those which always stay the same on a given machine, but may vary from machine to machine. These errors may be caused by optical or mechanical misalignments which are not compensated by calibrations. According to some embodiments, calibration errors can be considered to stay the same for all treatments on a given machine, but may change after recalibration. According to some embodiments, treatment errors can be considered to be those which stay constant during the ablation procedure, but may vary from treatment to treatment even on the same machine. These may be inaccuracies in the treatment plan, uncompensated machine drift between treatments, and the like. According to some embodiments, fluctuation errors may be considered to include random noises of all kinds, which may affect each laser pulse ablation individually.

When evaluating the average rate of success for a laser treatment technology, it may be desirable to consider all possible variations of alignment and calibration error types and average over the entire ensemble of all possible machines and patients as well as variations within each treatment procedure. Relatedly, it may be desirable to count the permanent error type as systematic errors and the others error types as random. When evaluating how large treatment deviations can be for a given machine, it may be desirable to count the permanent and alignment error types as systematic, because they stay the same for a given tool. Relatedly, calibration, treatment, and fluctuation type errors can be considered as random.

FIG. 30 depicts an error sources diagram for a laser ablation system according to embodiments of the present invention. As shown here, error source parameters may include a pulse ablation depth parameter, a spot shape parameter, and a position parameter. With regard to a pulse ablation depth parameter, the depth and profile of each laser pulse ablation may depend on two factors: the laser energy delivered to the patient's eye, and the effect of the delivered pulse on corneal tissue. Both factors may depend on technical parameters of the system, system calibration quality, and physiological effects. With regard to a pulse energy parameter, various factors may affect the energy of each laser pulse, delivered to the patient plane. Such factors may include a laser output fluctuation parameter, an integrator parameter, and an ozone parameter. As illustrated here, exemplary error analysis techniques can involve assessment of surgery parameters on the basis of a hierarchical or fishbone structure.

With regard to fluctuations of laser output, it is noted that laser output may randomly change from pulse to pulse. This variation may translate into a pulse-to-pulse random error in the ablation depth. According to some embodiments, a measured $3\sigma$ level of these fluctuations is 9%. It is possible to use this value as an empirical estimate of pulse-to-pulse fluctuations in the tissue ablation depth and simulate it with Gaussian distribution. Additional factors may contribute to random pulse-to-pulse ablation variations, such as lens transmittance changes and mirror reflectance changes for different rays, plumes of evaporated tissue, corneal tissue non-uniformity, and transient laser energy bursts and transient optical absorptions.

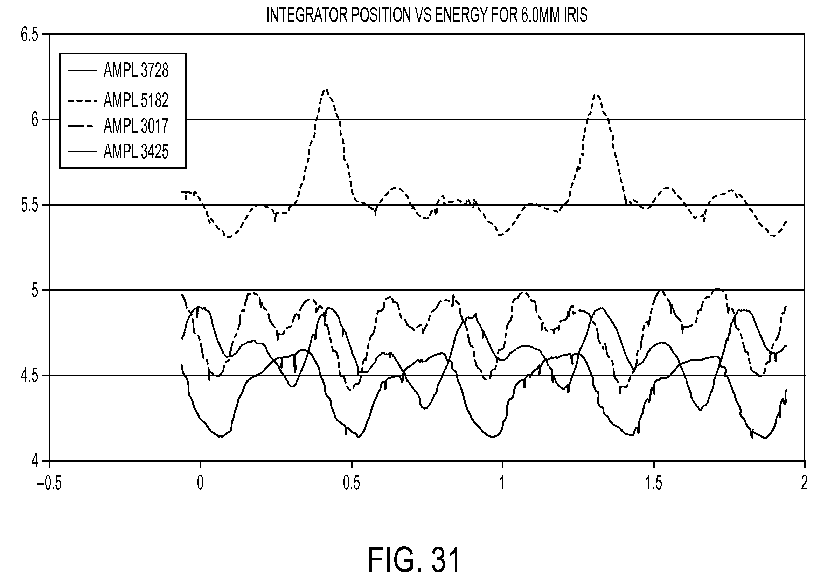

With regard to an integrator parameter, it is noted that a misalignment of rotation assembly / mounting may cause a periodic change of the laser energy at the treatment plane. The amplitude of these variations may be about 6%, for example as depicted in FIG. 31, which shows fluence or integrator transmission fluctuations at the treatment plane caused by integrator rotation as measured for various baseline system embodiments (plotted vs. time, sec). In some instances, a baseline system may encompass a STAR S4 IR™ Excimer Laser System. The frequency of the variations can be defined by an integrator rotation frequency, which may be about 2 Hz for a baseline system and 2.5 Hz for a modified system (e.g. a baseline system which has been modified). These variations can be simulated with a sinusoidal addition to the pulse ablation depth with 6% amplitude and a phase randomly changing from treatment to treatment. According to some embodiments, a quasi-periodic change of the laser energy at the treatment plane may be caused by a misalignment of a rotation assembly/mounting.

With regard to an ozone parameter, it is noted that ozone accumulated in a UV laser optical path can strongly affect the light absorption. This effect can be addressed or alleviated by an ozone compensation function with parameters, calibrated after the system is assembled and then occasionally during field service maintenance. An ozone compensation model can be based on exponential approximation for ozone accumulation. It is possible to simulate the ozone calibration error with the following function:

$$\delta I = \varepsilon \cdot \left(1 - \exp(-n/N_0)\right)$$

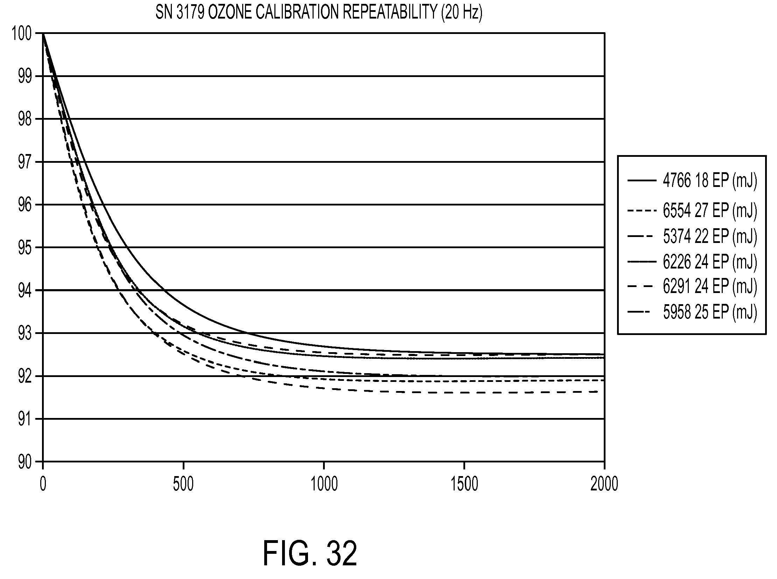

where $\varepsilon$ is the maximum ozone calibration error, $n$ is the number of pulses, and $N_0$ is the time constant, which depends on the system design with respect to ozone handling. Accuracy of the ozone compensation can be defined by the calibration accuracy as well as the system drift between calibrations. The calibration repeatability can be estimated for a baseline system, for example as depicted in **FIG. 32**, which shows ozone calibration repeatability curves. The time constant in the above function can be estimated from the data in **FIG. 32** as $N_0$=400. Maximum ozone calibration deviations in these measurements may be about 1%, according to embodiments of the present invention. In some cases, this may be considered as $3\sigma$ level for random variations. Another possible error source for ozone calibration is the system drift between calibrations, which may lead to a bias in the ozone compensation function up to 2% level. For a given treatment this error may be considered as a random value with $3\sigma$ equal to half of the maximum drift value. Assuming that calibration error and the drift bias are statistically independent, it is possible to estimate the total maximum error in ozone compensation function as $3\sigma = \sqrt{1^2 + (0.5 \cdot 2)^2} = 1.8\%$. This error may be considered as a randomly chosen constant for any given treatment in a baseline system. A modified system may have very different handling of ozone accumulation and parameters of the ozone compensation model may become very different. With higher repetition rate the ozone will accumulate faster. However, a design change may be implemented in a modified system to alleviate this effect. According to some embodiments, it is possible to assume that a modified system has the same ozone calibration error as a baseline system.

With regard to the tissue ablation parameter, it is noted that the ablation depth for a given laser pulse may vary due to an error in lens calibration. In some instances, there may be a calibration error of the pulse ablation depth. Daily lens calibration may be done by a spherical ablation on plastic, performed with a laser with pre-calibrated fluence. The created plastic lens can be measured with a lensometer for a baseline system (accuracy ~0.15D) or with a Hartmann-Shack aberrometer for a modified system (accuracy 0.05D). In some cases, the lens is intended to have a spherical refraction of 4D. This is related to the -4D in calibration discussed below. Therefore, the ablation accuracy on the plastic, defined as relative accuracy of optical path difference in the plastic, $\delta OPDP_p/OPD_p$, may be about 3.75% for a baseline system and 1.25% for a modified system. The ablation accuracy on a tissue may be different. To find the difference, it is possible to use **FIG. 33**, which shows the measured ablation optical path difference compared to laser pulse fluence. The linear trend lines near the nominal fluence ($I_0$=160 mJ/cm$^2$) are added to the plots with one outlier removed from the cornea data. It is possible to approximate the plastic and tissue curves near the nominal fluence by the linear trends as follows:

$$OPD_p = \alpha_p \cdot I + A_p$$

$$OPD_t = \alpha_t \cdot I + A_t$$

Here the coefficients $\alpha_p$ = 0.2079, $A_p$ = 17.662, $\alpha_t$ = 0.4716, and $A_t$ =10.68$^2$ can be estimated with the data selected from **FIG. 33.** The daily calibration may be intended to set the fluency level at the nominal value. The fluency level, measured with the plastic ablation, can be derived as $I = (OPD_p - A_p) / \alpha_p$. The calibration procedure may adjust the fluency to the nominal level, 160 mJ/cm$^2$. Accuracy of the adjustment process may be about $\delta I/I$ = 1%. Any deviations from the nominal value may induce errors in the tissue ablation depth. With uncorrelated errors in the inaccuracy in the plastic ablation measurement and in the fluency adjustment it is possible to find the relative inaccuracy of the tissue ablation depth as follows:

$$std\left(\frac{\delta OPD_t}{OPD_t}\right) = \frac{\sqrt{\left[std\left(\frac{\delta OPD_p}{OPD_p}\right)\right]^2 \left(1 + \frac{A_p}{\alpha_p I}\right)^2 + \left[std\left(\frac{\delta I}{I}\right)\right]^2}}{1 + \frac{A_t}{\alpha_t I}}$$

Given the relative measurement accuracy on the plastic, $\delta OPD_p/OPD_p$, it is possible to estimate the relative ablation error on tissue as $\delta OPD_t / OPD_t$. The standard deviation of this error std may be about 5% for a system using a lensometer for the lens calibration and may be about 2% for a system using Hartmann-Shack aberrometer. It is possible to use one of these values to model the ablation depth error as a normally distributed value, which changes randomly after each lens calibration.

As an illustrative example, a calibration plastic lens may be prepared and taken to a lensometer where a technician compares the expected value with a measured value. For example, the expected value may be -4D, and the measured value may deviate from that (e.g. -3.8D or -4.2D). If the technician does not or cannot accurately read the measured value, which may be due to any of a variety of reasons, then the technician's error can be carried over to the actual results. For example, if the technician interprets the measured value to deviate 0.125D from the actual measured value, the difference between the interpreted measured value and the actual measured value could introduce a systemic error for every patient treated with that system following calibration. An error budget analysis can be used to analyze and identify such errors, and therefore can be used to suggest improvements to a surgical process, for example by replacing the manual calibration performed by the technician with an automatic technique where the lens is evaluated with a wavefront sensor.

As indicated by **FIG. 33**, as the fluence level increases, both tissue ablations and plastic ablations become deeper. As also shown here, the ablation depth as a function of fluence level may vary between plastic ablation and tissue ablation. For example, as fluence level increases, the depth of the tissue ablations become greater relative to the depth of the plastic ablations. Hence, embodiments of the present invention encompass systems and methods for accounting for this difference between tissue and plastic response to fluence levels, when calibrating or administering a treatment. In some instances, it is possible to increase system accuracy by accounting for differences in plastic/tissue ablation depths that are dependent on fluence level.

As depicted in **FIG. 30**, a laser spot or spot shape parameter may include a spot size parameter and a spot shape or laser pulse shape parameter. A spot size parameter may include an iris size parameter, a control parameter, a measurement parameter, or a target plane level parameter. With regard to a spot size parameter, it is noted that laser spot size can be controlled by the iris. Switching the iris between different sizes may add about 10um random error to the spot size, which is possible to model with normally distributed value randomly changing after each iris size change. There may also be a systematic error in the spot size, which may depend on the accuracy of spot size calibration. The spot size calibration can be done by comparing images of a very precise 10 mm circular chrome-dot spot and the laser spot. The camera resolution may be about 640 x 480 pix. The chrome-dot image may almost fill in the camera view, and it may be possible to measure its size with about 1 pix accuracy. Accordingly, an inaccuracy of the image-based measurement may be below 25 um. According to some models, it is possible to simulate the spot size error by stretching the nominal spot field proportionally to fit the deviated size. The magnitude of the stretch may be a normally distributed random value, which may be constant for each system but may change randomly from system to system.

A laser pulse shape or spot shape parameter may include an ellipticity parameter or a uniformity parameter. With regard to the ellipticity parameter, it is noted that spot deviation from a circular shape may be a systematic error for each system and may be described in a first order approximation as an elliptic shape, parameterized with the different sizes along major and minor axis. According to some embodiments, it is possible to characterize the magnitude of the deviation by a stretch parameter, defined as the difference between major radius and minor radius, which may reach 100 $\mu$m. In some models, it is possible to stretch a circular nominal spot by a stretch value along a randomly directed major axis and squeeze by the same value in the orthogonal direction. A stretch magnitude may be assumed normally distributed with $3\sigma = 50$ $\mu$m and the direction may be distributed uniformly over the full circle. In some instances, both values may stay constant for each system but may change randomly from system to system. With regard to the uniformity parameter, it is noted that a laser beam can be produced by mixing several beamlets, which can homogenize the spot intensity. A simple parameterization of remaining intensity variation within the spot can be done by a linear intensity trend. This random error may reach a maximum of 2% between the opposite sides of the spot. In some instances, it is possible to simulate the spot non-uniformity by multiplying the spot field by a randomly oriented linear trend, which may equal 0 at the spot center and linearly deviates along that orientation closer to the spot periphery. The trend magnitude can be defined as a maximum intensity deviation at the spot periphery as compared to a spot center. In some instances, the magnitude may be assumed to be normally distributed with $3\sigma = 1\%$ and a gradient direction can be a random value distributed uniformly over a full circle. In some instances, both values may stay constant for each system but may change randomly from system to system.

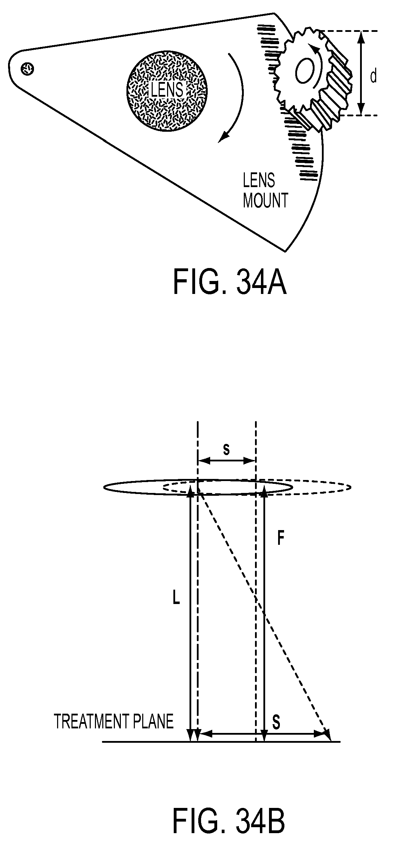

As further shown in **FIG. 30**, a position parameter may include a spot steering parameter or an eye location parameter. A spot steering parameter may include a spot position parameter or a steering scaling parameter. With regard to a spot steering parameter, it is noted that a laser spot position can be controlled by a steering lens, which can move in X and Y directions. **FIGS. 34A and 34B** show aspects of lens steering according to embodiments of the present invention. In a baseline system, the steering lens may be controlled by two rotating mounts each directed by a rotating pinion. As an example, **FIG. 34A** shows a rotating lens mount driven by a rotating pinion. A steering lens, which may be attached to approximate the center of each mount, can move along wide arcs approximately in X and Y directions. According to some embodiments, the pinion diameter may be about 1/8", and hence its full rotation can move the lens mount by 10 mm and the lens, located in the middle of the mount may move by 5 mm. The lens shift can be translated to the spot shift. For example, **FIG. 34B** shows the spot shift due to the shift in the steering lens. The lens shift can be translated to the spot shift as shown here. This simple geometry leads to the following equation, connecting the lens shift, s, and the spot shift, S:

$$S = s \cdot \left( \frac{L}{F} - 1 \right)$$

Here, F is the lens focal length and L is the distance between the lens and the treatment plane. According to some embodiments, an empirically measured amplification coefficient can be $K_{spot} \equiv S / s$ =1.81. With this coefficient it is possible to have the spot shift per one pinion revolution about 8.4 mm. This number can be design-specific and can be about the same for all the tools.

A modified system may have a different lens steering mechanism, controlled with a server motors separately in X and Y directions. Spot position can always have a random error, which may be defined by mechanical tolerances of the steering system. It is possible to simulate this error as a Gaussian random error for both X and Y coordinates, changing after each spot repositioning with $3\sigma = 20um$.

In addition to the random error there may be systematic errors in spot positioning, caused by small deviations in mechanical assembly, thermal stretches, and the like. Among these errors, steering scaling and positioning nonlinearity (e.g. in a baseline system) may be particularly relevant. With regard to a steering scaling parameter, it is noted that after the hyperopia module is mounted, the iris adjustment can be performed to ensure focusing on the treatment plane. This, as well as mechanical tolerances of lens mount, may cause deviations in the scaling factor, $K_{spot}$. The magnitude value of these deviations may be as high as 1%. The scaling error may stay constant for each system but may change randomly from system to system. According to some embodiments, in simulations it is possible to assume Gaussian distribution for the scaling error with $3\sigma$ =1%.

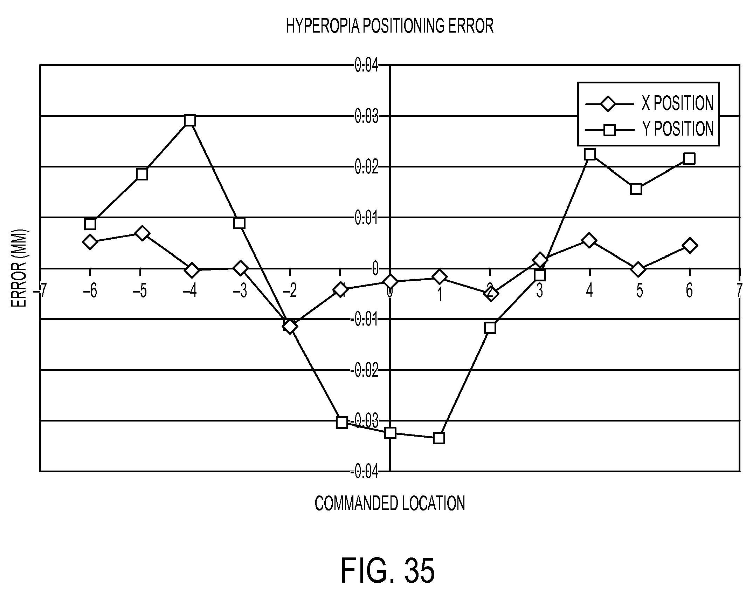

With regard to a positioning nonlinearity parameter, it is noted that a slight offset or a tilt in the pinion mount can make the pinion rotation de-centered. This may cause slight periodic deviations of the lens movement from a pure linear monotonic shift. The period of these deviations may be about 9 mm as defined by one full pinion revolution. Measured positioning errors for ablation spots on a silkscreen along X and Y directions show periods close to this value, which supports the estimate as indicated in **FIG. 35**, which depicts spot positioning errors along the X and Y arcs. According to some embodiments, the X=0 position may always be calibrated, and therefore the nonlinearity errors may cross zero point at zero coordinate. In some instances, a model of position nonlinearity may include periodically changing errors in X and Y directions with the period 8.5 mm and with random amplitude and phase as follows:

$$\delta x = A_X \cdot \left[ \sin\left( \frac{x}{P_{nl}} + \varphi_X \right) - \sin(\varphi_X) \right]$$

$$\delta y = A_Y \cdot \left[ \sin\left( \frac{y}{P_{nl}} + \varphi_Y \right) - \sin(\varphi_Y) \right]$$

The values $A_X$, $\varphi_X$, $A_Y$, $\varphi_Y$ may be constant for each system and may randomly change from system to system with Gaussian distribution for amplitudes and uniform distribution for phases. In some instances, the magnitude of nonlinearity, defined by the amplitude of periodic function in the equations above is tool-specific. It may depend on the pinion mount quality and may be limited by tolerances of mechanical assembly. It is possible to observe from several measurements that the amplitude can be as high as 40 um. A value of 50 um may be considered as $3\sigma$ for certain simulations, according to embodiments of the present invention.

As shown in **FIG. 30**, an eye location parameter may include an eye tracking parameter or an eye or iris registration parameter. With regard to an eye location parameter, it is noted that errors in ablation position may be caused by patient eye movements both in XY and torsional directions. In some instances, ablation may be intended to be aligned with the eye's pupil, which may be ensured by the eye registration system. In some instances, an eye tracker may follow fast eye movements during fixation, and spot positions may be expected to be moved accordingly. Both systems may have finite accuracy, which may cause systematic and random spot location errors.

With regard to an iris or eye registration parameter, it is noted that an iris XY registration may be intended to compensate for a difference between pupil-to-iris shifts for wavefront measurement data and the same eye image during the surgery. In some instances, the accuracy of XY eye registration is below $3\sigma = 50um$. A similar compensation for a torsional eye rotation may be performed in a modified system by a torsional iris registration with an accuracy below $3\sigma$ =1 deg. According to some embodiments, an iris registration can be performed once before treatment. Its error may be the same for each pulse within the treatment, and, therefore, systematic for each treatment. Accordingly, the magnitude of the eye registration error may be constant for each treatment but may change randomly from treatment to treatment with the Gaussian probability distribution. In some simulations, it is possible to apply these errors to XY coordinates of every pulse in a treatment (the same XY shift for each pulse) and a torsional rotation for the entire ablation surface.

With regard to an eye tracking parameter, it is noted that an eye tracker can follow random eye movements during surgery. In some instances, a baseline system may track only translational movements, whereas a modified system may have an eye tracker that follows both translational movements and torsional rotations. Accuracy of the eye tracking device may be affected by its finite speed, tracking latency (defined by data acquisition delay, laser pointing time, software data processing, and laser charging time) and finite accuracy of the eye location measurement. The latter may have both random and systematic error. However, the systematic part, which stays the same during the treatment, may be negligible compared to the eye registration error and can be ignored in some simulations. **Table 2** depicts aspects of eye tracker parameters associated with controlling the tracking performance, according to embodiments of the present invention.

| Table 2 | | |
|---|---|---|
| **Parameter** | **Baseline system** | **Modified system** |
| XY tracking precision | 20um (3$\sigma$) | 30um (3$\sigma$) |
| XY tracking latency | 0.05 sec | 0.03 sec |
| Torsional tracking precision | N/A | 0.5 deg (3$\sigma$) |
| Torsional tracking latency | N/A | 0.1 sec |

In some instances, it is possible to calculate the tracking position as the actual position, delayed by *latency* + 0.5 · *ETperiod* (where *ETperiod* is reciprocal of the repetition rate) with addition of a Gaussian random error. The same algorithm can be applied independently to XY, and (e.g. for a modified system) to torsional coordinates. A baseline system may not include a torsional eye tracker, and it is possible to estimate torsional positional errors in a baseline system by simulating torsional deviations with an eye movements model as further discussed elsewhere herein.

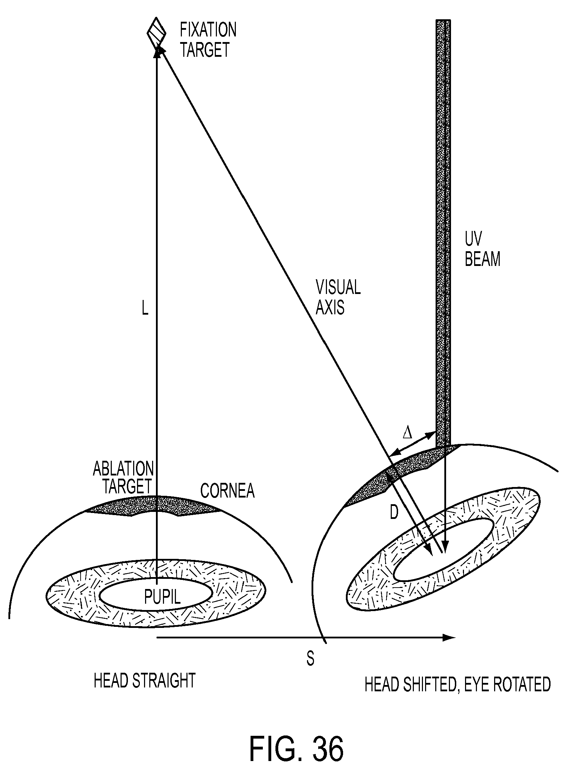

According to some embodiments, techniques may involve a head positioning parameter (e.g. head shift/rotation, or 6-Dimensional positioning). Both eye registration and eye tracker can be intended to locate a pupil relative to its position during wavefront measurement. Yet the pupil shift may not be the only source of eye positioning errors. Head movements and/or head rotations may change the tilt of the visual axis during a patient eye fixation. A difference of the visual axis tilt on the operating table from the tilt during wavefront measurement may induce an error, caused by the finite distance between the eye pupil and the corneal surface, as depicted in **FIG. 36**. As illustrated here, head shift has an effect on positioning error. The laser beam may aim toward a pupil position, while it is intended to ablate at a position on the cornea plane. Based on the geometry shown in **FIG. 36**, the distance between the corneal and pupil positions may be defined by the following formula:

$$\Delta = D \cdot \frac{S}{L}$$

Here *L*=30cm is the distance to a fixation target, *S* is the head shift, causing the eye rotation, *D*=3.5mm is the distance between the pupil plane and corneal surface. For a head shift *S*=0.5cm the positioning error can be about 50um. In some instances, the head shift error may have the same effect as the eye registration error. For example, the head shift error may add a bias to pulse positioning, which is the same for each pulse. Both errors may have the same statistics. Both errors may stay the same during the operation and may randomly change from treatment to treatment. Although these two errors have different origin, they may have comparable magnitude and may be added together in simulations. According to some embodiments, it is possible to increase system accuracy by accounting for differences in head shift (visual axis tilt) on operating table or during treatment as compared with head shift during diagnosis or wavefront measurement.

**Table 3** summarizes various simulated error-inducing factors for baseline and modified systems, and provides possible tolerances for error budget simulations, according to embodiments of the present invention.

Table 3

| Error Type | Error source name | baseline max value | modified max value | Pulse-to-pulse variations | Description |
|---|---|---|---|---|---|
| Pulse ablation depth | calAblation | 5% | 2% | Systematic | Calibration accuracy of ablation depth (lens cal) |
| | errAblation | 9% | 9% | Random | Precision of a single pulse ablation depth |
| | Integrator | 6% | 6% | Dynamic | Amplitude of integrator transmission oscillations |
| | calOzone | 1.8% | 1.8% | Dynamic | Max ozone calibration error |
| Laser spot | calSpotSize | 25μm | 25μm | Systematic | Accuracy of spot size calibration |
| | errSpotSize | 10 μm | 14 μm | Random | Precision of spot size calibration |
| | SpotEllipt | 50 μm | 50 μm | Systematic | Spot ellipticity factor (max deviation from circle) |
| | SpotUnif | 2% | 2% | Random | Spot uniformity; max fluence deviation from |
| Spot steering | errSpotPos | 20 μm | 20 μm | Random | Precision of spot positioning |
| | xcalePos | 1.0% | 1.0% | Systematic | Accuracy of spot positioning scaling factor |
| | xyNonlin | 50 μm | N/A | Dynamic | Amplitude of periodic positioning errors |
| Eye location | xyRegist | 50 μm | 50 μm | Systematic | Accuracy of XY iris registration |
| | torRegist | 1 deg | 1 deg | Systematic | Accuracy of torsional iris registration |
| | xyETsamp | 17ms (60Hz) | 4.2ms (240Hz) | Random | XY tracker sampling period |
| | xyETaccur | 20 μm | 30 μm | Random | Positioning precision of XY tracker |
| | xyETlatene | 0.05 sec | 0.03 sec | Random | Latency of XY tracker |
| | torETsamp | N/A | 83 ms (12Hz) | Random | Torsional tracker sampling period |
| | torETaccur | N/A | 0.5 deg | Random | Positioning precision of torsional tracker |
| | torLatency | N/A | 0.1 sec | Random | Latency of torsional tracker |
| | torSpeed | 0.1 deg/sec | N/A | Random | Eye torsional random-walk speed |

In some instances, a large percentage of eye movements (e.g. 99%) can be captured by a 20Hz tracker. Relatedly, trackers operating at a higher frequency can detect or account for saccadic eye movements and help to ensure that laser ablations are not delivered during the course of a saccadic eye movement. In some instances, it is desirable to take multiple measurements before each laser pulse is delivered, so as to determine where the eye is and at what velocity the eye is moving. In some instances, it is desirable to have an eye tracker that operates at a higher frequency than the frequency of the ablation beam delivery laser. For example, it may be desirable to provide an eye tracker that operates twice or three times the frequency of a laser, and to obtain more than one tracking measurement prior to delivering an individual pulse. As indicated in **Table 3**, a modified system may include an eye tracker that operates at 240 Hz. In some embodiments, a system may be configured to confirm a certain number of consistent tracking measurements before delivering an ablation pulse, and to validate the tracking measurements ensure consistency between the measurements.

Embodiments of the present invention encompass techniques for simulating fixational eye movements. In some instances, the tracking performance may depend on the eye movement, yielding better results when the eye moves smoothly and slowly, and less accurate results when they eye exhibits sudden fast moves. Estimation of the eye tracking accuracy can be done with a realistic simulation for the eye movement. In some instances, microscopic eye movements during visual fixation may include a slow random-walk drift with intermittent micro-saccadic jumps, resetting back to a close vicinity of the fixation position. In some instances, torsional eye movements may also be close to a random drift, but saccadic movements may be less pronounced. Because eye movements may be random, the tracking error may also be random. It is possible to model eye movement as a random walk, comprised of a sequence of ballistic movements for some period, followed by a random change in direction (random angle for XY movement or random sign for torsional rotation). The ballistic period (collision time) may also be random. Once the random walk exceeds the limiting distance from the reference point, the position can be re-set back close to the reference. The limiting distance can be a random value, uniformly distributed around the value *maxWander* (within the interval [0.5\**maxWander*; 1.5\**max Wander*]). The reset position distance from the reference can also be random, uniformly distributed around the reference (within a circle of radius *saccadAccuracy*). A similar model can be implemented for movements in XY plane and for torsional rotations. The latter, however, may not include saccadic resets in some instances.

**Table 4** depicts aspects of eye movement parameters according to embodiments of the present invention.

| Table 4 | | |
|---|---|---|
| **Parameter** | **Value** | **Description** |
| XY speed | 2 mm/sec | Speed of ballistic moves during random walk steps |
| XY collisionT | 0.022 sec | Average duration of random walk steps |
| XY maxWander | 0.3 mm | Average limiting distance for random walk |
| XY saccadAccuracy | 0.1 mm | Size of reset area for saccadic jumps |
| Torsional speed | 0.1 deg/sec | Speed of ballistic torsional moves during random walk |
| Torsional collisionT | 0.05 sec | Average duration of torsional random walk steps |

In some simulations, it is possible to model a random eye movement and then estimate the tracking errors, based on that movement. These random errors may depend on both eye tracking parameters (e.g. as shown in **Table 3**) and eye movement parameters (e.g as shown in **Table 4**). In some instances, the latter may be assumed to be constants. In some instances, eye tracking parameters may be considered to be variable, and it is possible to estimate errors, caused by each of them separately or together with other error sources. For a baseline system which does not have torsional tracking, it may be possible to model the XY tracking errors and assign position errors in torsional direction defined entirely by the random eye torsional movement.

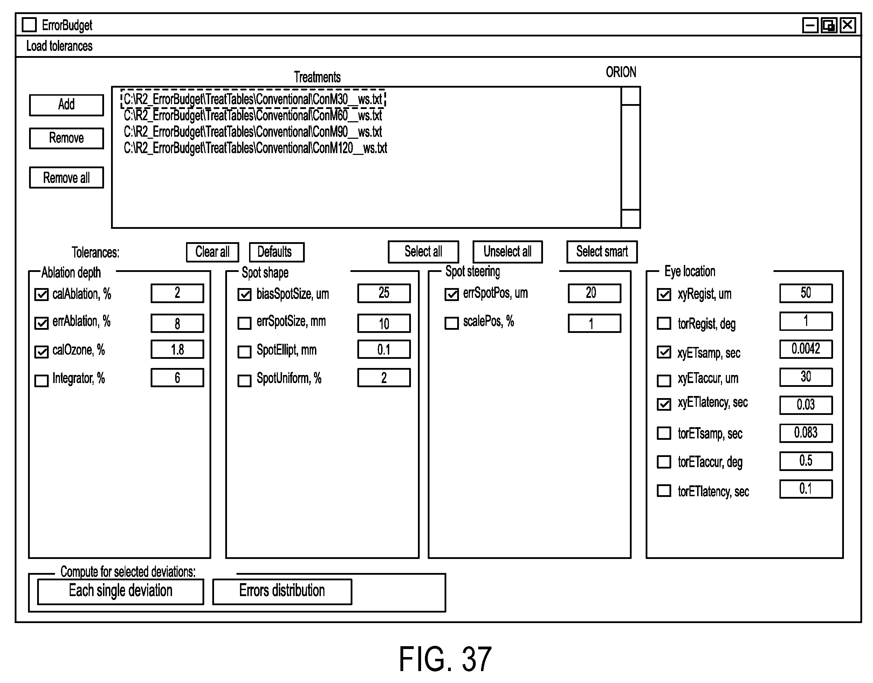

**FIG. 37** depicts a main window of a graphical user interface software or product for error analysis (e.g. numerical analysis) according to embodiments of the present invention. Such software or products can be used by hardware and system engineers, and can allow several operations. For example, it is possible to load tool-specific default tolerances for analyzed parameters. Exemplary tolerance values or tolerance value files are provided elsewhere herein and can be stored in a text file with the following format:

```
TYPE            toolType
# Panel
ParameterName_1    maxError_1    unit_1    description_1
ParameterName_2    maxError_2    unit_2    description_2
ParameterName_3    maxError_3    unit_3    description_3
```

The first line can define the tool type (e.g. 1 for a baseline system, 3 for a modified system). Lines with the '#' symbol can be panel names. Empty lines can be ignored. Each non-comment line can define the parameter name, its default tolerance value, its unit, and the parameter description string.

Further, it is possible to load one or more treatment tables (e.g. converted into the text format), edit a tolerance value for each parameter, select a subset of parameters, and for each of the selected parameters, compute RMS error for the ablation surface and also errors in sphere and cylinder refraction, as well as high order aberrations (e.g. spherical aberration, coma, and trefoil). Mean, standard deviation, maximum, and 95th percentile can also be computed for each error.

Embodiments of the present invention encompass various analysis techniques. In a factor analysis technique, for each system parameter tolerance, assuming the other parameters are perfectly substantially accurate (e.g. their deviations are zero), it is possible to compute errors for each of the cases. Such computations may be done by Monte Carlo simulation, where the parameter deviates by random values within the parameter tolerance. In an error distribution function technique, a simulation may assume that all error factors (e.g. with the exception of the iris tracker period and latency) have random values with Gaussian distribution with the standard deviations defined by one third of their tolerances. The distribution of errors can be computed, including its mean, standard deviation, min, max, and 95th percentile. Aspects of exemplary embodiments are depicted in **FIGS. 24 and 28**, for example.

According to some embodiments, it is possible to use various treatment table types. For example, in some simulations it is possible to use sets (e.g. three sets) of treatment tables, corresponding to conventional treatments for myopia (3D, 6D, 9D, 12D), VSS refractive treatments (myopia, hyperopia, myopic astigmatism, hyperopic astigmatism, and mixed astigmatism with no high order aberrations), as well as CustomVue™ treatments. Simulations can be performed for both baseline system and modified system tools.

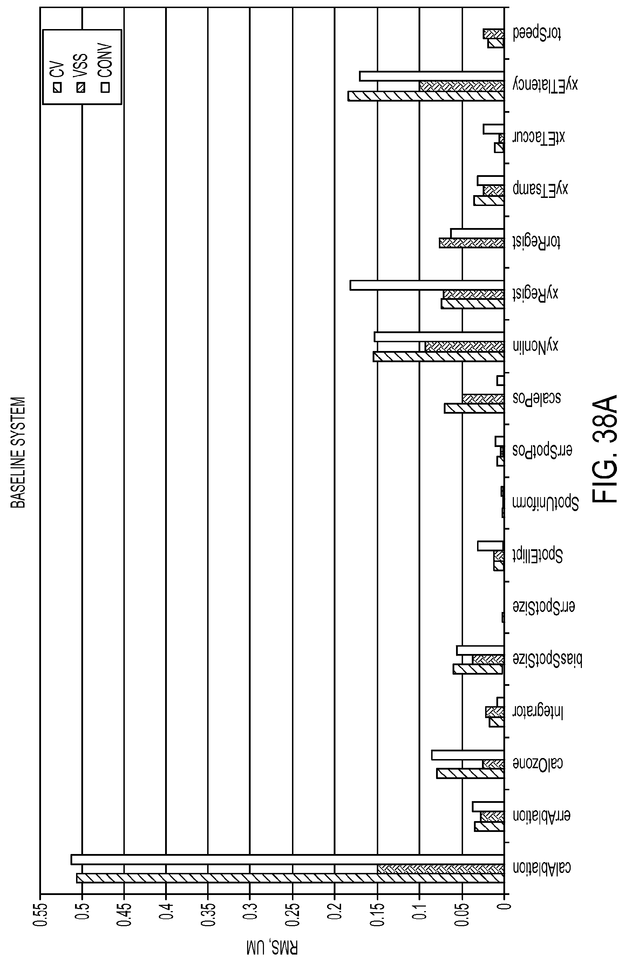

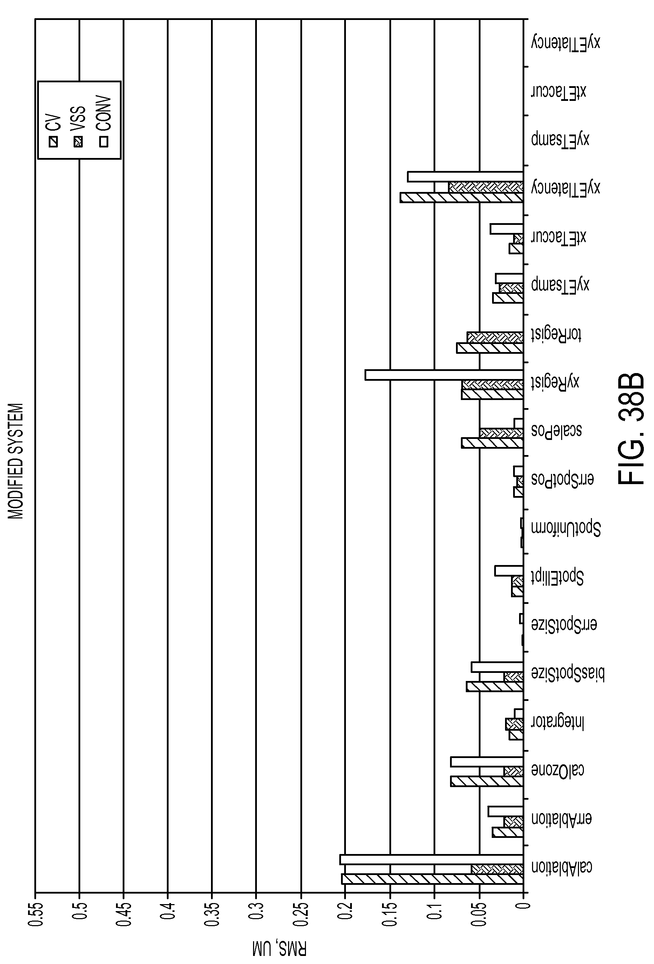

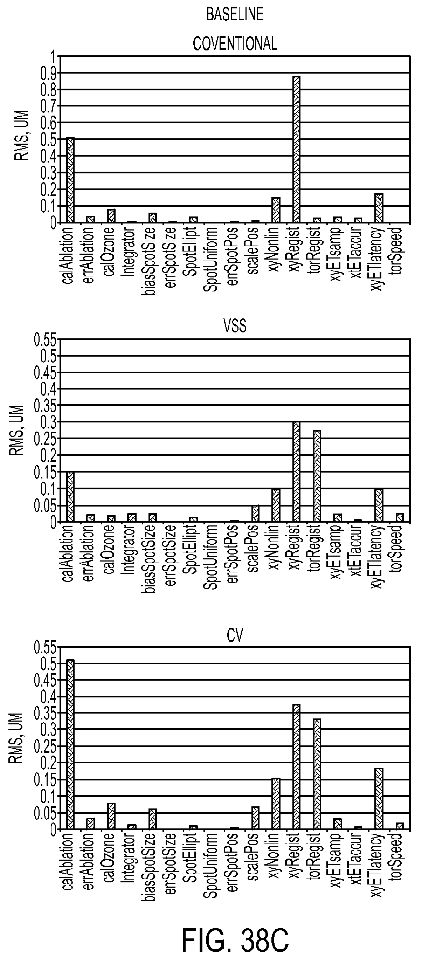

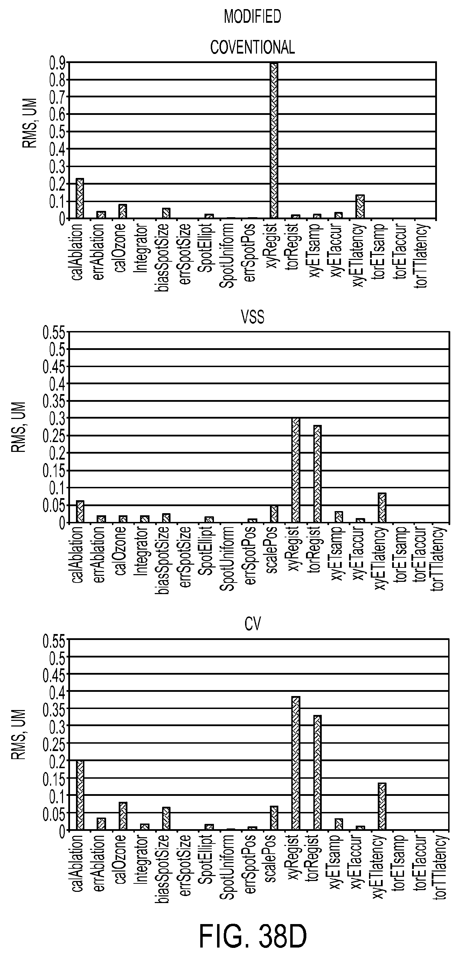

**FIGS. 38A and 38B** depict factor analysis (maximum 95th percentile RMS for all cases) for various simulated errors in baseline and modified systems, respectively, according to embodiments of the present invention. **FIGS. 38C and 38D** depict factor analysis (maximum 95th percentile RMS for all cases) for various simulated errors in baseline and modified systems, respectively, according to embodiments of the present invention.

The factor analysis for these various treatment samples indicate that according to some embodiments, the following error sources are virtually insignificant (compare with list in **Table 3**): Baseline System (errAblation, Integrator, errSpotSize, SpotEllipt, SpotUniform, errSpotPos, xyETsamp, xyETaccur, torSpeed) and Modified System (errAblation, Integrator, errSpotSize, SpotEllipt, SpotUniform, errSpotPos, xyETsamp, xyETaccur, torETsamp, torETaccur, torETlatency). These parameter deviations make $95^{th}$ percentile errors below 0.05um RMS and below 0.025um for SA, coma, and trefoil.

**Table 5** includes error sources having considerably higher errors (e.g. RMS>0.05 um).

**Table 5**

| BASELINE | MODIFIED | Description |
|---|---|---|
| cal Ablati on | cal Ablati on | Calibration accuracy of ablation depth (lens Cal) |
| cal Oz one | cal Oz one | Max oz one calibration error |
| cal SpatSize | cal SpotSize | Accuracy of spot size calibration |
| a calePos | a calePos | Accuracy of spot positioning scaling factor |
| x yNonlin | | Amplitude of periodic positioning errors |
| x yRegist | x xyRegist | Accuracy of XY iris registration |
| torRegist | torRegist | Accuracy of torsional iris registration |
| x yE Tlatency | x yE Tlatency | Latency of XY tracker |

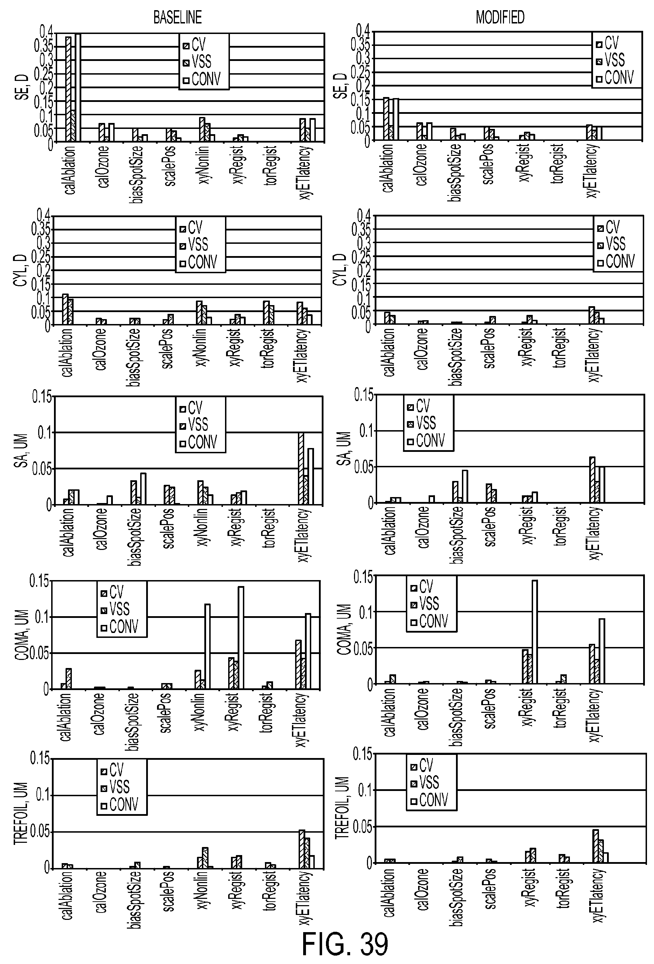

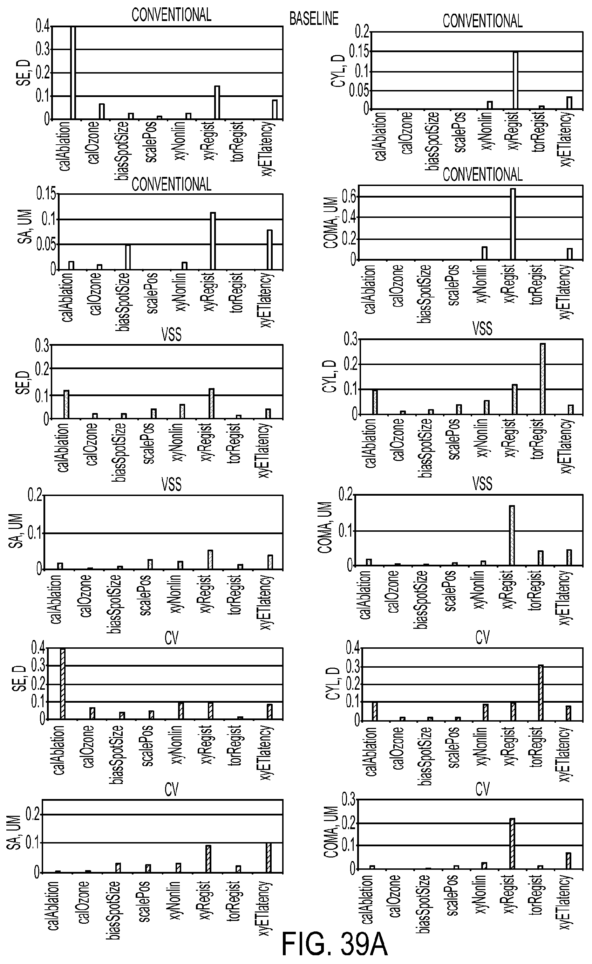

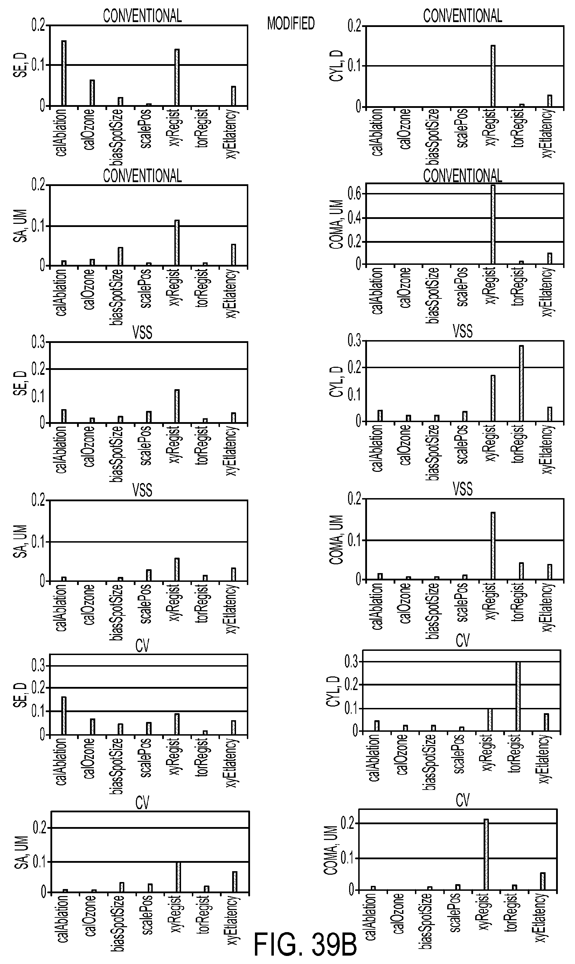

Corresponding to these significant error sources, **FIG. 39** depicts the factor analysis (maximum 95th percentile for all cases) for both low order aberrations and high order aberrations, for baseline and modified systems, according to embodiments of the present invention. Relatedly, **FIGS. 39A and 39B** depict factor analysis (maximum 95th percentile for all cases) for aberrations with significant error sources for baseline and modified systems, respectively, according to embodiments of the present invention. Such analysis shows that only very few error sources can make a significant difference in aberrations, for example as depicted in **Tables 6 and 7**, which illustrates significant error sources for low- and high-order aberrations, for baseline and modified systems, respectively.

**Table 6**

| | cal Ablation | cal Ozone | xy Nonlin | xy Reqist | tor Resist | xyET latency |
|---|---|---|---|---|---|---|
| **SE, D** | 0.40 | 0.06 | 0.09 | | | 0.68 |
| **Cyl, D** | 0.11 | | 0.09 | | 0.08 | 0.08 |
| **SA, um** | | | | | | 0.10 |

(continued)

|  | cal Ablation | cal Ozone | xy Nonlin | xy Reqist | tor Resist | xyET latency |
|---|---|---|---|---|---|---|
| **Coma, um** |  |  | 0.12 | 0.14 |  | 0.10 |
| **Trefoil, um** |  |  |  |  |  | 0.05 |

**Table 7**

|  | Cal Ablation | cal Ozone | xy RegIst | tor Regist | xyET latency |
|---|---|---|---|---|---|
| **SE, D** | 0.16 | 0.06 |  |  | 0.06 |
| **Cyl, D** |  |  |  | 0.08 | 0.07 |
| **SA, um** |  |  |  |  | 0.06 |
| **Coma, um** |  |  | 0.14 |  | 0.09 |
| **Trefoil, um** |  |  |  |  |  |

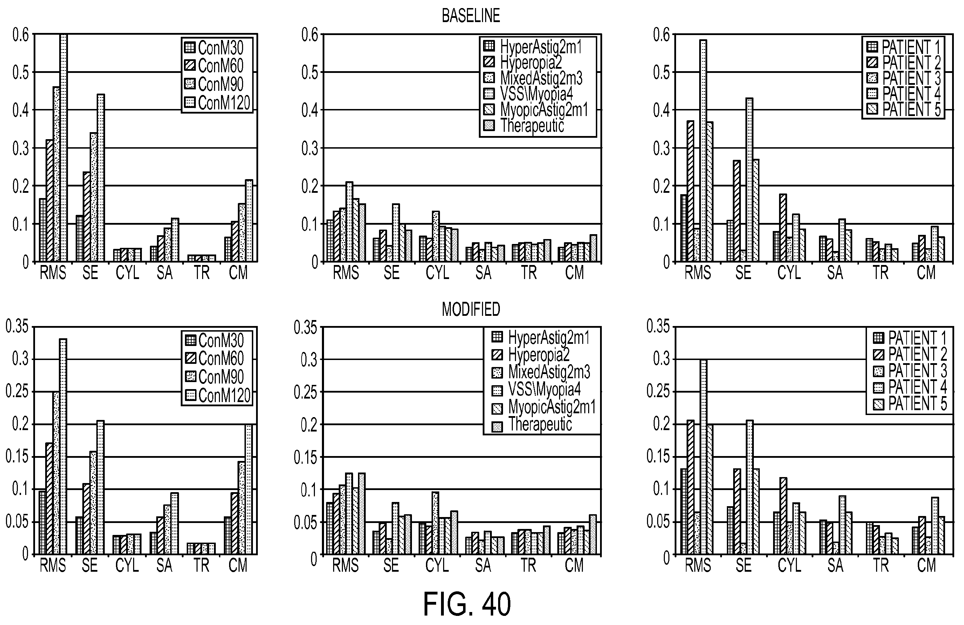

Monte Carlo simulations with all error factors combined can provide a realistic estimate of ablation errors, including RMS, refractive and high-order aberrations, as depicted in **FIG. 40**, which shows total error estimates (maximum 95th percentile) with all error sources included.

According to the embodiment represented in **Tables 6 and 7**, it is possible to see that the two most significant error sources are calAblation (calibration accuracy of ablation depth; lens calibration inaccuracy) and xyRegist (iris XY registration accuracy). These factors mostly affect SE and Coma errors. A modified system may have substantially smaller SE error as compared to a baseline system, for example where the modified system uses an aberrometer for lens calibration and the baseline system uses a lensometer for lens calibration. Such differences can be characterized by the error analysis techniques disclosed herein.

In some instances, a modified system may use a hyperopia module design which includes lighter components in the beam placement lenses, so there is less inertia and the beam can change direction rapidly, thus diminishing potential misplacements of the beam, and allowing the beam to run at higher frequency pulse rates. Such modified hyperopia modules can remove or reduce another important error source, namely xyNonlin (periodic positioning errors). Total error RMS for a modified system can be below 0.35um, which is about twice smaller than for a baseline system. High-order aberrations are generally below 0. 1um, although some may reach as high as 0.2um for very high myopia cases. According to some embodiments, the high level of coma is caused mainly by iris XY registration error and also by XY tracker latency.

According to some embodiments, a tolerance file (containing exemplary tolerance values) for a baseline system may include the following:

```
# Ablation depth
calAblation 2 % Ablation depth calibration accuracy
errAblation 8 % Ablation depth fluctuations
calOzone 1.8 % Ozone calibration error
Integrator 6 % Fluence oscillations due to rotating integrator


# Spot shape
biasSpotSize 25 um Spot size accuracy
errSpotSize 10 um Spot size precision
SpotEllipt 0.1 mm Spot ellipticity: size difference between main axes
SpotUniform 2 % Spot non-uniformity: fluence max deviation from the center


# Spot steering
errSpotPos 20 um spot positioning precision
scalePos 1 % spot positioning scaling accuracy
xyNonlin 50 um Max amplitude of periodic positioning errors


# Eye location
```

xyRegist 50 um XY eye registration error
torRegist 1 deg torsional eye registration error
xyETsamp 0.0042 sec XY eye tracker sampling period (1 / 200Hz)
xyETaccur 20 um XY eye tracker accuracy
xyETlatency 0.05 sec XY eye tracker XY latency
torSpeed 0.1 deg/sec Eye torsional random-walk speed

According to some embodiments, a tolerance file (containing exemplary tolerance values) for a modified system may include the following:

# Ablation depth
calAblation 2 % Ablation depth calibration accuracy
errAblation 8 % Ablation depth fluctuations
calOzone 1.8 % Ozone calibration error
Integrator 6 % Fluence oscillations due to rotating integrator

# Spot shape
biasSpotSize 25 um Spot size accuracy
errSpotSize 10 um Spot size precision
SpotEllipt 0.1 mm Spot ellipticity: size difference between main axes
SpotUniform 2 % Spot non-uniformity: fluence max deviation from the center

# Spot steering
errSpotPos 20 um spot positioning precision
scalePos 1 % spot positioning scaling accuracy

# Eye location
xyRegist 50 um XY eye registration error
torRegist 1 deg torsional eye registration error
xyETsamp 0.0042 sec XY eye tracker sampling period (1 / 240Hz)
xyETaccur 30 um XY eye tracker accuracy
xyETlatency 0.03 sec XY eye tracker XY latency
torETsamp 0.083 sec torsional eye tracker sampling period (1 / 12Hz)
torETaccur 0.5 deg torsional eye tracker repeatability
torETlatency 0.1 sec torsional eye tracker latency

In some instances, inverse sensitivity analysis can produce upper limits on system tolerances, which may be allowed for a given system quality. Optionally, error linearity analysis can be used to check the importance of high system variations. According to some embodiments, simultaneous analysis of different ablation plans can be used to compare the robustness and quality of different treatment types. In some instances, given system parameter tolerances, a prediction can be made for the surgical outcome based on pre-operational diagnostics for a patient. Examples of treatment tables provided herein can be used as patient-specific estimations of errors.

According to some embodiments, a variety of error sources have been found to be too small to have any considerable effect on system performance as compared to other more important factors. These include fluctuations of the treatment plane, skewed XY steering (e.g. for a modified system), and laser arm vibrations.

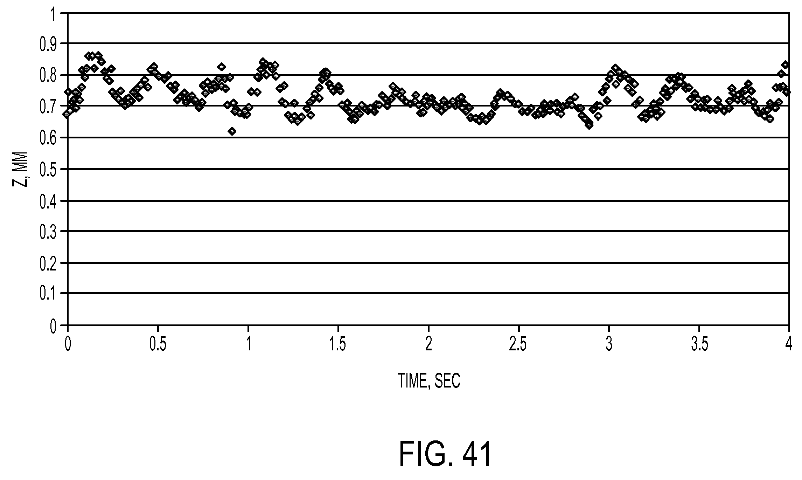

With regard to fluctuations of the treatment plane, it is noted that the patient's cornea may be moving up and down during the treatment, for example as depicted in FIG. 41. These near periodic movements can be caused by the patient heart beat or breathing, or oscillations of the patient chair. FIG. 41 illustrates typical vertical (Z) oscillations of the patient cornea measured with a baseline system eye tracker.

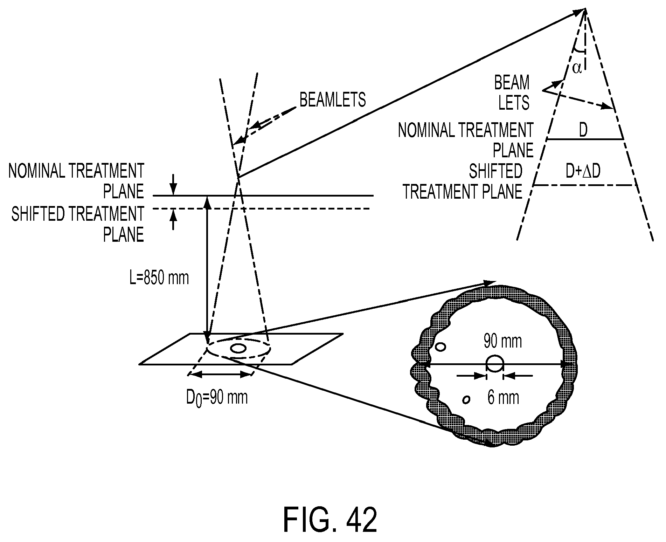

Further, it is noted that vertical oscillations of the cornea surface may cause divergence of the laser beamlets and widening of the laser spot on the cornea, as depicted in **FIG. 42**, which illustrates defocusing of the laser spot. According to some embodiments, the divergence angle, $\alpha$, can be estimated from experiment results, where a greatly widened spot was obtained by illuminating a surface at a distance $L = 850mm$ from the laser focus with the laser beam. Diverging beamlets formed a ring of diameter $D = 90mm$ on that surface, which gives the divergence angle $\alpha = 90 / 850 \approx 0.106rad$. It is possible to estimate the spot widening, caused by a shift of the treatment plane, $\Delta z$, as:

$$\Delta D = 2 \cdot \Delta z \cdot \alpha$$

In some simulations, it is possible to assume that the cornea vertical shift is a sinusoidal function which has amplitude 0.1 mm and is randomly chosen during the treatment. The spot size change can be sinusoidal with $\Delta D = 0.02mm$ amplitude. Also the laser fluency at the corneal surface may be varying accordingly with $\Delta D/D$ relative amplitude, which may be 2% for 1 mm spot size and 0.3% for 6 mm spots. Some baseline system designs include "passive" tracking in Z direction, which only makes sure that the corneal shift does not exceed 2.5 mm value. Smaller variations, however, passed unnoticed, may cause ablations with different spot sizes and also different fluency. This error source may be expected to be rather small, since the effect of 0.02 mm widening may only be relevant for small pulses. But the total volume ablated by these pulses may be about the same, since a wider spot size can be compensated by a lower fluence and the total amount of laser energy delivered by the pulse may stay almost the same.

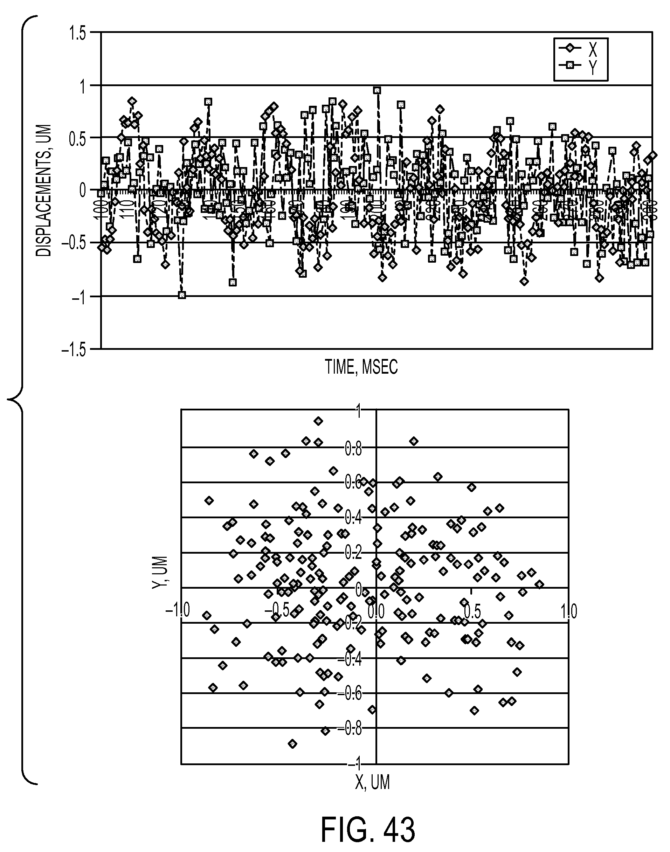

With regard to skewed XY steering (e.g. for a modified system), it is noted that the XY encoders alignment in a modified system may slightly deviate from orthogonal. A mechanical tolerance of 1 mrad may be allowed for the assembly. At a distance of 4 mm from the center this may lead to 4 um positioning error. Compared to an eye registration error of 50 um this may be considered to be a negligible addition. With regard to laser arm vibrations, it is noted that a modified system may experience vibrations of the laser arm, induced mainly by hyperopia module. These vibrations may cause deviations of a laser spot, which can be measured with a light beam reflected from a small mirror located at the treatment plane and projected on a wall. The X and Y components of the wall reflection spot can be charted with respect to time at 1 KHz sampling, for example as illustrated in **FIG. 43**, which shows laser spot vibrations, measured with light reflection at the treatment plane.

As illustrated by the embodiment depicted here, the displacements standard deviation can be 0.65 um and there may be no visible correlation between X and Y components. There may be a periodic trend in the X component with a frequency about 70 Hz. It can be tracked with the 200 Hz eye tracker. Other than that periodicity, the vibrations may appear largely random and, therefore, may be simply added to the tracker positioning error. Compared to the tracker precision (e.g. 6 or 10 um), the vibration caused error is rather small. Accordingly, even if the tracker is completely unable to follow these random vibrations (which is an unlikely worst case scenario), the errors due to these vibrations are negligible. A psychological effect on the system operator can be considered separately.

Embodiments of the present invention encompass various techniques for analyzing the extent to which factors or inaccuracies in system or surgery parameters may contribute to imperfect outcomes of a laser vision correction treatment. Parameters may be prioritized and compared based on the analysis. According to some embodiment, significant error sources may include lens calibration inaccuracy and iris registration inaccuracy. These factors may primarily affect spherical equivalence (SE) and coma errors.

In some instances, a modified system may have a substantially smaller SE error than a baseline system, because it uses aberrometer rather than lensometer for lens calibration. A modified system with a modified hyperopia module design may eliminate or reduce another important error source - periodically oscillating spot positioning errors. Total error RMS for a modified system may be below 0.35 um, which is about twice smaller than for a baseline system. High-order aberrations are generally below 0.1 um, although coma may reach as high as 0.2 um for high myopia treatments. The high level of coma is caused mainly by iris registration error and also by eye tracker latency.

As discussed elsewhere herein, a surgical or laser refractive correction procedure may encompass several stages or aspects, including manual operations as well as patient physiological and psychological responses. Hence, the success or outcome of an operation may be affected by various distinctive types of errors or operating parameters. For example, technological parameters or error types may involve errors in machine performance, calibrations, numerical algorithms, and the like. Physiological parameters or error types may involve individual differences in tissue response and the healing process. Human parameters or error sources may involve deviations from a standard procedure, including such possibilities as a doctor's mishandling of the equipment or a patient's violations of a prescribed behavior. Psychological parameters or error sources may involve patient perception differences, including those which may affect visual acuity measurements. Various objective factors can be classified into certain types (e.g. permanent, alignment, calibration, treatment, and fluctuations), which may have different statistical behavior. In some cases, ablation errors may be considered as either systematic errors or random errors. According to some embodiments, the tolerance values discussed herein may be defined as a three standard deviations level.

According to some embodiments, it is possible to simulate ablation or surgery errors or deviations by calculating two targets, one corresponding to a non-perturbed system with all nominal parameters and no errors, and another with one with one more parameter deviations and errors applied. The difference between these two targets, decomposed into Zernike series for example, may provide an estimate of ablation or surgery errors, caused by these deviations. In some cases, it is possible to use a Monte-Carlo approach, where multiple parameter deviations are randomly applied and the resulting statistics of ablation errors can be calculated. Various types of simulations can be performed. For example, a factor analysis can be performed where a single deviation is applied with no other deviations. This approach makes it possible to compare contributions of all deviations relative to each other. In another example, a total error level analysis can be performed, where all parameter deviations are applied simultaneously and randomly and the resulting statistics of ablation errors can represent a realistic estimate of total ablation errors. Simulations may include main root causes of system errors, such as laser system deviations and eye movements.

By evaluating the relative contribution of various error sources or surgery parameters, it is possible to identify or weight the extent to which those sources or parameters impact an overall error analysis or outcome. In this way it is possible to prioritize or identify target aspects of the surgery for further refinement or improvement. It is also possible to use the analysis to estimate the contribution of a particular error on a treatment, in terms of an impact on clinical results, so that a risk analysis can be performed evaluating the potential impact of the error in a surgical procedure. The error sources or surgery parameters can impact any of a variety of outcome parameters, such as intended energy distribution or uniformity, positional distribution on the corneal surface, beam diameter, and the like.

It can be appreciated by one of skill in the art that all parameters, variables, factors, and the like can be incorporated into method steps or system modules. While the specific embodiments have been described in some detail, by way of example and for clarity of understanding, a variety of adaptations, changes, and modifications will be obvious to those of skill in the art. Although the invention has been described with specific reference to a wavefront system using lenslets, other suitable wavefront systems that measure angles of light passing through the eye may be employed. For example, systems using the principles of ray tracing aberrometry, tscherning aberrometry, and dynamic skiascopy may be used with the current invention. The above systems are available from TRACEY Technologies of Bellaire, Texas, Wavelight of Erlangen, Germany, and Nidek, Inc. of Fremont, California, respectively. The invention may also be practiced with a spatially resolved refractometer as described in U.S. Patent Nos. 6,099,125; 6,000,800; and 5,258,791. Treatments that may benefit from the invention include intraocular lenses, contact lenses, spectacles and other surgical methods in addition to lasers. Therefore, the scope of the present invention is limited solely by the appended claims.

## Claims

1. A method of evaluating error in a vision correction procedure, the method comprising:

   receiving a treatment table containing laser device instructions;
   receiving a set of surgery parameters associated with the vision correction procedure;
   determining an original treatment based on original values for the set of surgery parameters;
   generating a plurality of random modified values for at least one parameter of the set of surgery parameters;
   simulating a treatment based on the plurality of random modified values and the treatment table; and
   evaluating error in the vision correction procedure based on a comparison between the original treatment and the simulated treatment,
   wherein the plurality of random modified values are generated for a parameter associated with a member selected from the group consisting of a permanent error, an alignment error, a calibration error, a treatment error, and a noise fluctuation.

2. The method according to claim 1, wherein the set of surgery parameters comprises a member selected from the group consisting of a treatment plan parameter, a flap incision parameter, an ablation parameter, a human error parameter, a psychology parameter, a physiology parameter, a patient perception parameter, a surgical condition parameter, and a surgical environment parameter.

3. The method according to claim 1, wherein the plurality of random modified values are generated for a parameter associated with a permanent error.

4. The method according to claim 1, wherein the plurality of random modified values are generated for a parameter associated with an alignment error.

5. The method according to claim 1, wherein the plurality of random modified values are generated for a parameter associated with a calibration error.

6. The method according to claim 1, wherein the plurality of random modified values are generated for a parameter associated with a treatment error.

7. The method according to claim 1, wherein the plurality of random modified values are generated for a parameter associated with a noise fluctuation.

8. A system for evaluating error in a vision correction procedure, the system comprising:

an input that receives a treatment table containing laser device instructions;
an input that receives a set of surgery parameters associated with the vision correction procedure;
a module that determines an original treatment based on original values for the set of surgery parameters;
a module that generates a plurality of random modified values for at least one parameter of the set of surgery parameters;
a module that simulates a treatment based on the plurality of random modified values and the treatment table; and
a module that evaluates error in the vision correction procedure based on a comparison between the original treatment and the simulated treatment,
wherein the plurality of random modified values are generated for a parameter associated with a member selected from the group consisting of a permanent error, an alignment error, a calibration error, a treatment error, and a noise fluctuation.

9. The system according to claim 8, wherein the set of surgery parameters comprises a member selected from the group consisting of a treatment plan parameter, a flap incision parameter, an ablation parameter, a human error parameter, a psychology parameter, a physiology parameter, a patient perception parameter, a surgical condition parameter, and a surgical environment parameter.

10. The system according to claim 8, wherein the plurality of random modified values are generated for a parameter associated with a permanent error.

11. The system according to claim 8, wherein the plurality of random modified values are generated for a parameter associated with an alignment error.

12. The system according to claim 8, wherein the plurality of random modified values are generated for a parameter associated with a calibration error.

13. The system according to claim 8, wherein the plurality of random modified values are generated for a parameter associated with a treatment error.

14. The system according to claim 8, wherein the plurality of random modified values are generated for a parameter associated with a noise fluctuation.

**Patentansprüche**

1. Verfahren zum Beurteilen eines Fehlers in einem Sehkorrekturvorgang, wobei das Verfahren Folgendes umfasst:

Empfangen einer Behandlungstabelle, die Laservorrichtungsanweisungen enthält;
Empfangen einer Gruppe von Chirurgieparametern, die dem Sehkorrekturvorgang zugeordnet sind;
Bestimmen einer ursprünglichen Behandlung anhand von Originalwerten für die Gruppe von Chirurgieparametern;
Erzeugen von mehreren zufällig geänderten Werten für mindestens einen Parameter der Gruppe von Chirurgieparametern;
Simulieren einer Behandlung anhand der mehreren zufällig geänderten Werten und der Behandlungstabelle; und
Beurteilen eines Fehlers in dem Sehkorrekturvorgang anhand eines Vergleichs zwischen der ursprünglichen Behandlung und der simulierten Behandlung,
wobei die mehreren zufällig geänderten Werte für einen Parameter erzeugt werden, der einem Element zugeordnet ist, das aus der Gruppe ausgewählt ist, die aus einem permanenten Fehler, einem Ausrichtungsfehler, einem Kalibrierungsfehler, einem Behandlungsfehler und einer Rauschfluktuation besteht.

2. Verfahren nach Anspruch 1, wobei die Gruppe der Chirurgieparameter ein Element umfasst, das aus der Gruppe ausgewählt ist, die aus einem Behandlungsplanparameter, einem Klappeneinschnittparameter, einem Ablationsparameter, einem Parameter eines menschlichen Fehlers, einem Psychologieparameter, einem Physiologieparameter, einem Parameter der Wahrnehmung des Patienten, einem Parameter der chirurgischen Bedingungen und einem Parameter der chirurgischen Umgebung besteht.

**3.** Verfahren nach Anspruch 1, wobei die mehreren zufällig geänderten Werte für einen Parameter erzeugt werden, der einem permanenten Fehler zugeordnet ist.

**4.** Verfahren nach Anspruch 1, wobei die mehreren zufällig geänderten Werte für einen Parameter erzeugt werden, der einem Ausrichtungsfehler zugeordnet ist.

**5.** Verfahren nach Anspruch 1, wobei die mehreren zufällig geänderten Werte für einen Parameter erzeugt werden, der einem Kalibrierungsfehler zugeordnet ist.

**6.** Verfahren nach Anspruch 1, wobei die mehreren zufällig geänderten Werte für einen Parameter erzeugt werden, der einem Behandlungsfehler zugeordnet ist.

**7.** Verfahren nach Anspruch 1, wobei die mehreren zufällig geänderten Werte für einen Parameter erzeugt werden, der einer Rauschfluktuation zugeordnet ist.

**8.** System zum Beurteilen eines Fehlers in einem Sehkorrekturvorgang, wobei das System Folgendes umfasst:

einen Eingang, der eine Behandlungstabelle empfängt, die Laservorrichtungsanweisungen enthält;
einen Eingang, der eine Gruppe von Chirurgieparametern empfängt, die dem Sehkorrekturvorgang zugeordnet sind;
ein Modul, das eine ursprüngliche Behandlung anhand von Originalwerten für die Gruppe von Chirurgieparametern bestimmt;
ein Modul, das mehrere zufällig geänderte Werte für mindestens einen Parameter der Gruppe von Chirurgieparametern erzeugt;
ein Modul, das eine Behandlung anhand der mehreren zufällig geänderten Werte und der Behandlungstabelle simuliert; und
ein Modul, das einen Fehler in dem Sehkorrekturvorgang anhand eines Vergleichs zwischen der ursprünglichen Behandlung und der simulierten Behandlung beurteilt,
wobei die mehreren zufällig geänderten Werte für einen Parameter erzeugt werden, der einem Element zugeordnet ist, das aus der Gruppe ausgewählt ist, die aus einem permanenten Fehler, einem Ausrichtungsfehler, einem Kalibrierungsfehler, einem Behandlungsfehler und einer Rauschfluktuation besteht.

**9.** System nach Anspruch 8, wobei die Gruppe der Chirurgieparameter ein Element umfasst, das aus der Gruppe ausgewählt ist, die aus einem Behandlungsplanparameter, einem Klappeneinschnittparameter, einem Ablationsparameter, einem Parameter eines menschlichen Fehlers, einem Psychologieparameter, einem Physiologieparameter, einem Parameter der Wahrnehmung des Patienten, einem Parameter der chirurgischen Bedingungen und einem Parameter der chirurgischen Umgebung besteht.

**10.** System nach Anspruch 8, wobei die mehreren zufällig geänderten Werte für einen Parameter erzeugt werden, der einem permanenten Fehler zugeordnet ist.

**11.** System nach Anspruch 8, wobei die mehreren zufällig geänderten Werte für einen Parameter erzeugt werden, der einem Ausrichtungsfehler zugeordnet ist.

**12.** System nach Anspruch 8, wobei die mehreren zufällig geänderten Werte für einen Parameter erzeugt werden, der einem Kalibrierungsfehler zugeordnet ist.

**13.** System nach Anspruch 8, wobei die mehreren zufällig geänderten Werte für einen Parameter erzeugt werden, der einem Behandlungsfehler zugeordnet ist.

**14.** System nach Anspruch 8, wobei die mehreren zufällig geänderten Werte für einen Parameter erzeugt werden, der einer Rauschfluktuation zugeordnet ist.

**Revendications**

1. Procédé d'évaluation d'une erreur d'une procédure de correction de la vue, le procédé comprenant :

   la réception d'une table de traitement contenant des instructions d'un dispositif laser ;
   la réception d'un ensemble de paramètres chirurgicaux associés à la procédure de correction de la vue ;
   la détermination d'un traitement initial en fonction de valeurs initiales de l'ensemble de paramètres chirurgicaux ;
   la génération d'une pluralité de valeurs modifiées de manière aléatoire d'au moins un paramètre de l'ensemble de paramètres chirurgicaux ;
   la simulation d'un traitement en fonction de la pluralité de valeurs modifiées de manière aléatoire et de la table de traitement ; et
   l'évaluation d'une erreur de la procédure de correction de la vue en fonction d'une comparaison entre le traitement initial et le traitement simulé,
   dans lequel la pluralité de valeurs modifiées de manière aléatoire est générée pour un paramètre associé à un élément sélectionné dans le groupe consistant en une erreur permanente, une erreur d'alignement, une erreur de calibrage, une erreur de traitement, et une fluctuation de bruit.

2. Procédé selon la revendication 1, dans lequel l'ensemble de paramètres chirurgicaux comprend un paramètre de protocole de traitement, un paramètre d'incision de lambeau, un paramètre d'ablation, un paramètre d'erreur humaine, un paramètre psychologique, un paramètre physiologique, un paramètre de perception du patient, un paramètre d'état chirurgical, et un paramètre d'environnement chirurgical.

3. Procédé selon la revendication 1, dans lequel la pluralité de valeurs modifiées de manière aléatoire est générée pour un paramètre associé à une erreur permanente.

4. Procédé selon la revendication 1, dans lequel la pluralité de valeurs modifiées de manière aléatoire est générée pour un paramètre associé à une erreur d'alignement.

5. Procédé selon la revendication 1, dans lequel la pluralité de valeurs modifiées de manière aléatoire est générée pour un paramètre associé à une erreur de calibrage.

6. Procédé selon la revendication 1, dans lequel la pluralité de valeurs modifiées de manière aléatoire est générée pour un paramètre associé à une erreur de traitement.

7. Procédé selon la revendication 1, dans lequel la pluralité de valeurs modifiées de manière aléatoire est générée pour un paramètre associé à une fluctuation de bruit.

8. Système d'évaluation d'une erreur d'une procédure de correction de la vue, le système comprenant :

   une entrée qui reçoit une table de traitement contenant des instructions d'un dispositif laser ;
   une entrée qui reçoit un ensemble de paramètres chirurgicaux associés à la procédure de correction de la vue ;
   un module qui détermine un traitement initial en fonction de valeurs initiales de l'ensemble de paramètres chirurgicaux ;
   un module qui génère une pluralité de valeurs modifiées de manière aléatoire d'au moins un paramètre de l'ensemble de paramètres chirurgicaux ;
   un module qui simule un traitement en fonction de la pluralité de valeurs modifiées de manière aléatoire et de la table de traitement ; et
   un module qui évalue une erreur de la procédure de correction de la vue en fonction d'une comparaison entre le traitement initial et le traitement simulé,
   dans lequel la pluralité de valeurs modifiées de manière aléatoire est générée pour un paramètre associé à un élément sélectionné dans le groupe consistant en une erreur permanente, une erreur d'alignement, une erreur de calibrage, une erreur de traitement, et une fluctuation de bruit.

9. Système selon la revendication 8, dans lequel l'ensemble de paramètres chirurgicaux comprend un élément sélectionné dans le groupe consistant en un paramètre de protocole de traitement, un paramètre d'incision de lambeau, un paramètre d'ablation, un paramètre d'erreur humaine, un paramètre psychologique, un paramètre physiologique, un paramètre de perception du patient, un paramètre d'état chirurgical, et un paramètre d'environnement chirurgical.

**10.** Système selon la revendication 8, dans lequel la pluralité de valeurs modifiées de manière aléatoire est générée pour un paramètre associé à une erreur permanente.

**11.** Système selon la revendication 8, dans lequel la pluralité de valeurs modifiées de manière aléatoire est générée pour un paramètre associé à une erreur d'alignement.

**12.** Système selon la revendication 8, dans lequel la pluralité de valeurs modifiées de manière aléatoire est générée pour un paramètre associé à une erreur de calibrage.

**13.** Système selon la revendication 8, dans lequel la pluralité de valeurs modifiées de manière aléatoire est générée pour un paramètre associé à une erreur de traitement.

**14.** Système selon la revendication 8, dans lequel la pluralité de valeurs modifiées de manière aléatoire est générée pour un paramètre associé à une fluctuation de bruit.

FIG. 1

EP 2 874 584 B1

FIG. 3

FIG. 3A

WAVEFRONT DEVICE

1. SPOT IDENTIFICATION
2. ACCOMMODATION
3. RECONSTRUCTION

LASER ABLATION PROFILE

1. PULSE PROFILE
2. SPOT SIZE VARIABILITY
3. BEAM UNIFORMITY
4. ABLATION RATE

LASER REGISTRATION
AND TRACKING SYSTEM

1. REGISTRATION
2. LINEAR TRACKING
3. TORSIONAL TRACKING

WAVEFRONT BOX
(WAVEFRONT MAP)

ALGORITHM BOX
(TREATMENT TABLE)

ABLATION BOX
(CORNEA SHAPE)

INPUT PROFILE
(HIGH RESOLUTION
SURFACE)

POINT-TO-POINT
SURFACE
COMPARISON

OUTPUT PROFILE
(HIGH RESOLUTION
SURFACE)

INPUT CASES

1. MYOPIA
2. HYPEROPIA
3. MYOPIC ASTIGMATISM
4. HYPEROPIC ASTIGMATISM
5. MIXED ASTIGMATISM
6. THERAPEUTIC

METRICS

1. ACCURACY
2. HEALING
3. TREATMENT TIME

RESULTS

1. RMS ERROR IN MICRONS
2. TEMPERATURE
3. ABLATION TIME

FIG. 4

WAVEFRONT DEVICE

1. SPOT IDENTICATION
2. ACCOMMODATION
3. RECONSTRUCTION

LASER
ABLATION PROFILE

1. LASER BEAM PROFILE
2. GRID GEOMETRY
3. ABLATION ALGORITHM

LASER
REGISTRATION
AND
TRACKING SYSTEM

1. REGISTRATION
2. LINEAR TRACKING
3. TORSIONAL
   TRACKING

LASER DELIVERY
SYSTEM

1. BEAM UNIFORMITY
2. ABLATION RATE

WAVEFRONT
DEVICE

(WAVEFRONT MAP)

FITTING BOX

(TREATMENT TABLE)

ALIGNMENT BOX

(CORNEA SHAPE)

ABLATION BOX

(CORNEA SHAPE)

HEALING BOX

(CORNEA SHAPE)

MICROKERATOME
SYSTEM

1. SPECIAL ABERRATION
2. HINGE EFFECT
3. ORIENTATION

LASIK FLAP BOX

(CORNEA SHAPE)

BIOMECHANICAL
EFFECT

1. LASER-TISSUE
   INTERACTION
2. HEALING

FIG. 5

EP 2 874 584 B1

FIG. 6

EP 2 874 584 B1

RMS ERROR DUE TO ACCOMMODATION

FIG. 7

RMS ERROR DUE TO RECONSTRUCTION

FIG. 8

HIGH ORDER ABERRATIONS OF A THERAPEUTIC EYE

FIG. 8A

FLYING SPOT SCANNING PROFILE

DISTANCE FROM PUPIL CENTER IN MM

FIG. 9A

VARIABLE SPOT SCANNING PROFILE

FIG. 9B

FITTING ERROR COMPARISON (VSS VS FSS)

FIG. 9C

FIG. 10A

FIG. 11A

FIG. 12A

PV DISTRIBUTION GRAPH FOR FSS

FIG. 12B

FIG. 13A

FIG. 13B

REGISTRATION INDUCED RMS ERROR FOR FSS

FIG. 13C

TORSIONAL REGISTRATION RMS ERROR FOR FSS

FIG. 13D

REGISTRATION ERROR COMPARISON (VSS VS FSS)

FIG. 13E

FIG. 14A

FIG. 14B

SIMULATED X MOTION

FIG. 14C

SIMULATED Y MOTION

FIG. 14D

FIG. 15A

FIG. 15B

FIG. 15C

TRACKING EFFICIENCY WITH RMS ERROR FOR FSS

FIG. 15D

TRACKING EFFICIENCY WITH RMS ERROR FOR FSS

FIG. 15E

TRACKING EFFICIENCY WITH RMS ERROR FOR FSS

FIG. 15F

FIG. 16A

FIG. 16B

FIG. 16C

FIG. 16D

FIG. 16E

FIG. 16F

FIG. 17A

FIG. 17B

FIG. 17C

FIG. 18A

FIG. 18B

FIG. 18C

FIG. 18D

FIG. 18E

FIG. 18F

FIG. 19A

FIG. 19B

FIG. 20

FIG. 21

FIG. 22

ERROR COMPARISON (VSS VS FSS)

FIG. 23A

TRACKING ERROR COMPARISON (VSS VS FSS)

FIG. 23B

2400

2420

SET OF SURGERY PARAMETERS
HAVING ORIGINAL VALUES
(E.G. A, B, C)
(FOR AN ORIGINAL TREATMENT)

2440

TREATMENT TABLE

CONTAINING LASER DEVICE
INSTRUCTIONS ASSOCIATED
WITH A VISION TREATMENT

2410

COMPARE
ORIGINAL TREATMENT
WITH SIMULATED
TREATMENT

SET OF SURGERY PARAMETERS
HAVING RANDOM VALUES
(E.G. A*, B, C)
(E.G. A*, B*, C*)
(FOR A SIMULATED TREATMENT)

2430

EVALUATE ERROR IN THE VISION
TREATMENT BASED ON THE
COMPARISON

2450

FIG. 24

2400a

| INPUTTING A REFRACTIVE CASE | 2410a |

| DETERMINE A MODEL OPTICAL SURFACE SHAPE BASED ON THE REFRACTIVE CASE AND A SET OF REFRACTIVE SURGERY SYSTEM PARAMETERS | 2420a |

| COMPARING THE REFRACTIVE CASE AND THE MODEL OPTICAL SURFACE SHAPE TO DETERMINE AN ABERRATION INDUCED BY THE SET OF REFRACTIVE SURGERY SYSTEM PARAMENTERS | 2430a |

| ADJUSTING THE SET OF REFRACTIVE SURGERY SYSTEM PARAMETERS SO AS TO INHIBIT THE INDUCED ABERRATION | 2440a |

| ADMINISTERING THE REFRACTIVE TREATMENT TO A PATIENT, WHERE THE REFRACTIVE TREATMENT IS BASED ON THE ADJUSTED SET OF REFRACTIVE SURGERY SYSTEM PARAMETERS | 2450a |

FIG. 24A

2400b

INPUTTING A REFRACTIVE CASE TO AN INPUT DEVICE OF A COMPUTER SYSTEM — 2410b

DETERMINING A MODEL OPTICAL SURFACE SHAPE BASED ON THE REFRACTIVE CASE AND A SET OF REFRACTIVE SURGERY SYSTEM PARAMETERS WITH A DETERMINATION MODULE OF THE COMPUTER SYSTEM, WHERE THE SET OF REFRACTIVE SURGERY SYSTEM PARAMETERS IS EMBODIED WITHIN A DATA FILE OF A REFRACTIVE SURGERY SYSTEM — 2420b

COMPARING THE REFRACTIVE CASE AND THE MODEL OPTICAL SURFACE SHAPE TO DETERMINE AN ABERRATION INDUCED BY THE SET OF REFRACTIVE SURGERY SYSTEM PARAMETERS EMBODIED WITHIN THE DATA FILE OF THE REFRACTIVE SURGERY SYSTEM WITH A COMPARISON MODULE OF THE COMPUTER SYSTEM — 2430b

ADJUSTING THE SET OF REFRACTIVE SURGERY SYSTEM PARAMETERS EMBODIED WITHIN THE DATA FILE OF THE REFRACTIVE SURGERY SYSTEM SO AS TO INHIBIT THE INDUCED ABERRATION WITH AN ADJUSTMENT MODULE OF THE COMPUTER SYSTEM — 2440b

FIG. 24B

2400c

INPUTTING A REFRACTIVE CASE TO AN INPUT DEVICE OF A COMPUTER SYSTEM ⎯2410c

↓

DETERMINING A MODEL OPTICAL SURFACE SHAPE BASED ON THE REFRACTIVE CASE AND A SET OF REFRACTIVE SURGERY SYSTEM PARAMETERS WITH A DETERMINATION MODULE OF THE COMPUTER SYSTEM, WHERE THE SET OF REFRACTIVE SURGERY SYSTEM PARAMETERS IS EMBODIED WITHIN MACHINE READABLE DATA OF A TANGIBLE STORAGE MEDIA OF A REFRACTIVE SURGERY SYSTEM ⎯2420c

↓

COMPARING THE REFRACTIVE CASE AND THE MODEL OPTICAL SURFACE SHAPE TO DETERMINE AN ABERRATION DISTRIBUTION WITH A COMPARISON MODULE OF THE COMPUTER SYSTEM ⎯2430c

↓

ADJUSTING THE SET OF REFRACTIVE SURGERY SYSTEM PARAMETERS EMBODIED WITHIN MACHINE READABLE DATA OF THE TANGIBLE STORAGE MEDIA OF THE REFRACTIVE SURGERY SYSTEM SO AS TO ALTER THE ABERRATION DISTRIBUTION WITH AN ADJUSTMENT MODULE OF THE COMPUTER SYSTEM ⎯2440c

## FIG. 24C

2400d

| INPUTTING A REFRACTIVE CASE TO AN INPUT DEVICE OF A COMPUTER SYSTEM |
| --- |

2410d

| DETERMINING A MODEL OPTICAL SURFACE SHAPE BASED ON THE REFRACTIVE CASE AND A SET OF REFRACTIVE SURGERY SYSTEM PARAMETERS WITH A DETERMINATION MODULE OF THE COMPUTER SYSTEM, WHERE THE SET OF REFRACTIVE SURGERY SYSTEM PARAMETERS IS EMBODIED WITHIN A STORAGE MODULE OF A REFRACTIVE SURGERY SYSTEM |
| --- |

2420d

| ADJUSTING THE SET OF REFRACTIVE SURGERY SYSTEM PARAMETERS EMBODIED WITHIN THE STORAGE MODULE OF THE REFRACTIVE SURGERY SYSTEM SO AS TO INHIBIT THE ABERRATION WITH AN ADJUSTMENT MODULE OF THE COMPUTER SYSTEM |
| --- |

2430d

# FIG. 24D

2400e

```
┌──────────────────────────────────────────────────────────────────────┐
│      RECEIVING A TREATMENT TABLE CONTAINING LASER DEVICE INSTRUCTIONS   │──2410e
└──────────────────────────────────────────────────────────────────────┘
                                    │
                                    ▼
┌──────────────────────────────────────────────────────────────────────┐
│   RECEIVING A SET OF SURGERY PARAMETERS ASSOCIATED WITH THE VISION      │
│                     CORRECTION PROCEDURE                                │──2420e
└──────────────────────────────────────────────────────────────────────┘
                                    │
                                    ▼
┌──────────────────────────────────────────────────────────────────────┐
│  DETERMINING AN ORIGINAL TREATMENT BASED ON ORIGINAL VALUES FOR THE SET │
│                    OF SURGERY PARAMETERS                                │──2430e
└──────────────────────────────────────────────────────────────────────┘
                                    │
                                    ▼
┌──────────────────────────────────────────────────────────────────────┐
│ GENERATING A PLURALITY OF RANDOM MODIFIED VALUES FOR AT LEAST ONE       │
│        PARAMETER OF THE SET OF SURGERY PARAMETERS                       │──2440e
└──────────────────────────────────────────────────────────────────────┘
                                    │
                                    ▼
┌──────────────────────────────────────────────────────────────────────┐
│ SIMULATING A TREATMENT BASED ON THE PLURALITY OF RANDOM MODIFIED VALUES │
│               AND THE TREATMENT TABLE                                   │──2450e
└──────────────────────────────────────────────────────────────────────┘
                                    │
                                    ▼
┌──────────────────────────────────────────────────────────────────────┐
│ EVALUATING ERROR IN THE VISION CORRECTIONS PROCEDURE BASED ON A         │
│ COMPARISON BETWEEN THE ORIGINAL TREATMENT AND THE SIMULATED TREATMENT   │──2460e
└──────────────────────────────────────────────────────────────────────┘
```

FIG. 24E

2500

2520

2530

2510

VIRTUAL ABLATION
SIMULATOR WITH
LOADABLE
COMPONENTS

FINAL ABLATED
SURFACE

REFRACTIVE CASES

IDEAL CORRECTION
SURFACE

RESIDUAL ERROR

2540

2550

## FIG. 25

FIG. 26

FIG. 27

2800

2806

RECEIVING A SET OF OPERATIONAL PARAMETERS

OPERATIONAL PARAMTER A <-> (E.G. WAVEFRONT MEASUREMENT PARAMETER)
OPERATIONAL PARAMTER B <-> (E.G. STROMA REGENERATION PARAMETER)
OPERATIONAL PARAMTER C <-> (E.G. PULSE ABLATION DEPTH PARAMETER)

2804

TREATMENT
TABLE

2802

INPUT FOR
RECEIVING A
TREATMENT TABLE

2810

RECEIVING
A SELECTION FOR TYPE OF ANALYSIS

2812

*ERROR ANALYSIS*

*FACTOR ANALYSIS (TYPE A)* — 2820

ASSOCIATING A TOLERANCE RANGE WITH A
SELECTED OPERATIONAL PARAMETER

2822

RANDOMLY GENERATING A PLURALITY OF VALUES
(WITHIN THE TOLERANCE RANGE) FOR THE
SELECTED OPERATIONAL PARAMETER

2824

COMPUTING AN ERROR VALUE ASSOCIATED WITH
THE SELECTED OPERATIONAL PARAMETER, BASED
ON:
  A) THE RANDOMLY GENERATED VALUES;
  B) DEFAULT VALUES FOR THE NON-SELECTED
     OPERATIONAL PARAMETERS;
     AND
  C) THE TREATMENT TABLE

2826

*SYSTEM PERFOMANCE (TYPE B)* — 2830

ASSOCIATING A TOLERANCE RANGE WITH A
OPERATIONAL PARAMETER

2832

RANDOMLY GENERATING A PLURALITY OF VALUES
(WITHIN THE ASSOCIATED TOLERANCE RANGE) FOR
EACH OF THE OPERATIONAL PARAMETERS

2834

COMPUTING AN ERROR VALUE ASSOCIATED WITH
THE SET OF OPERATIONAL PARAMETERS, BASED
ON:
  A) THE RANDOMLY GENERATED VALUES; AND
  B) THE TREATMENT TABLE

2836

ERROR VALUE (METRICS):

A) MAY BE COMPUTED AS RMS ERROR FOR AN ABLATION SURFACE

B) MAY BE COMPUTED AS ERRORS IN LOAs (SPHERE AND CYLINDER REFRACTION)

C) MAY BE COMPUTED AS ERRORS IN HOAs (SPHERICAL AND ABERRATION, COMA, TREFOIL)

2840

FOR EACH ERROR VALUE:
COMPUTE MEAN, STD DEV, MAX, AND 95TH %LLE

2850

# FIG. 28

FIG. 29

FIG. 30

EP 2 874 584 B1

INTEGRATOR POSITION VS ENERGY FOR 6.0MM IRIS

Legend:
— AMPL 3728
---- AMPL 5182
- - AMPL 3017
— AMPL 3425

FIG. 31

EP 2 874 584 B1

SN 3179 OZONE CALIBRATION REPEATABILITY (20 Hz)

| | |
|---|---|
| —— | 4766 18 EP (mJ) |
| –·– | 6554 27 EP (mJ) |
| –·– | 5374 22 EP (mJ) |
| —— | 6226 24 EP (mJ) |
| – – | 6291 24 EP (mJ) |
| — | 5958 25 EP (mJ) |

FIG. 32

OPD OF CORNEA VS. FLUENCE. CALIBRATION PLASTIC AND VISX ABLATIONS

Y = 0.4716X + 10.682
R2 = 0.9754

Y = 0.2079X + 17.662
R2 = 0.9869

FLUENCE (mJ/cm2)

OPD, NM/PULSE

FIG. 33

◇ CORNEA (VISX)     * CAL. PLASTIC        —— CORNEA LINEAR
—— PLASTIC LINEAR   —— LINEAR (CORNEA LINEAR)   —— LINEAR (PLASTIC LINEAR)

FIG. 34A

FIG. 34B

HYPEROPIA POSITIONING ERROR

FIG. 35

EP 2 874 584 B1

FIG. 36

FIG. 37

FIG. 38A

EP 2 874 584 B1

FIG. 38B

BASELINE

FIG. 38C

FIG. 38D

FIG. 39

FIG. 39A

FIG. 39B

FIG. 40

EP 2 874 584 B1

FIG. 41

EP 2 874 584 B1

FIG. 42

FIG. 43

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6315413 B
- US 5713892 A
- US 5683379 A
- US 6203539 B
- US 6331177 B
- US 4665913 A
- US 5807379 A
- US 5646791 A
- US 4669466 A
- US 4732148 A
- US 4770172 A
- US 4773414 A
- US 5207668 A
- US 5108388 A
- US 5219343 A
- US 5163934 A
- US 6004313 A
- US 6095651 A
- US 6271915 B
- US 6673062 B
- US 60553580 B
- US 6099125 A
- US 6000800 A
- US 5258791 A

### Non-patent literature cited in the description

- **DAI.** Modal wave-front reconstruction with Zernike polynomials and Karhunen-Loève functions. *J. Opt. Soc. Am. A,* 1996, vol. 13, 1218-1225
- **CYNTHIA ROBERTS.** The cornea is not a piece of plastic. *J. Refract. Surg.,* 2000, vol. 16 (4), 407-413
- **JASON PORTER et al.** Separate effects of the microkeratome incision and laser ablation on the eye's wave aberration. *Am. J. Ophthalmol.,* 2003, vol. 136 (2), 327-337
- **DAVID HUANG et al.** Mathematical model of corneal surface smoothing after laser refractive surgery. *Am. J. Ophthalmol.,* 2003, vol. 135 (3), 267-278